(19) Europäisches Patentamt
European Patent Office
Office européen des brevets

(11) EP 0 697 407 B1

(12) **EUROPEAN PATENT SPECIFICATION**

(45) Date of publication and mention of the grant of the patent:
**25.06.1997 Bulletin 1997/26**

(51) Int. Cl.[6]: **C07D 239/54**, A61K 31/505

(21) Application number: **95305781.7**

(22) Date of filing: **18.08.1995**

(54) **Pyrimidinedione derivatives and antiarrhythmic compositions containing same**
Pyrimidin-Dion-Derivate und diese enthaltende antiarrhythmische Zusammensetzungen
Dérivés de pyrimidinedione et compositions antiarrhythmiques les contenant

(84) Designated Contracting States:
**DE FR GB IT**

(30) Priority: **19.08.1994 JP 195188/94**

(43) Date of publication of application:
**21.02.1996 Bulletin 1996/08**

(73) Proprietor: **MITSUI TOATSU CHEMICALS, Inc.**
**Chiyoda-Ku Tokyo 100 (JP)**

(72) Inventors:
- **Kaiho, Tatsuo**
  **Omuta-shi, Fukuoka-ken (JP)**
- **Fujiwara, Junya**
  **Mobara-shi, Chiba-ken (JP)**
- **Tomoshige, Naoki**
  **Mobara-shi, Chiba-ken (JP)**
- **Sakamoto, Youichi**
  **Higashi-ku, Fukuoka-ken (JP)**
- **Banno, Hitoshi**
  **Mobara-shi, Chiba-ken (JP)**
- **Suga, Hiroyuki**
  **Kiryu-shi, Gunma-ken (JP)**
- **Sato, Takashi**
  **Mobara-shi, Chiba-ken (JP)**
- **Hosoya, Junko**
  **Mobara-shi, Chiba-ken (JP)**

(74) Representative: **Stuart, Ian Alexander et al**
**MEWBURN ELLIS**
**York House**
**23 Kingsway**
**London WC2B 6HP (GB)**

(56) References cited:
**EP-A- 0 369 627**
**EP-A- 0 430 693**
**EP-A- 0 452 107**
**EP-A- 0 454 498**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 697 407 B1

## Description

This invention relates to pyrimidinedione derivatives and pharmaceutically acceptable salts thereof, to processes for producing the same, and to antiarrhythmic compositions containing the same as active ingredients.

The mechanism of the occurrence of arrhythmia is complicated, and abnormalities in stimulus production and disorders in the conducting system or combinations thereof are considered to be responsible. As to disorders in excitation conduction, the reentry theory is representative. One of the conditions of occurrence of arrhythmia is irregularity in the refractory period in various parts of the heart. In addition, unidirectional block, shortened refractory period, delayed conduction, and the presence of circus movement seem to be complicatedly involved. Conventionally, a variety of medicinal agents have been developed and used as antiarrhythmic agents. In the existing circumstances, however, no satisfactory results have been achieved in the treatment of arrhythmia which has complicated generating mechanisms and requires the administration of antiarrhythmic agents for a long period of time. Accordingly, a search for ideal antiarrhythmic agents is being continued.

E.M. Vaughan Williams ("Advances in drug research, Vol. 9", ed. by Harper N.J., Simmonds A.B., Academic Press, London, 1974, pp. 69-101) classified antiarrhythmic agents into the following four groups according to their effect on the action potential of the cardiac muscle or the ionic current which generates the action potential.

Class I: Sodium channel depressors

These agents reduce the maximum rising velocity ($V_{max}$) of the sodium current. The antiarrhythmic agents belonging to this class have a powerful antiarrhythmic effect but, on the other hand, strongly suppress cardiac functions. Careful consideration is required in administering them to patients with cardiac insufficiency or hypotension.

Class II: Beta-blocking agents

These agents, represented by propranolol, are efficacious owing to their beta-blocking action and are useful in the treatment of patients with arrhythmia in which the sympathetic nerve is involved. However, care must be taken in using them because their beta-blocking action may cause side effects such as suppression of cardiac functions, induction of bronchial asthmatic attacks, and induction of hypoglycemic seizures.

Class III: Agents for prolonging the retention time of the action potential

These agents are effective in remarkably prolonging the retention time of the action potential of the cardiac muscle and thereby extending the effective refractory period. It is believed that reentry arrhythmia can be suppressed by the action of these Class III type agents. For example, amiodarone and bretylium are known as antiarrhythmic agents of the Class III type. However, these agents have severe side effects and, therefore, careful consideration is required in using them.

Class IV: Calcium antagonists

These agents control the calcium channel and suppress arrhythmia due to automatic sthenia of the sinoatrial node and ventricular tachycardia containing the atrioventricular node in the reentry circuit.

Among these antiarrhythmic agents, those of the Class III type are known to be effective for ventricular arrhythmia which involves the greatest risk of being fatal, and are hence considered to be highly useful and particularly important.

As novel Class III type antiarrhythmic agents, the compounds disclosed in, for example, European Publication No. 245,997 A2, European Publication No. 127,435 A2, European Publication No. 235,752 A2 and U.S. Patent No. 4,544,654 are now under development. Moreover, pyrimidinedione derivatives having various substituted amino groups introduced at the 6-position of the pyrimidinedione ring and exhibiting an antiarrhythmic effect are disclosed in European Publication No. 369,627 A2, European Publication No. 430,693 B1, European Publication No. 452,107 A1, European Publication No. 454,498 A2 and the like.

With regard to the pyrimidinedione derivatives having various substituted amino groups introduced at the 6-position of the pyrimidinedione ring as disclosed in the aforementioned European Publication No. 369,627 and the like, it has been reported that the nitrogen-carbon bond linking the substituted amino group to the pyrimidinedione ring tends to be cleaved by hydrolysis under acid conditions [see, for example, SYNTHETIC COMMUNICATION, 14, 675-680(1984)]. Moreover, the pyrimidinedione derivatives having a hydroxyl-terminated alkyl group as disclosed in the aforementioned European Publication No. 369,627 involve the problem of being easily eliminated from the living body after administration. It has been found that this eliminating effect results from the *in vivo* conjugation of the terminal hydroxyl group of the alkyl group with glucuronic acid.

Accordingly, there is a need for novel Class III type antiarrhythmic agents showing a further improvement in the

duration of action in vivo.

Preferred embodiments of the invention in first and second aspects are novel compounds having an improved duration of action in vivo as Class III type antiarrhythmic agents and processes for producing the same. Another aspect of the present invention concerns antiarrhythmic compositions containing such compounds as active ingredients.

In the course of intensive investigations made in order to develop novel and useful compounds having an improved duration of action in vivo as Class III type antiarrhythmic agents and processes for producing the same, the present inventors have found that, if the conventional substituted amino group attached to the 6-position of the pyrimidinedione ring is replaced by a substituted alkyl group having a specific structure, pyrimidinedione derivatives having high stability under acid conditions and an improved duration of action while retaining their effect as Class III type antiarrhythmic agents can be obtained.

Pyrimidinedione derivatives in accordance with the present invention have a structure represented by formula (1)

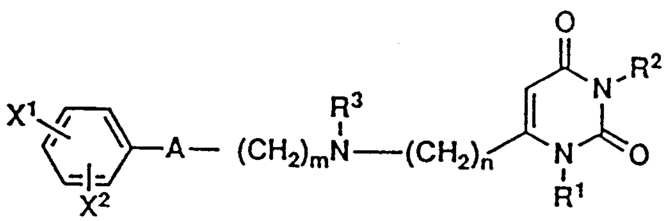

(1)

wherein

$R^1$ and $R^2$ each independently represent a hydrogen atom or an alkyl group of 1 to 6 carbon atoms;
$R^3$ represents a hydrogen atom, an alkyl group of 1 to 6 carbon atoms, an alkyl group of 1 to 4 carbon atoms which is substituted by a dialkylamino group of 2 to 12 carbon atoms, or an alkyl group of 1 to 4 carbon atoms which is substituted by an alkyloxy group of 1 to 6 carbon atoms;
A represents a methylene group or an oxygen atom;
$X^1$ and $X^2$ each independently represent a hydrogen atom, an alkanoyl group of 2 to 6 carbon atoms, a benzoyl group, an alkanoylamino group of 2 to 6 carbon atoms, an amino group, a nitro group, an alkylsulfonylamino group of 1 to 6 carbon atoms, a benzenesulfonylamino group, a cyano group, a fluorine atom, a chlorine atom, a trifluoromethyl group, an imidazole group, an alkyloxy group of 1 to 6 carbon atoms, a trifluoromethoxy group, or an alkylthio group of 1 to 6 carbon atoms;
m represents an integer of 1 to 4; and
n represents an integer of 2 to 4.

These pyrimidinedione derivatives can also be used as pharmaceutically acceptable salts thereof.

The preferred derivatives of the present invention and pharmaceutically acceptable salts thereof have high stability under acid conditions, a long half-time in the blood, and an improved duration of action even when used orally, and are hence useful as the active ingredients of Class III type antiarrhythmic compositions for the prevention and treatment of ventricular tachycardia, ventricular fibrillation, atrial flutter-fibrillation and cardiac insufficiency.

The compounds of the preferred embodiments are adventageous in particular as active ingredient of antiarrhythmic compositions for oral administration, because they are stable also at pH about 1.2 in the human stomach.

## DETAILED DESCRIPTION OF PREFERRED EMBODIMENTS

The present invention is more specifically described and exemplified hereinbelow.

In the above formula (1), the alkyl group of 1 to 6 carbon atoms represented by $R^1$, $R^2$ or $R^3$ is a straight-chain, branched or cyclic alkyl group. Examples thereof include methyl, ethyl, n-propyl, n-butyl, n-pentyl, n-hexyl, isopropyl, 2-methylpropyl, 1-methylpropyl; t-butyl, 3-methylbutyl, 2-methylbutyl, 1-methylbutyl, 1,2-dimethylpropyl, 1,1-dimethylpropyl, 2,2-dimethylpropyl, 4-methylpentyl, 3-methylpentyl, 2-methylpentyl, 1-methylpentyl, 1,2,2-trimethylpropyl, 1,1-dimethylbutyl, 1,1,2-trimethylpropyl, 2,2-dimethylbutyl, 1,3-dimethylbutyl, 2,3-dimethylbutyl, cyclopropyl, cyclobutyl, cyclopentyl and cyclohexyl groups.

The alkyl group of 1 to 4 carbon atoms which is substituted by a dialkylamino group of 2 to 12 carbon atoms, as represented by $R^3$, has a structure in which one of the hydrogen atoms possessed by a straight-chain, branched or cyclic alkyl group of 1 to 4 carbon atoms is replaced by a dialkylamino group of 2 to 12 carbon atoms represented by -NR'R", wherein R' and R" each independently represent an alkyl group of 1-6 carbon atoms. Such alkyl groups of 1 to 4 carbon atoms include, for example, methyl, ethyl, n-propyl, n-butyl, isopropyl, 2-methylpropyl, 1-methylpropyl, t-butyl, cyclopropyl and cyclobutyl groups. The alkyl groups possessed by the dialkylamino substituent group may have a

straight-chain, branched or cyclic structure. Examples of the dialkylamino group include dimethylamino, diethylamino, di-n-propylamino, di-n-butylamino, di-n-pentylamino, di-n-hexylamino, diisopropylamino, di-t-butylamino, di(2-methylpropyl)amino, di(1-methylpropyl)amino, dicyclopropylamino, dicyclobutylamino, dicyclopentylamino, dicyclohexylamino, methylethylamino, methyl-n-propylamino, ethylisopropylamino, n-propyl-n-butylamino, methyl-t-butylamino, ethyl-n-pentylamino and n-propyl-n-hexylamino groups.

The alkyl group of 1 to 4 carbon atoms which is substituted by an alkyloxy group of 1 to 6 carbon atoms, as represented by $R^3$, has a structure in which one of the hydrogen atoms possessed by a straight-chain, branched or cyclic alkyl group of 1 to 4 carbon atoms is replaced by an alkyloxy group of 1 to 6 carbon atoms. Such alkyl groups of 1 to 4 carbon atoms include, for example, the groups enumerated above for the dialkylamino-substituted alkyl group. The alkyloxy substituent group may have a straight-chain, branched or cyclic alkyl group of 1 to 6 carbon atoms. Examples of the alkyloxy group include methoxy, ethoxy, n-propyloxy, n-butyloxy, n-pentyloxy, n-hexyloxy, isopropyloxy, t-butyloxy, 2-methylpropyloxy, 1-methylpropyloxy, cyclopropyloxy, cyclobutyloxy, cyclopentyloxy and cyclohexyloxy groups.

Examples of the alkanoyl group of 2 to 6 carbon atoms which is represented by $X^1$ or $X^2$ include acetyl, propionyl, butyryl, valeryl, hexanoyl, isobutyryl, isovaleryl, pivaloyl and cyclopentylcarbonyl groups.

Examples of the alkanoylamino group of 2 to 6 carbon atoms include acetylamino, propionylamino, butyrylamino, valerylamino, hexanoylamino, isobutyrylamino, isovalerylamino, pivaloylamino and cyclopentylcarbonylamino groups.

Examples of the alkylsufonylamino group of 1 to 6 carbon atoms include methanesulfonylamino, ethanesulfonylamino, n-propanesulfonylamino, n-butanesulfonylamino, n-pentanesulfonylamino, n-hexanesulfonylamino, isopropanesulfonylamino, 2-methylpropanesulfonylamino, 1-methylpropanesulfonylamino, t-butanesulfonylamino, 3-methylbutanesulfonylamino, 2-methylbutanesulfonylamino, 1-methylbutanesulfonylamino, 1,2-dimethylpropanesulfonylamino, 1,1-dimethylpropanesulfonylamino, 2,2-dimethylpropanesulfonylamino, 4-methylpentanesulfonylamino, 3-methylpentanesulfonylamino, 2-methylpentanesulfonylamino, 1-methylpentanesulfonylamino, 1,2,2-trimethylpropanesulfonylamino, 1,1-dimethylbutanesulfonylamino, 1,1,2-trimethylpropanesulfonylamino, 2,2-dimethylbutanesulfonylamino, 1,3-dimethylbutanesulfonylamino, 2,3-dimethylbutanesulfonylamino, cyclopropanesulfonylamino, cyclobutanesulfonylamino, cyclopentanesulfonylamino and cyclohexanesulfonylamino.

Examples of the alkyloxy group of 1 to 6 carbon atoms include methoxy, ethoxy, n-propyloxy, n-butyloxy, n-pentyloxy, n-hexyloxy, isopropyloxy, t-butyloxy, 2-methylpropyloxy, 1-methylpropyloxy, cyclopropyloxy, cyclobutyloxy, cyclopentyloxy and cyclohexyloxy groups.

Examples of the alkylthio group of 1 to 6 carbon atoms include methylthio, ethylthio, n-propylthio, n-butylthio, n-pentylthio, n-hexylthio, isopropylthio, t-butylthio, 2-methylpropylthio, 1-methylpropylthio, cyclopropylthio, cyclobutylthio, cyclopentylthio and cyclohexylthio groups.

Examples of the imidazole group include 1-imidazole, 2-imidazole, 4-imidazole and 5-imidazole groups.

$R^1$ and $R^2$ are each preferably an alkyl group of 1 to 3 carbon atoms. $R^3$ is preferably a hydrogen atom or an alkyl group of 1 to 4 carbon atoms. $X^1$ and $X^2$ are each preferably an alkanoyl group of 2 to 6 carbon atoms, an amino group, a nitro group, an alkylsulfonylamino group of 1 to 6 carbon atoms, an imidazole group, a fluorine atom or a chlorine group.

When the $R^1$, $R^2$, $R^3$, $X^1$ or $X^2$ group in formula (1) has an asymmetric carbon atom, the compounds of formula (1) in accordance with the present invention include optically active substances.

Specific examples of the compounds of formula (1) in accordance with the present invention are enumerated in Tables 5 and 6 which will be given after several tests. However, it is to be understood that the present invention is not limited to these compounds.

Now, the processes for producing the compounds of the present invention and intermediates useful in the production thereof are described hereinbelow. It is to be understood that, in the chemical formulas given below, the symbols which are in common with the foregoing formula (1) have the same meanings as described for formula (1), unless otherwise indicated.

Production Process 1

Compounds of formula (1) can be synthesized by mixing a benzene derivative of formula (2)

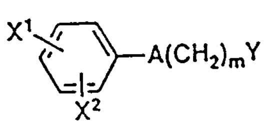

(2)

wherein Y represents a chlorine atom, a bromine atom, an iodine atom, a mesyloxy group or a tosyloxy group, with a pyrimidinedione derivative of formula (3)

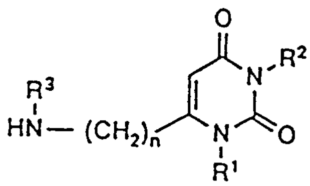

(3)

without using any solvent, or by reacting both reactants in a reaction system formed by dissolving or suspending them in a suitable solvent or dispersion medium.

Although no particular limitation is placed on the ratio at which the compound of formula (2) and the compound of formula (3) are used, they are usually used in a molar ratio of the former to the latter ranging from 1:10 to 10:1 and preferably from 1:2 to 2:1. The copresence of a base in the reaction system permits the reaction to proceed favorably. Such bases serving to promote this reaction include, for example, organic bases such as pyridine, dimethylaminopyridine, triethylamine and diisopropylethylamine; and inorganic bases such as potassium carbonate, sodium carbonate, sodium hydroxide and potassium hydroxide. Solvents or dispersion media suitable for use in this reaction include, for example, alcohols (such as methanol, ethanol, propanol and isopropanol), methyl ethyl ketone, dioxane, dimethylformamide, dimethyl sulfoxide, benzene, toluene, chloroform and dichloromethane. This reaction may be carried out at a temperature ranging from room temperature to the reflux temperature of the reaction mixture, for example, at a temperature in the range of 0 to 150°C and preferably 60 to 100°C. The reaction time ranges from about 1 to 20 hours, though it depends upon the conditions employed.

Pyrimidine derivatives of formula (3) wherein R3 is other than a hydrogen atom can be prepared by reacting a pyrimidine derivative of formula (5), which will be given later, with an alkylamine. Pyrimidine derivatives of formula (3) wherein R3 is a hydrogen atom can be prepared by hydrolyzing a pyrimidine derivative of formula (9)

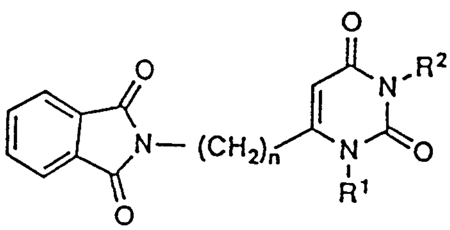

(9)

or by reacting it with hydrazine.

Compounds of formula (9) can be synthesized by any of well-known processes, for example, by reacting a metallic salt of phthalimide (such as potassium phthalimide) with a compound of formula (5) (wherein Y represents a chlorine atom, a bromine atom or an iodine atom) which will be given later, or by reacting phthalimide with a compound of formula (12), which will be given later, using a condensing agent [an application of Mitsunobu's reaction (O. Mitsunobu; Synthesis, 1-28(1981))].

Production Process 2

Compounds of formula (1) can be synthesized by reacting a compound of formula (4)

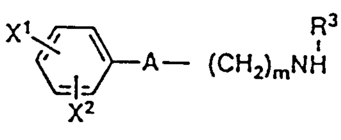

(4)

with a compound of formula (5)

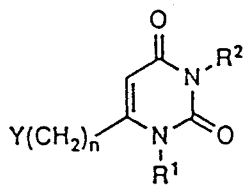

(5)

wherein Y is as defined for formula (2), in a reaction system formed by dissolving or suspending both reactants in a suitable solvent or dispersion medium.

Although no particular limitation is placed on the ratio at which the compound of formula (4) and the compound of formula (5) are used, they are usually used in a molar ratio of the former to the latter ranging from 1:10 to 10:1 and preferably from 1:2 to 2:1. The reaction conditions are the same as described for Production Process 1.

Production Process 3

Compounds of formula (1) wherein A is an oxygen atom can be prepared by reacting a compound of formula (6)

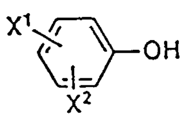

(6)

with a pyrimidine derivative of formula (7)

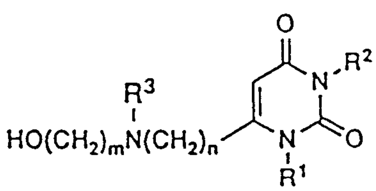

(7)

in the presence of a dehydrating condensing agent, in a reaction system formed by dissolving or suspending both reactants in a suitable solvent or dispersion medium (an application of Mitsunobu's reaction).

Although no particular limitation is placed on the ratio at which the compound of formula (6) and the compound of formula (7) are used, they are usually used at a molar ratio of the former to the latter ranging from 1:1 to 10:1 and preferably from 1:1 to 2:1. The reaction is carried out at a temperature equal to or lower than the reflux temperature of the solvent or dispersion medium used, and preferably at a temperature in the range of -10 to 80°C. The dehydrating condensing agent used in this reaction can be any of various dehydrating condensing agents commonly used for the formation of an ether linkage. Among others, a mixed dehydrating condensing agent comprising triphenylphosphine and diethyl azodicarboxylate is preferred. As the reaction medium, any inert solvent or dispersion medium may be used without limit, and examples thereof include tetrahydrofuran, ether, dioxane, chloroform and dichloromethane. The reaction time ranges from about 30 minutes to 12 hours, though it depends upon the conditions employed.

Next, the processes for producing novel intermediates useful in the production of compounds of formula (1) are described hereinbelow.

Intermediate Production Process 1

Compounds of formula (5)

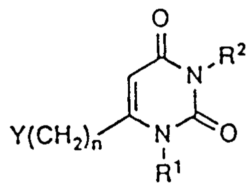

(5)

wherein Y is as defined for formula (2), can be obtained by reacting a 1,3-ketoester derivative of formula (10)

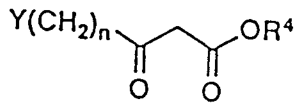

(10)

wherein $R^4$ represents a straight-chain, branched or cyclic alkyl group of 1 to 4 carbon atoms and Y is as defined for formula (2), with a urea derivative of formula (11)

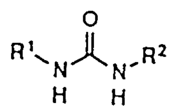

(11)

in the presence of an inorganic acid (such as hydrochloric acid or sulfuric acid), an organic acid (such as methanesulfonic acid, benzenesulfonic acid or p-toluenesulfonic acid) or an acid ion-exchange resin, under reduced or atmospheric pressure and at a temperature in the range of 0 to 150°C, preferably 60 to 100°C, without using any solvent or in a solvent such as ethanol, propanol, toluene or xylene. Compounds of formula (10) can be synthesized by a process reported by Leonard Lermer [Can. J. Chem., 70, 1427(1992)].

Although no particular limitation is placed on the ratio at which the compound of formula (10) and the compound of formula (11) are used, they are usually used in a molar ratio of the former to the latter ranging from 1:1 to 10:1 and preferably from 1:1 to 2:1. The reaction time ranges from about 1 to 20 hours, though it depends upon the conditions employed.

Intermediate Production Process 2

Pyrimidine derivatives of formula (5)

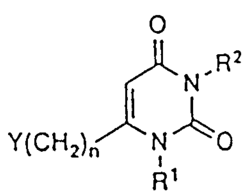

(5)

wherein Y is as defined for formula (2), can be synthesized by converting a compound of formula (12)

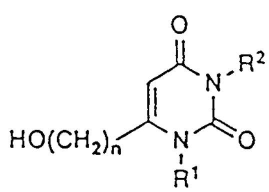

(12)

to the corresponding halide compound using thionyl chloride, phosphorus tribromide, or a reagent such as carbon tetrachloride/triphenylphosphine or carbon tetrabromide/triphenylphosphine, or by converting a compound of formula (12) to the corresponding sulfonate compound using methanesulfonyl chloride, p-toluenesulfonyl chloride or the like. The reactions may be carried out at a temperature in the range of -50 to 150°C, preferably -10 to 100°C, without using any solvent or in a solvent such as ether, benzene, toluene, xylene, dichloromethane, chloroform or carbon tetrachloride.

Intermediate Production Process 3

Compounds of formula (12)

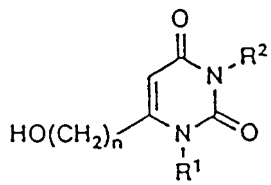

(12)

can be synthesized using a compound of formula (13)

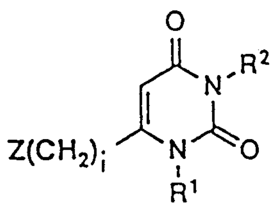

(13)

wherein Z represents a benzyloxy group, an alkyloxycarbonyl group of 2 to 7 carbon atoms, or a vinyl group; and i represents an integer of 1 to 3 when Z is an alkyloxycarbonyl group of 2 to 7 carbon atoms, an integer of 0 or 2 when Z is a vinyl group, or an integer of 2 to 4 in other cases. For example, compounds of formula (12) can be obtained by the reduction of a compound of formula (13) wherein Z is an alkyloxycarbonyl group of 2 to 7 carbon atoms, using aluminum lithium hydride, sodium boron hydride, lithium boron hydride or calcium boron hydride, at a temperature in the range of -50 to 60°C, preferably -20 to 0°C, in a solvent such as ether, tetrahydrofuran or methanol.

Moreover, compounds of formula (12) can also be synthesized by the hydrogenation of a compound of formula (13) wherein Z is a benzyloxy group. The hydrogenation reaction may be carried out in the presence of a catalyst such as palladium /carbon (hereinafter abbreviated as "Pd/C") or platinum oxide, under atmospheric or elevated pressure in a solvent such as methanol, ethanol or DMF.

Furthermore, compounds of formula (12) can also be synthesized by the hydroboration of a compound of formula (13) wherein Z is a vinyl group, as reported by Isao Saito [J. Org. Chem., 51, 5148(1986)]. Solvents or dispersion media suitable for use in this reaction include, for example, ether and THF, and useful reagents include borane-THF complex, borane-dimethyl sulfide complex, 9-borabicyclo[3.3.1.]nonane (9-BBN) and the like. This reaction may be carried out at a temperature ranging from room temperature to the reflux temperature of the reaction mixture, for example, at a temperature in the range of -50 to 150°C and preferably -10 to 80°C. The reaction time ranges from about 1 to 20 hours, though it depends upon the conditions employed.

Intermediate Production Process 4

Compounds of formula (13)

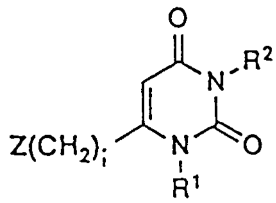

(13)

can be obtained by reacting a compound of formula (14)

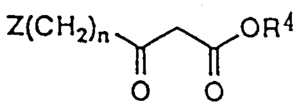

(14)

wherein $R^4$ is as defined for formula (10) and Z is as defined for formula (13), with a urea derivative of formula (11) under the same conditions as employed in Intermediate Production Process 1.

Intermediate Production Process 5

Pyrimidinedione derivatives of the foregoing formula (7) can be synthesized by reacting a pyrimidine derivative of the foregoing formula (5) with a compound of formula (15)

$$HO(CH_2)_mNR^3H \qquad (15)$$

in a reaction system formed by dissolving or suspending both reactants in a suitable solvent or dispersion medium. Although no particular limitation is placed on the ratio at which the compound of formula (5) and the compound of formula (15) are used, they are usually used in a molar ratio of the former to the latter ranging from 1:1 to 1:10 and preferably from 1:1 to 1:3. The copresence of a base in the reaction system permits the reaction to proceed favorably. Such bases serving to promote this reaction include, for example, organic bases such as pyridine, dimethylaminopyridine, triethylamine and diisopropylethylamine; and inorganic bases such as potassium carbonate, sodium carbonate, sodium hydroxide and potassium hydroxide. Solvents or dispersion media suitable for use in this reaction include, for example, alcohols (such as methanol, ethanol, propanol and isopropanol), methyl ethyl ketone, dioxane, dimethylformamide, dimethyl sulfoxide, benzene, toluene, chloroform and dichloromethane. This reaction may be carried out at a temperature ranging from room temperature to the reflux temperature of the reaction mixture, for example, at a temperature in the range of 0 to 150°C and preferably 60 to 100°C. The reaction time ranges from about 1 to 20 hours, though it depends upon the conditions employed.

Pyrimidinedione derivatives of formula (8)

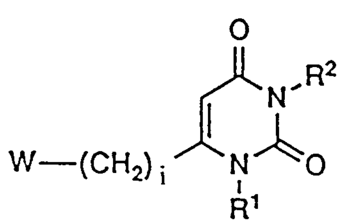

(8)

wherein W represents a hydroxyl group, a chlorine atom, a bromine atom, an iodine atom, a mesyloxy group, a tosyloxy group, a benzyloxy group, a phthaloylamino group, an alkyloxycarbonyl group of 2 to 7 carbon atoms, a vinyl group, -$NHR^3$ or -$NR^3(CH_2)_mOH$; and i represents an integer of 1 to 3 when W is an alkyloxycarbonyl group of 2 to 7 carbon atoms, an integer of 0 or 2 when W is a vinyl group, or an integer of 2 to 4 in other cases, are novel intermediates useful in the production of compounds of formula (1) and include compounds represented by the foregoing formulas (3), (5), (7), (9), (12) and (13).

The compounds of formula (1) produced by the above-described processes have a substituted alkyl group as the

substituent group attached to the 6-position of the pyrimidinedione ring. As a result, they not only show a marked improvement in chemical stability but also have excellent absorbability through apparatus digestorius (digestive system), and are hence useful as the active ingredient of novel Class III type antiarrhythmic compositions having an improved duration of action in vivo.

If desired, the compounds of formula (1) may be converted into pharmaceutically acceptable salts thereof. Such salts can be prepared, for example, by reacting the compounds of formula (1) with an inorganic acid such as hydrochloric acid, hydrobromic acid, phosphoric acid or sulfuric acid, or an organic acid such as formic acid, acetic acid, fumaric acid, citric acid, maleic acid, oxalic acid, malic acid, tartaric acid, methanesulfonic acid, benzenesulfonic acid or p-toluene sulfonic acid, in water or an organic solvent (such as an alcohol) or a mixture thereof.

Where the compounds of formula (1) and pharmaceutically acceptable salts thereof in accordance with the present invention are used as therapeutic agents for the treatment of patients with cardiac malfunctions such as arrhythmia and cardiac insufficiency, they may be administered orally or parenterally. Their doses and dosage forms may vary according to the properties of the compound of the present invention used as active ingredient and the symptoms presented by the patient to be treated.

Pharmaceutical compositions can be prepared according to well-known techniques.

Various dosage forms can be chosen according to the intended therapeutic purpose, and typical examples thereof include solid preparations, liquid preparations and other preparations such as suppositories. These dosage forms are more specifically described hereinbelow.

Solid preparations include tablets, pills, powders, granules, capsules and the like; liquid preparations include injectable solutions, suspensions, syrups, emulsions and the like; and other preparations include suppositories and the like.

In preparing the pharmaceutical compositions in the form of tablets, a wide variety of carriers conventionally known in this field can be used. Examples thereof include excipients such as lactose, sucrose, sodium chloride, glucose, urea, starch, calcium carbonate, kaolin, crystalline cellulose and silicic acid; binders such as water, ethanol, propanol, simple syrup, glucose solution, starch solution, gelatin solution, shellac solution, methylcellulose solution, hydroxypropylcellulose solution, polyvinyl pyrrolidone solution and carboxymethylcellulose solution; disintegrators such as dry starch, sodium alginate, agar powder, sodium hydrogen carbonate, calcium carbonate, polyoxyethylene sorbitan fatty acid esters, sodium lauryl sulfate, stearic acid monoglyceride, starch and lactose; disintegration inhibitors such as sucrose, stearic acid, cacao butter and hydrogenated oil; absorption promoters such as quaternary ammonium bases and sodium lauryl sulfate; humectants such as glycerin and starch; adsorbents such as starch, lactose, kaolin, bentonite, colloidal silicic acid, crystalline cellulose and light anhydrous silicic acid; and lubricants such as talc, stearates, boric acid powder and polyethylene glycol.

In the case of tablets, they can also be formed, as desired, into common coated tablets such as sugar-coated tablets, gelatin-coated tablets, enteric coated tablets and film-coated tablets, or into bilayer or multilayer tablets.

In preparing the pharmaceutical compositions in the form of pills, a wide variety of carriers conventionally known in this field can be used. Examples thereof include excipients such as glucose, lactose, starch, cacao butter, hardened vegetable oil, kaolin and talc; binders such as powdered acacia, powdered tragacanth and gelatin; and disintegrators such as carboxymethylcellulose calcium and agar.

Capsules are usually prepared according to conventional procedure, by mixing the compound used as active ingredient with some of the various carriers enumerated above and filling the resulting mixture into hard gelatin capsules, soft capsules or the like.

In preparing the pharmaceutical compositions in the form of injections or in the form of solutions, emulsions or suspensions, various diluents commonly used in this field can be used. Examples thereof include water, ethanol, macrogol, propylene glycol, ethoxylated isostearyl alcohol, polyoxylated isostearyl alcohol, polyoxyethylene sorbitan fatty acid esters, cotton seed oil, corn oil, peanut oil and olive oil. Moreover, the compounds of the present invention can also be used in the form of aqueous suspensions prepared by adding water thereto in the presence of a suitable surfactant, or in the form of emulsions prepared with the aid of a surfactant such as HCO-60. Furthermore, sodium chloride, glucose and/or glycerol may be contained in such pharmaceutical compositions, and common solubilizers, buffers and/or soothing agents may be added thereto.

In preparing the pharmaceutical compositions in the form of suppositories, a wide variety of conventionally known carriers can be used. Examples thereof include polyethylene glycol, cacao butter, higher alcohols, esters of higher alcohols, gelatin and semisynthetic glycerides.

If desired, the pharmaceutical compositions can further contain colorants, preservatives, perfumes, flavors, sweeteners and/or other drugs.

The amount of the compound used as active ingredient in the pharmaceutical compositions of the present invention is not particularly limited but may vary widely. However, the amount is usually in the range of about 1 to 70% by weight, preferably about 5 to 50% by weight, based on the total weight of the pharmaceutical composition.

No particular limitation is placed on the method of administration of the pharmaceutical compositions of the present invention, and they may be administered according to the form of the pharmaceutical composition used, the age, sex and other conditions of the patient, and the severity of the disease. For example, tablets, pills, solutions, suspensions,

emulsions, powders, granules, syrups and capsules are administered orally. Injections may be intravenously administered alone or in admixture with a common infusion fluid containing glucose, amino acids and the like. Alternatively, they may also be administered intramuscularly, subcutaneously or intraperitoneally as required. Suppositories are administered intrarectally.

The doses of the pharmaceutical compositions of the present invention may be suitably determined according to the method of administration, the age, sex and other conditions of the patient, and the severity of the disease. However, their doses should usually be determined so that the compound used as active ingredient is given in a daily dose of about 0.001 to 1,000 mg for adults. Moreover, it is desirable that each unit dosage form contains the compound used as active ingredient in an amount ranging from about 0.001 to 1,000 mg.

In order to illustrate the production, evaluation and testing of compounds of formula (1) in accordance with the present invention, the following examples are given. However, these examples are not to be construed to limit the invention. The illustrative compound numbers correspond to the compound numbers in Tables 5 & 6, respectively.

Example 1

Synthesis of ethyl 6-chloro-3-oxohexanoate.

Meldrum's acid (86.4 g) and pyridine (107 ml) were dissolved in dichloromethane (600 ml) and the resulting solution was cooled to -5°C. While its bulk temperature was maintained at -5 to -3°C, chlorobutyric acid chloride (75 ml) was added dropwise thereto over a period of 1-1/4 hours, i.e., 75 minutes. After this mixture was stirred at -3°C for an additional 2 hours, the bulk temperature of the reaction mixture was raised to 10°C.

The reaction mixture was washed with water (500 ml), 1N hydrochloric acid (500 ml x 2) and a saturated aqueous solution of sodium chloride (500 ml) in that order. Then, the reaction mixture was dried over magnesium sulfate and concentrated in vacuo. After the addition of ethanol (500 ml) to the residue, the resulting solution was heated under reflux for 2 hours and then concentrated in vacuo. The resulting oil was distilled under reduced pressure (b.p. 102-105°C/l mmHg) to obtain the desired compound (75 g).

Example 2

Synthesis of methyl 6-chloro-3-oxohexanoate.

The desired compound (b.p. 104-106°C/0.55 mmHg) was obtained by repeating the procedure of Example 1, except that methanol was used in place of ethanol.

Example 3

Synthesis of 6-(3-chloropropyl)-1,3-dimethyl-2,4(1H,3H)pyrimidinedione.

[Synthesis Process 1]

The ethyl 6-chloro-3-oxohexanoate (73 g) synthesized in Example 1 was mixed with dimethylurea (33.4 g) and p-toluenesulfonic acid (0.3 g), and this mixture was heated at 110°C under reduced pressure (30 mmHg). After cooling, the reaction mixture was sludged with ether, and the resulting precipitate was separated by filtration and washed with n-hexane to obtain a crude product (62 g). This crude product was purified by silica gel column chromatography (ethyl acetate/n-hexane = 1/1 to 2/1) to obtain the desired compound (46 g; m.p. 71-72.5°C).

[Synthesis Process 2]

The ethyl 6-chloro-3-oxohexanoate (0.58 g) synthesized in Example 1, together with dimethylurea (0.29 g) and p-toluenesulfonic acid (0.08 g), was dissolved in toluene (10 ml). This mixture was reacted for 5.5 hours by the application of heat while the resulting ethanol and water were being removed by azeotropic distillation. After being allowed to cool, the reaction mixture was concentrated. The residue was dissolved in chloroform, washed with a 1N aqueous solution of sodium hydroxide, dried over anhydrous sodium sulfate, and then concentrated. The residue obtained by the concentration was purified by silica gel column chromatography (ethyl acetate/n-hexane = 1/1) to obtain the desired compound (0.33 g).

Example 4

Synthesis of 6-(3-iodopropyl)-1,3-dimethyl-2,4(1H,3H)pyrimidinedione.

Sodium iodide (11.3 g) and the 6-(3-chloropropyl)-1,3-dimethyl-2,4(1H,3H)pyrimidinedione (5.45 g) synthesized in Example 3 were mixed with 2-butanone (100 ml), and this mixture was heated under reflux for 11 hours. After being

allowed to cool, the reaction mixture was concentrated. The residue was suspended in chloroform and extracted by the addition of ice water. The aqueous phase was extracted again with chloroform. The organic phases obtained from the respective extraction treatments were combined, dried over anhydrous sodium sulfate, and then concentrated to obtain the desired compound (7.8 g) as yellow crystals.

Example 5

Synthesis of 6-(3-chloropropyl)-1,3-diethyl-2,4(1H,3H)pyrimidinedione.

The ethyl 6-chloro-3-oxohexanoate (15.4 g) synthesized in Example 1 was mixed with diethylurea (9.76 g) and p-toluenesulfonic acid (0.15 g), and this mixture was heated at 110°C under reduced pressure (10 mmHg) for 9 hours. After cooling, the reaction mixture was dissolved in chloroform and extracted by the addition of a saturated aqueous solution of sodium bicarbonate. The aqueous phase was extracted again with chloroform. The organic phases obtained from the respective extraction treatments were combined, dried over anhydrous sodium sulfate, and then concentrated. The residue was purified by silica gel column chromatography (methanol/chloroform = 1/30) to obtain the desired compound (5.81 g) as a yellow oil.

Example 6

Synthesis of 6-(3-chloropropyl)-1,3-diisopropyl-2,4(1H,3H)pyrimidinedione.

[Synthesis Process 1]

The desired compound was obtained by repeating Synthesis Process 1 of Example 3, except that diisopropylurea was used in place of dimethylurea.

[Synthesis Process 2]

The ethyl 6-chloro-3-oxohexanoate (3.85 g) synthesized in Example 1, together with diisopropylurea (3.02 g) and p-toluenesulfonic acid (0.04 g), was dissolved in toluene (20 ml). This mixture was reacted for 5 hours by the application of heat while the resulting ethanol and water were being removed by azeotropic distillation. After being allowed to cool, the reaction mixture was concentrated. The residue was dissolved in chloroform and this solution was filtered to remove any insoluble matter. The filtrate was washed with a 1N aqueous solution of sodium hydroxide, dried over anhydrous sodium sulfate, and then concentrated. The residue was purified by silica gel column chromatography (ethyl acetate/n-hexane = 1/2) to obtain the desired compound (0.82 g) as a yellow oil.

Example 7

Synthesis of 6-(3-benzyloxypropyl)-1,3-dimethyl-2,4(1H,3H)pyrimidinedione.

Ethyl 4-benzyloxybutanoylacetate (8.8 g), which had been synthesized according to a well-known process (Can. J. Chem., 70, 1142(1992)), was mixed with dimethylurea (3.1 g) and p-toluenesulfonic acid (0.2 g), and this mixture was stirred at 110°C under reduced pressure (about 45 mmHg) for 17 hours.

After being allowed to cool, the brown reaction mixture was purified by silica gel column chromatography (ethyl acetate/n-hexane = 1/1) to obtain the desired compound (6.1 g) as a yellow oil.

Example 8

Synthesis of 6-(3-methoxycarbonyl)ethyl-1,3-dimethyl-2,4(1H,3H)pyrimidinedione.

A mixture of ethyl 5-methoxycarbonyl-3-oxopentanoate (27.9 g), dimethylurea (17.8 g) and p-toluenesulfonic acid (0.12 g) was stirred at 110°C under a reduced pressure of 35 mmHg for 3 hours.

The reaction mixture was brought back to room temperature and allowed to stand overnight so that crystals would separate out. The resulting crystals were dissolved in ethyl acetate and then recrystallized by the addition of n-hexane (20 ml). The crystals thus obtained were washed with a solvent mixture (ethyl acetate/n-hexane = 1/1) to obtain the desired compound (12.9 g; m.p. 104-106°C).

Example 9

Synthesis of 6-(3-hydroxypropyl)-1,3-dimethyl-2,4(1H,3H)pyrimidinedione.

[Synthesis Process 1]

The 6-(3-benzyloxypropyl)-1,3-dimethyl-2,4(1H,3H)-pyrimidinedione (1.8 g) synthesized in Example 7 was dissolved in 40 ml of ethanol and subjected to atmospheric hydrogenation for an hour in the presence of Pd/C (0.2 g; purity 10%). After completion of the reaction, the catalyst was separated by filtration and the filtrate was concentrated to obtain the desired compound (0.94 g) as an oil.

[Synthesis Process 2]

The 6-(3-methoxycarbonylethyl)-1,3-dimethyl-2,4(1H,3H)pyrimidinedione (3 g) synthesized in Example 8 was dissolved in dry THF (60 ml) and this solution was added dropwise at room temperature to a suspension of lithium boron hydride (0.35 g) in dry THF (30 ml). Then, this mixed solution was stirred at 55°C for 7 hours and allowed to stand overnight. Thereafter, the reaction mixture was mixed with water (15 ml) and acidified by adding a 5N solution of hydrochloric acid dropwise thereto. The resulting solution was freed of THF by vacuum distillation and then extracted six times with chloroform. The organic phases obtained from the respective extraction treatments were combined, dried over anhydrous sodium sulfate, and then concentrated in vacuo to obtain the desired compound (2.33 g).

[Synthesis Process 3]

6-Allyl-1,3-dimethyl-2,4(1H,3H)pyrimidinedione (0.29 g), which was synthesized according to a well-known process (J. Org. Chem., 51, 5148(1986)), was dissolved in dry THF. Then, 9-BBN dimer (0.39 g) was added to the resulting solution under cooling with ice. After this mixture was stirred at room temperature for 26 hours, water was added thereto and a 3N aqueous solution of sodium hydroxide (4 ml) was further added thereto under cooling with ice. After the lapse of 20 minutes, an aqueous hydrogen peroxide solution (35%) was added dropwise thereto. The resulting mixture was brought back to room temperature, stirred and then allowed to stand overnight.

Thereafter, the reaction mixture was extracted three times with ethyl acetate. The organic phases obtained from the respective extraction treatments were combined, dried over anhydrous sodium sulfate, and then concentrated. The residue was purified by silica gel column chromatography (methanol/chloroform = 1/50) to obtain the desired compound (0.23 g) as a colorless oil.

Example 10

Synthesis of 6-methoxycarbonylmethyl-1,3-dimethyl-2,4(1H, 3H)pyrimidinedione.

1,3-Dimethylurea (50.5 g) was dissolved in dimethyl 1,3-acetonedicarboxylate (100 g), followed by the addition of p-toluenesulfonic acid (0.34 g). Then, the resulting mixture was stirred at 110°C under reduced pressure (about 30 mmHg) for 5 hours.

After the reaction mixture was allowed to cool, the crystals so precipitated were collected by filtration and washed with ether to obtain the desired compound (52.5 g) as white crystals.

Example 11

Synthesis of 6-(2-hydroxyethyl)-1,3-dimethyl-2,4(1H,3H)pyrimidinedione.

The 6-methoxycarbonylmethyl-1,3-dimethyl-2,4(1H,3H)-pyrimidinedione (10 g) synthesized in Example 10, together with calcium chloride (7.4 g), was dissolved in methanol (200 ml). While the resulting solution was cooled to and maintained at -5°C, sodium boron hydride (20 g) was added slowly thereto.

After the resulting mixture was stirred for an hour under cooling with ice, water (30 ml) was added thereto and the reaction was stopped by the addition of a small amount of 2N hydrochloric acid. The reaction mixture was concentrated in vacuo and the residue was extracted six times with chloroform. The organic phases obtained from the respective extraction treatments were combined, dried over anhydrous sodium sulfate, and then concentrated to obtain the desired compound (6 g) as a colorless oil. This oil crystallized in isopropanol/ether (m.p. 91-94°C).

Example 12

Synthesis of 6-[3-[N-ethyl-N-(2-hydroxyethyl)amino]-propyl]-1,3-dimethyl-2,4(1H,3H)pyrimidinedione.

The 6-(3-chloropropyl)-1,3-dimethyl-2,4(1H,3H)pyrimidinedione (7.4 g) synthesized in Example 3, 2-ethylaminoethanol (4.3 ml), triethylamine (6.1 ml) and sodium iodide (6 g) were added to dioxane (100 ml), and the resulting suspension was heated under reflux for 5.5 hours. After the solvent was removed by distillation, a 1N aqueous solution of sodium hydroxide (100 ml) was added to the residue and this mixture was extracted with chloroform (100 ml x 4). The organic phases obtained from the respective extraction treatments were combined, dried over magnesium sulfate, and

then concentrated to obtain the desired compound (9.1 g) as an oil.

Example 13

Synthesis of 6-[3-[N-(2-hydroxyethyl)-N-methylamino]-propyl]-1,3-dimethyl-2,4(1H,3H)pyrimidinedione.

The desired compound was obtained by repeating the procedure of Example 12, except that 2-methylaminoethanol was used in place of 2-ethylaminoethanol.

Example 14

Synthesis of 6-[3-[N-(2-hydroxyethyl)-N-propylamino]-propyl]-1,3-dimethyl-2,4(1H,3H)pyrimidinedione.

The desired compound was obtained by repeating the procedure of Example 12, except that 2-propylaminoethanol was used in place of 2-ethylaminoethanol.

Example 15

Synthesis of 6-[3-[N-(2-hydroxyethyl)-N-isopropylamino]propyl]-1,3-dimethyl-2,4(1H,3H)pyrimidinedione.

The desired compound was obtained by repeating the procedure of Example 12, except that 2-isopropylaminoethanol was used in place of 2-ethylaminoethanol.

Example 16

Synthesis of 6-[2-[N-(2-hydroxyethyl)-N-methylamino]-ethyl]-1,3-dimethyl-2,4(1H,3H)pyrimidinedione.

The 1,3-dimethyl-6-(2-hydroxyethyl)-2,4(1H,3H)pyrimidinedione (2.6 g) synthesized in Example 11, p-toluenesulfonyl chloride (3 g) and triethylamine (2.2 g) were added to dichloromethane (20 ml), and the resulting mixture was stirred at room temperature for 2 hours and allowed to stand overnight.

After standing, the reaction mixture was concentrated in vacuo and dissolved in dioxane (20 ml). Then, 2-methylaminoethanol (6.4 g) was added dropwise to the resulting solution at room temperature. Thereafter, the resulting mixture was heated under reflux for 4 hours and the reaction mixture was concentrated in vacuo. The residue was extracted three times with chloroform/2N hydrochloric acid. The aqueous phases obtained from the respective extraction treatments were combined, adjusted to pH 14 with a 2N aqueous solution of sodium hydroxide, and then extracted three times with chloroform. The organic phases obtained from the respective extraction treatments were combined, dried over anhydrous sodium sulfate, and then concentrated in vacuo to obtain the desired compound (2.23 g) as a yellowish-brown oil.

Example 17

Synthesis of 6-[2-[N-ethyl-N-(2-hydroxyethyl)amino]-ethyl]-1,3-dimethyl-2,4(1H,3H)pyrimidinedione.

The desired compound was obtained by repeating the procedure of Example 16, except that 2-ethylaminoethanol was used in place of 2-methylaminoethanol.

Example 18

Synthesis of 6-[2-[N-(2-hydroxyethyl)-N-propylamino]-ethyl]-1,3-dimethyl-2,4(1H,3H)pyrimidinedione.

The desired compound was obtained by repeating the procedure of Example 16, except that 2-propylaminoethanol was used in place of 2-methylaminoethanol.

Example 19

Synthesis of 6-[2-[N-(2-hydroxyethyl)-N-isopropylamino]ethyl]-1,3-dimethyl-2,4(1H,3H)pyrimidinedione.

The desired compound was obtained by repeating the procedure of Example 16, except that 2-isopropylaminoethanol was used in place of 2-methylaminoethanol.

Example 20

Synthesis of 6-[3-[N-ethyl-N-(3-hydroxypropyl)amino]-propyl]-1,3-dimethyl-2,4(1H,3H)pyrimidinedione.

The 6-(3-chloropropyl)-1,3-dimethyl-2,4(1H,3H)-pyrimidinedione (4.4 g) synthesized in Example 3, 3-ethylaminopropanol (2.3 g), sodium iodide (3 g) and triethylamine (3.2 ml) were added to dioxane (50 ml), and the resulting mixture was heated under reflux for 8 hours.

After being allowed to cool, the orange reaction mixture was concentrated, and the residue was dissolved in chloroform and washed with a 5% aqueous solution of sodium hydroxide. The wash liquid (aqueous phase) was extracted again with chloroform. The respective organic phases were combined, dried over anhydrous sodium sulfate, and then concentrated to obtain the desired compound (5.3 g) as an orange oil.

Example 21

Synthesis of 6-(3-ethylaminopropyl)-1,3-dimethyl-2,4(1H,3H)pyrimidinedione.

The 6-(3-chloropropyl)-1,3-dimethyl-2,4(1H,3H)pyrimidinedione (0.5 g) synthesized in Example 3 was dissolved in dioxane (5 ml), followed by the addition of ethylamine (70% aqueous solution; 2.97 g). The resulting mixture was refluxed with stirring for 24 hours. The reaction mixture was concentrated in vacuo, and the residue was mixed with chloroform and washed with a 1N aqueous solution of sodium hydroxide. The washed organic phase was dried over anhydrous sodium sulfate and concentrated in vacuo to obtain the desired compound (0.5 g) as a liver brown oil.

Example 22

Synthesis of 6-[3-(N-methoxyethylamino)propyl]-1,3-dimethyl-2,4(1H,3H)pyrimidinedione.

The 6-(3-chloropropyl)-1,3-dimethyl-2,4(1H,3H)pyrimidinedione (1.52 g) synthesized in Example 3, methoxyethylamine (3 ml) and sodium iodide (1.26 g) were added to dioxane (30 ml), and the resulting mixture was heated under reflux for 5.5 hours.

After being allowed to cool, the yellow reaction mixture was concentrated, and the residue was dissolved in chloroform and extracted by the addition of 1N hydrochloric acid. The aqueous phase was alkalified with a 5% aqueous solution of sodium hydroxide and extracted twice with chloroform. The organic phases obtained from the respective extraction treatments were combined, dried over anhydrous sodium sulfate, and then concentrated to obtain the desired compound (1.65 g) as a yellow oil.

Example 23

Synthesis of 6-(2-ethylaminoethyl)-1,3-dimethyl-2,4(1H,3H)pyrimidinedione.

The 6-(2-hydroxyethyl)-1,3-dimethyl)-2,4(1H,3H)pyrimidinedione (3.73 g) synthesized in Example 11, p-toluenesulfonyl chloride (4.25 g) and triethylamine (3.08 g) were added to dichloromethane (30 ml), and the resulting mixture was stirred at room temperature for 2 hours and allowed to stand overnight. After standing, the reaction solution was concentrated in vacuo and dissolved in dioxane (30 ml). Then, ethylamine (70% aqueous solution; 13.1 g) was added dropwise to the resulting solution at room temperature. Thereafter, the resulting solution was stirred at room temperature for 10 minutes and the reaction solution was concentrated in vacuo. The residue was extracted three times with chloroform/2N hydrochloric acid. The aqueous phases obtained from the respective extraction treatments were combined, adjusted to pH 12 with a 2N aqueous solution of sodium hydroxide, and then extracted three times with chloroform. The organic phases obtained from the respective extraction treatments were combined, dried over anhydrous sodium sulfate, and then concentrated in vacuo to obtain the desired compound (2.55 g) as a yellow oil.

Example 24

Synthesis of 1,3-dimethyl-6-(3-phthaloylamino)propyl-2,4(1N,3H)pyrimidinedione.

[Synthesis Process 1]

The 6-(3-hydroxypropyl)-1,3-dimethyl-2,4(1H,3H)-pyrimidinedione (1.69 g) synthesized in Example 9, phthalimide (1.38 g) and triphenylphosphine (2.47 g) were dissolved in THF (40 ml), following by stirring. Then, a solution of diethyl azodicarboxylate (1.63 g) in THF (15 ml) was added dropwise thereto under cooling with water.

After completion of the addition, the resulting mixture was stirred at room temperature for 30 minutes, so that crystals separated out. These crystals were collected by filtration, washed with THF, and then dried to obtain the desired compound (0.96 g) as white crystals (m.p. 194-196°C).

[Synthesis Process 2]

The 6-(3-chloropropyl)-1,3-dimethyl-2,4(1H,3H)-pyrimidinedione (10 g) synthesized in Example 3 and potassium phthalimide (8.98 g) were dissolved in DMF (40 ml), and the resulting solution was heated at 80°C with stirring for 6 hours.

After the reaction solution was allowed to cool, chloroform (100 ml) was added thereto and the resulting mixture

was washed with water. The washed organic phase was dried over anhydrous sodium sulfate and concentrated. The residue obtained by the concentration was washed with n-hexane to obtain the desired compound (12.3 g) as white powder.

Example 25

Synthesis of 6-(3-aminopropyl)-1,3-dimethyl-2,4(1H,3H)pyrimidinedione.

The 1,3-dimethyl-6-(3-phthaloylamino)propyl-2,4(1H,3H)pyrimidinedione (4.07 g) synthesized in Example 24 was dissolved in ethanol (120 ml), and hydrazine monohydrate (1.87 g) was added dropwise to the resulting solution. This solution was stirred at 70°C for 2.5 hours, concentrated in vacuo, alkalified by the addition of a 2N aqueous solution of sodium hydroxide, and then extracted six times with chloroform. The organic phases obtained from the respective extraction treatments were combined, washed once with a saturated aqueous solution of sodium chloride, dried over anhydrous sodium sulfate, and then concentrated to obtain the desired compound (2.56 g).

Example 26

Synthesis of 1,3-dimethyl-6-[3-(p-toluenesulfonyloxy)propyl]-2,4(1H,3H)pyrimidinedione.

The 6-(3-hydroxypropyl)-1,3-dimethyl-2,4(1H,3H)-pyrimidinedione (3.95 g) synthesized in Example 9 and triethylamine (6.1 ml) were added to dichloromethane (60 ml), and p-toluenesulfonyl chloride (4.2 g) was added to the resulting solution under cooling with ice. This solution was warmed to room temperature and then stirred for 10 hours. After the reaction mixture was concentrated, a 10% aqueous solution of potassium carbonate was added to the residue. The resulting mixture was extracted with chloroform, and the organic phase was washed with 1N hydrochloric acid, dried over anhydrous sodium sulfate, and then concentrated. The residue was recrystallized from n-hexane/ethyl acetate to obtain the desired compound (4.9 g) as white crystals.

Example 27 (Illustrative Compound No. 612)

Synthesis of 1,3-dimethyl-6-[3-[N-isopropyl-N-[2-(4-nitrophenoxy)ethyl]amino]propyl]-2,4(1H,3H)pyrimidinedione.

[Synthesis Process 1]

The 6-[3-[N-(2-hydroxyethyl)-N-isopropylamino]-propyl]-1,3-dimethyl-2,4(1H,3H)pyrimidinedione (0.95 g) synthesized in Example 15 and 4-nitrophenol (0.51 g) were dissolved in dry THF (20 ml), and triphenylphosphine (0.97 g) was added to the resulting solution at room temperature. Then, in an atmosphere of nitrogen, a solution of diethyl azodicarboxylate (0.64 g) in dry THF (5 ml) was slowly added dropwise thereto under cooling with water. After being stirred at room temperature for an hour, the reaction solution was concentrated and the residue was extracted three times with chloroform/2N hydrochloric acid. The aqueous phases obtained from the respective extraction treatments were combined, adjusted to pH 14 with a 2N aqueous solution of sodium hydroxide, and then extracted three times with chloroform. The organic phases obtained from the respective extraction treatments were combined, dried over anhydrous sodium sulfate, and then concentrated. The residue was purified by silica gel column chromatography (chloroform/methanol = 50/1) to obtain the desired compound (0.55 g) as a pale-yellow oil.

[Synthesis Process 2]

N-isopropyl-2-(4-nitrophenoxy)ethylamine (3.58 g) synthesized according to conventional procedure, the 6-(3-chloropropyl)-1,3-dimethyl-2,4(1H,3H)pyrimidinedione (4.16 g) synthesized in Example 3, sodium iodide (2.88 g) and triethylamine (4.5 ml) were added to dioxane (50 ml), and the resulting suspension was heated under reflux for 14.5 hours.

After being allowed to cool, the yellow reaction mixture was concentrated, and the residue was diluted with chloroform and washed with a 5% aqueous solution of sodium hydroxide. The wash liquid (aqueous phase) was separated and further extracted with chloroform. The combined chloroform phases were dried over anhydrous sodium sulfate and then concentrated. The residue was purified by silica gel column chromatography (methanol/chloroform = 1/50) to obtain the desired compound (2.66 g) as a yellow oil.

Example 28 (Illustrative Compound No. 610)

Synthesis of 6-[3-[N-ethyl-N-[2-(4-nitrophenoxy)ethyl]amino]propyl]-1,3-dimethyl-2,4(1H,3H)pyrimidinedione.

The 6-[3-[N-ethyl-N-(2-hydroxyethyl)amino]propyl]-1,3-dimethyl-2,4(1H,3H)pyrimidinedione (2.05 g) synthesized in Example 12, 4-nitrophenol (1.06 g) and triphenylphosphine (2.2 g) were dissolved in THF (20 ml), and diethyl azodicarboxylate (1.3 ml) was added dropwise to the resulting solution at room temperature. After being stirred at room temperature for an hour, the orange reaction mixture was concentrated in vacuo. The residue was purified by silica gel column chromatography to obtain the desired compound (2.24 g) as yellow crystals.

Subsequently, this pyrimidinedione derivative was treated with a methanolic solution of hydrochloric acid according to conventional procedure to obtain the hydrochloride (m.p. 77-80°C) of the desired compound.

Example 29 (Illustrative Compound No. 609)

Synthesis of 6-[3-[N-methyl-N-[2-(4-nitrophenoxy)ethyl]amino]propyl]-1,3-dimethyl-2,4(1H,3H)pyrimidinedione.

The 6-[3-[N-(2-hydroxyethyl)-N-methylamino]propyl]-1,3-dimethyl-2,4(1H,3H)pyrimidinedione (3 g) synthesized in Example 13 and 4-nitrophenol (1.8 g) were dissolved in dry THF (50 ml), and triphenylphosphine (1.8 g) was added to the resulting solution at room temperature. Further, in an atmosphere of nitrogen, a solution of diethyl azodicarboxylate (3.4 g) in dry THF (10 ml) was slowly added dropwise thereto under cooling with water. After being stirred at room temperature for an hour, the reaction solution was concentrated in vacuo. The residue was purified by silica gel column chromatography (chloroform/methanol = 20/1) to obtain the desired compound (1.1 g) as pale-yellow crystals.

Subsequently, this pyrimidinedione derivative was treated with a methanolic solution of hydrochloric acid according to conventional procedure to obtain the hydrochloride [m.p. 236-240°C (dec.)] of the desired compound.

Example 30 (Illustrative Compound No. 611)

Synthesis of 1,3-dimethyl-6-[3-[N-[2-(4-nitrophenoxy)ethyl]-N-propylamino]propyl]-2,4(1H,3H)pyrimidinedione.

The 6-[3-[N-(2-hydroxyethyl)-N-propylamino]propyl]-1,3-dimethyl-2,4(1H,3H)pyrimidinedione (2.5 g) synthesized in Example 14 and 4-nitrophenol (1.35 g) were dissolved in dry THF (30 ml), and triphenylphosphine (2.55 g) was further added to the resulting solution at room temperature. Then, in an atmosphere of nitrogen, a solution of diethyl azodicarboxylate (1.7 g) in dry THF (10 ml) was slowly added dropwise thereto under cooling with water. After being stirred at room temperature for 30 minutes, the reaction solution was concentrated in vacuo.

The resulting concentrate was extracted three times with chloroform/2N hydrochloric acid. The aqueous phases obtained from the respective extraction treatments were combined, adjusted to pH 14 with a 2N aqueous solution of sodium hydroxide, and then extracted three times with chloroform. The organic phases obtained from the respective extraction treatments were combined, dried over anhydrous sodium sulfate, and then concentrated. The residue was purified by silica gel column chromatography (chloroform/methanol = 25/1) to obtain the desired compound (1.9 g) as a pale-yellow oil.

Example 31 (Illustrative Compound No. 622)

Synthesis of 6-[3-[N-ethyl-N-[3-(4-nitrophenoxy)propyl]amino]propyl]-1,3-dimethyl-2,4(1H,3H)pyrimidinedione.

The 6-[3-[N-ethyl-N-(2-hydroxypropyl)amino]propyl]-1,3-dimethyl-2,4(1H,3H)pyrimidinedione (2 g) synthesized in Example 20, 4-nitrophenol (0.89 g) and triphenylphosphine (1.86 g) were dissolved in THF (10 ml), and diethyl azodicarboxylate (1.24 g) was added dropwise to the resulting solution at room temperature. After being stirred at room temperature for an hour, the reaction mixture was concentrated. The residue was purified by silica gel column chromatography (methanol/chloroform = 1/20) to obtain the desired compound (1.21 g) as a yellow oil.

Example 32 (Illustrative Compound No. 604)

Synthesis of 6-[2-[N-ethyl-N-[2-(4-nitrophenoxy)ethyl]amino]ethyl]-1,3-dimethyl-2,4(1H,3H)pyrimidinedione.

The 6-[2-[N-ethyl-N-(2-hydroxyethyl)amino]ethyl]-1,3-dimethyl-2,4(1H,3H)pyrimidinedione (0.96 g) synthesized in Example 17 and 4-nitrophenol (0.58 g) were dissolved in dry THF (20 ml), and triphenylphosphine (1.1 g) was added to the resulting solution at room temperature. Further, in an atmosphere of nitrogen, a solution of diethyl azodicarboxylate (0.72 g) in dry THF (5 ml) was slowly added dropwise thereto under cooling with water. After being stirred at room temperature for an hour, the reaction solution was concentrated in vacuo. The residue was purified by silica gel column chromatography (chloroform/methanol = 50/1) to obtain the desired compound (0.68 g) as a colorless oil.

Subsequently, this pyrimidinedione derivative was treated with a methanolic solution of hydrochloric acid according to conventional procedure to obtain the hydrochloride (m.p. 196-197°C) of the desired compound.

Example 33 (Illustrative Compound No. 603)

Synthesis of 6-[2-[N-methyl-N-[2-(4-nitrophenoxy)ethyl]amino]ethyl]-1,3-dimethyl-2,4(1H,3H)pyrimidinedione.

The 6-[2-[N-(2-hydroxyethyl)-N-methylamino]ethyl]-1,3-dimethyl-2,4(1H,3H)pyrimidinedione (2.23 g) synthesized in Example 16 and 4-nitrophenol (1.52 g) were dissolved in dry THF (50 ml), and triphenylphosphine (2.86 g) was

added to the resulting solution at room temperature. Further, in an atmosphere of nitrogen, a solution of diethyl azodicarboxylate (1.9 g) in dry THF (10 ml) was slowly added dropwise thereto under cooling with water. After being stirred at room temperature for an hour, the reaction solution was concentrated in vacuo. The residue was purified by silica gel column chromatography (chloroform/methanol = 50/1) to obtain the desired compound (1.17 g) as pale-yellow crystals.

Example 34 (Illustrative Compound No. 606)

Synthesis of 6-[2-[N-isopropyl-N-[2-(4-nitrophenoxy)-ethyl]amino]ethyl]-1,3-dimethyl-2,4(1H,3H)pyrimidinedione.

The 6-[2-[N-(2-hydroxyethyl)-N-isopropylamino]ethyl]-1,3-dimethyl-2,4(1H,3H)pyrimidinedione (0.74 g) synthesized in Example 19 and 4-nitrophenol (0.42 g) were dissolved in dry THF (20 ml), and triphenylphosphine (0.8 g) was added to the resulting solution at room temperature. Further, in an atmosphere of nitrogen, a solution of diethyl azodicarboxylate (0.53 g) in dry THF (5 ml) was slowly added dropwise thereto under cooling with water. After being stirred at room temperature for 30 minutes, the reaction solution was concentrated in vacuo. The residue was extracted three times with chloroform/2N hydrochloric acid. The aqueous phases obtained from the respective extraction treatments were combined, adjusted to pH 14 with a 2N aqueous solution of sodium hydroxide, and then extracted three times with chloroform. The organic phases obtained from the respective extraction treatments were combined, dried over anhydrous sodium sulfate, and then concentrated. The residue was purified by silica gel column chromatography (chloroform/methanol = 50/1) to obtain the desired compound (0.46 g) as a colorless oil.

Example 35 (Illustrative Compound No. 605)

Synthesis of 1,3-dimethyl-6-[2-[N-[2-(4-nitrophenoxy)ethyl]-N-propylamino]ethyl]-2,4(1H,3H)pyrimidinedione.

The 6-[2-[N-(2-hydroxyethyl)-N-propylamino]ethyl]-1,3-dimethyl-2,4(1H,3H)pyrimidinedione (0.56 g) synthesized in Example 18 and 4-nitrophenol (0.32 g) were dissolved in dry THF (10 ml), and triphenylphosphine (0.6 g) was added to the resulting solution at room temperature. Further, in an atmosphere of nitrogen, a solution of diethyl azodicarboxylate (0.4 g) in dry THF (5 ml) was slowly added dropwise thereto under cooling with water. After being stirred at room temperature for 30 minutes, the reaction solution was concentrated in vacuo. The residue was extracted three times with chloroform/2N hydrochloric acid. The aqueous phases obtained from the respective extraction treatments were combined, adjusted to pH 14 with a 2N aqueous solution of sodium hydroxide, and then extracted three times with chloroform. The organic phases obtained from the respective extraction treatments were combined, dried over anhydrous sodium sulfate, and then concentrated. The residue was purified by silica gel column chromatography (chloroform/methanol = 33/1) to obtain the desired compound (1.1 g) as a colorless oil.

Example 36 (Illustrative Compound No. 608)

Synthesis of 1,3-dimethyl-6-[3-[N-[2-(4-nitrophenoxy)ethyl]amino]propyl]-2,4(1H,3H)pyrimidinedione.

2-(4-nitrophenoxy)ethyl p-toluenesulfonate (5.06 g), the 6-(3-aminopropyl)-1,3-dimethyl-2,4(1H,3H)pyrimidinedione (3.25 g) synthesized in Example 25 and triethylamine (3.03 g) were dissolved in dioxane (70 ml), and the resulting solution was refluxed with stirring for 27.5 hours. After the solvent was removed at reduced pressure, the residue was dissolved in chloroform, and 1N hydrochloric acid was added to the resulting solution. The aqueous phase was separated, adjusted to pH 10-11 with sodium hydroxide, and then extracted with chloroform. The respective chloroform phases were combined, dried over anhydrous sodium sulfate, and then concentrated in vacuo. The residue was purified by silica gel column chromatography (chloroform/methanol = 10/1) to obtain the desired compound (3.29 g) as yellow crystals.

Example 37 (Illustrative Compound No. 1212)

Synthesis of 6-[3-[N-[2-(4-chlorophenoxy)ethyl]-N-ethylamino]propyl]-1,3-dimethyl-2,4(1H,3H)pyrimidinedione.

The procedure of Example 28 was repeated, except that 4-chlorophenol was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 158-161°C) of the desired compound.

Example 38 (Illustrative Compound No. 1208)

Synthesis of 6-[3-[N-ethyl-N-[2-(3-fluorophenoxy)ethyl]amino]propyl]-1,3-dimethyl-2,4(1H,3H)pyrimidinedione.

The procedure of Example 28 was repeated, except that 3-fluorophenol was used in place of 4-nitrophenol. Thus, the desired compound was obtained as a yellow oil.

Subsequently, this pyrimidinedione derivative was treated with a methanolic solution of hydrochloric acid according

to conventional procedure to obtain the hydrochloride (m.p. 115-123°C) of the desired compound.

Example 39 (Illustrative Compound No. 1209)

Synthesis of 6-[3-[N-ethyl-N-[2-(4-fluorophenoxy)ethyl]amino]propyl]-1,3-dimethyl-2,4(1H,3H)pyrimidinedione.

The procedure of Example 28 was repeated, except that 4-fluorophenol was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 156-157°C) of the desired compound.

Example 40 (Illustrative Compound No. 1207)

Synthesis of 6-[3-[N-ethyl-N-[2-(2-fluorophenoxy)ethyl]amino]propyl]-1,3-dimethyl-2,4(1H,3H)pyrimidinedione.

The procedure of Example 28 was repeated, except that 2-fluorophenol was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 146-149°C) of the desired compound.

Example 41 (Illustrative Compound No. 1185)

Synthesis of 6-[3-[N-[2-(3-fluorophenoxy)ethyl]-N-methylamino]propyl]-1,3-dimethyl-2,4(1H,3H)pyrimidinedione.

The procedure of Example 29 was repeated, except that 3-fluorophenol was used in place of 4-nitrophenol. Thus, the desired compound was obtained as pale-yellow crystals.

Subsequently, this pyrimidinedione derivative was treated with a methanolic solution of hydrochloric acid according to conventional procedure to obtain the hydrochloride (m.p. 165.5-168°C) of the desired compound.

Example 42 (Illustrative Compound No. 1186)

Synthesis of 6-[3-[N-[2-(4-fluorophenoxy)ethyl]-N-methylamino]propyl]-1,3-dimethyl-2,4(1H,3H)pyrimidinedione.

The procedure of Example 29 was repeated, except that 4-fluorophenol was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 106-111°C) of the desired compound.

Example 43 (Illustrative Compound No. 1213)

Synthesis of 6-[3-[N-[2-(4-cyanophenoxy)ethyl]-N-ethylamino]propyl]-1,3-dimethyl-2,4(1H,3H)pyrimidinedione.

The procedure of Example 28 was repeated, except that 4-cyanophenol was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 160-164°C) of the desired compound.

Example 44 (Illustrative Compound No. 1216)

Synthesis of 6-[3-[N-[2-(4-acetylphenoxy)ethyl]-N-ethylamino]propyl]-1,3-dimethyl-2,4(1H,3H)pyrimidinedione.

The procedure of Example 28 was repeated, except that 4-hydroxyacetophenone was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 125-128°C) of the desired compound.

Example 45 (Illustrative Compound No. 1214)

Synthesis of 6-[3-[N-[2-(2-acetylphenoxy)ethyl]-N-ethylamino]propyl]-1,3-dimethyl-2,4(1H,3H)pyrimidinedione.

The procedure of Example 28 was repeated, except that 2-hydroxyacetophenone was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 155.5-157°C) of the desired compound.

Example 46 (Illustrative Compound No. 1193)

Synthesis of 6-[3-[N-[2-(4-acetylphenoxy)ethyl]-N-methylamino]propyl]-1,3-dimethyl-2,4(1H,3H)pyrimidinedione.

The procedure of Example 29 was repeated, except that 4-hydroxyacetophenone was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 148-153°C) of the desired compound.

Example 47 (Illustrative Compound No. 1239)

Synthesis of 6-[3-[N-[2-(4-acetylphenoxy)ethyl]-N-propylamino]propyl]-1,3-dimethyl-2,4(1H,3H)pyrimidinedione.

The procedure of Example 30 was repeated, except that 4-hydroxyacetophenone was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 107-110°C) of the desired compound.

Example 48 (Illustrative Compound No. 1262)

Synthesis of 6-[3-[N-[2-(4-acetylphenoxy)ethyl]-N-isopropylamino]propyl]-1,3-dimethyl-2,4(1H,3H)-pyrimidinedione.

The procedure of Example 27 was repeated, except that 4-hydroxyacetophenone was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 193-194°C) of the desired compound.

Example 49 (Illustrative Compound No. 1221)

Synthesis of 6-[3-[N-ethyl-N-[2-(4-trifluoromethylphenoxy)ethyl]amino]propyl]-1,3-dimethyl-2,4(1H,3H)-pyrimidinedione.

The procedure of Example 28 was repeated, except that 4-trifluoromethylphenol was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 110-111.5°C) of the desired compound.

Example 50 (Illustrative Compound No. 1222)

Synthesis of 6-[3-[N-ethyl-N-[2-(4-trifluoromethoxyphenoxy)ethyl]amino]propyl]-1,3-dimethyl-2,4(1H,3H)pyrimidinedione.

The procedure of Example 28 was repeated, except that 4-trifluoromethoxyphenol was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 122-128°C) of the desired compound.

Example 51 (Illustrative Compound No. 1217)

Synthesis of 6-[3-[N-[2-(4-benzoylphenoxy)ethyl]-N-ethylamino]propyl]-1,3-dimethyl-2,4(1H,3H)pyrimidinedione.

The procedure of Example 28 was repeated, except that 4-hydroxybenzophenone was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 95-97°C) of the desired compound.

Example 52 (Illustrative Compound No. 1223)

Synthesis of 6-[3-[N-ethyl-N-[2-(4-(1H-imidazol-1-yl)phenoxy)ethyl]amino]propyl]-1,3-dimethyl-2,4(1H,3H)pyrimidinedione.

The procedure of Example 28 was repeated, except that 4-(1H-imidazol-1-yl)phenol was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride [m.p. 133-141°C (dec.)] of the desired compound.

Example 53 (Illustrative Compound No. 1269)

Synthesis of 6-[3-[N-[2-(4-(1H-imidazol-1-yl)phenoxy)ethyl]-N-isopropylamino]propyl]-1,3-dimethyl-2,4(1H,3H)pyrimidinedione.

The procedure of Example 27 was repeated, except that 4-(1H-imidazol-1-yl)phenol was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride [m.p. 141-146°C (dec.)] of the desired compound.

Example 54 (Illustrative Compound No. 1246)

Synthesis of 6-[3-[N-[2-(4-(1H-imidazol-1-yl)phenoxy)ethyl]-N-propylamino]propyl]-1,3-dimethyl-2,4(1H,3H)pyrimidinedione.

The procedure of Example 30 was repeated, except that 4-(1H-imidazol-1-yl)phenol was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 110-113°C) of the desired compound.

Example 55 (Illustrative Compound No. 1200)

Synthesis of 6-[3-[N-[2-(4-(1H-imidazol-1-yl)phenoxy)ethyl]-N-methylamino]propyl]-1,3-dimethyl-2,4(1H,3H)pyrimidinedione.

The procedure of Example 29 was repeated, except that 4-(1H-imidazol-1-yl)phenol was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 155-160°C) of the desired compound.

Example 56 (Illustrative Compound No. 1131)

Synthesis of 6-[2-[N-ethyl-N-[2-[4-(1H-imidazol-1-yl)phenoxy]ethyl]amino]ethyl]-1,3-dimethyl-2,4(1H,3H)-pyrimidinedione.

The procedure of Example 32 was repeated, except that 4-(1H-imidazol-1-yl)phenol was used in place of 4-nitrophenol. Thus, the desired compound was obtained as a colorless oil.

Example 57 (Illustrative Compound No. 1225)

Synthesis of 6-[3-[N-[2-(2-acetyl-4-chlorophenoxy)ethyl]-N-ethylamino]propyl]-1,3-dimethyl-2,4(1H,3H)pyrimidinedione.

The procedure of Example 28 was repeated, except that 5-chloro-2-hydroxyacetophenone was used in place of 4-nitrophenol. Thus, the desired compound was obtained as white crystals.

Subsequently, this pyrimidinedione derivative was treated with a methanolic solution of hydrochloric acid according to conventional procedure to obtain the hydrochloride (m.p. 159-161°C) of the desired compound.

Example 58 (Illustrative Compound No. 1226)

Synthesis of 6-[3-[N-[2-(2-acetyl-4-fluorophenoxy)ethyl]-N-ethylamino]propyl]-1,3-dimethyl-2,4(1H,3H)pyrimidinedione.

The procedure of Example 28 was repeated, except that 5-fluoro-2-hydroxyacetophenone was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 180-181°C) of the desired compound.

Example 59 (Illustrative Compound No. 1228)

Synthesis of 6-[3-[N-[2-(4-acetyl-2-fluorophenoxy)ethyl]-N-ethylamino]propyl]-1,3-dimethyl-2,4(1H,3H)pyrimidinedione.

The procedure of Example 28 was repeated, except that 3-fluoro-4-hydroxyacetophenone was used in place of 4-nitrophenol. Thus, the desired compound was obtained as a yellow oil.

Subsequently, this pyrimidinedione derivative was treated with a methanolic solution of hydrochloric acid according

to conventional procedure to obtain the hydrochloride (m.p. 152-156°C) of the desired compound.

Example 60 (Illustrative Compound No. 1203)

Synthesis of 6-[3-[N-[2-(2-acetyl-4-fluorophenoxy)ethyl]-N-methylamino]propyl]-1,3-dimethyl-2,4(1H,3H)pyrimidinedione.

The procedure of Example 29 was repeated, except that 5-fluoro-2-hydroxyacetophenone was used in place of 4-nitrophenol. Thus, the desired compound was obtained as a yellow oil.

Subsequently, this pyrimidinedione derivative was treated with a methanolic solution of hydrochloric acid according to conventional procedure to obtain the hydrochloride (m.p. 164-166°C) of the desired compound.

Example 61 (Illustrative Compound No. 1134)

Synthesis of 6-[2-[N-[2-(2-acetyl-4-fluorophenoxy)ethyl]-N-ethylamino]ethyl]-1,3-dimethyl-2,4(1H,3H)pyrimidinedione.

The procedure of Example 32 was repeated, except that 5-fluoro-2-hydroxyacetophenone was used in place of 4-nitrophenol. Thus, the desired compound was obtained as a colorless oil.

Subsequently, this pyrimidinedione derivative was treated with a methanolic solution of hydrochloric acid according to conventional procedure to obtain the hydrochloride (m.p. 167-174°C) of the desired compound.

Example 62 (Illustrative Compound No. 1409)

Synthesis of 6-[3-[N-[3-(2-acetyl-4-chlorophenoxy)-propyl]-N-ethylamino]propyl]-1,3-dimethyl-2,4(1H,3H)-pyrimidinedione.

The procedure of Example 31 was repeated, except that 5-chloro-2-hydroxyacetophenone was used in place of 4-nitrophenol. Thus, the desired compound was obtained as a yellow oil.

Subsequently, this pyrimidinedione derivative was treated with a methanolic solution of fumaric acid according to conventional procedure to obtain the fumarate (m.p. 155-158°C) of the desired compound.

Example 63 (Illustrative Compound No. 1568)

Synthesis of 6-[3-[N-[2-(4-fluorophenoxy)ethyl]amino]propyl]-1,3-dimethyl-2,4(1H,3H)pyrimidinedione.

2-(4-Fluorophenoxy)ethyl p-toluenesulfonate (2.79 g), the 6-(3-aminopropyl)-1,3-dimethyl-2,4(1H,3H)pyrimidinedione (1.97 g) synthesized in Example 25 and triethylamine (2.27 g) were dissolved in dioxane (15 ml), and the resulting solution was refluxed with stirring for 8 hours. After the solvent was removed by vacuum distillation, the residue was dissolved in chloroform, and 1N hydrochloric acid was added to the resulting solution, so that crystals separated out. The aqueous phase was separated, adjusted to pH 10-11 with sodium hydroxide, and then extracted with chloroform. On the other hand, 1N sodium hydroxide was added to the crystals that separated out, and the resulting mixture was extracted with chloroform. The organic phases obtained from the respective extraction treatments were combined, dried over anhydrous sodium sulfate, and then concentrated. The residue was purified by silica gel column chromatography (chloroform/methanol = 10/1) to obtain the desired compound (1.8 g) as a yellow oil.

Subsequently, this pyrimidinedione derivative was treated with a methanolic solution of hydrochloric acid according to conventional procedure to obtain the hydrochloride (m.p. 216-218°C) of the desired compound.

Example 64 (Illustrative Compound No. 1264)

Synthesis of 6-[3-[N-isopropyl-N-[2-(2-methoxyphenoxy)ethyl]amino]propyl]-1,3-dimethyl-2,4(1H,3H)pyrimidinedione.

The procedure of Example 27 was repeated, except that 2-methoxyphenol was used in place of 4-nitrophenol. Thus, the desired compound was obtained as a yellow oil.

Example 65 (Illustrative Compound No. 1546)

Synthesis of 6-[3-[N-[2-(4-fluorophenoxy)ethyl]-N-(dimethylamino)ethylamino]propyl]-1,3-dimethyl-2,4(1H,3H)-pyrimidinedione.

N-dimethylaminoethyl-[2-(4-fluorophenoxy)ethyl]amine (1.18 g) synthesized according to conventional procedure, the 6-(3-chloropropyl)-1,3-dimethyl-2,4(1H,3H)pyrimidinedione (1.34 g) synthesized in Example 3, sodium iodide (0.93 g) and triethylamine (1.4 ml) were added to dioxane (30 ml), and the resulting suspension was heated under reflux for 8 hours.

After being allowed to cool, the yellow reaction mixture was concentrated, and the residue was diluted with chloroform and washed with a 5% aqueous solution of sodium hydroxide. The wash liquid (aqueous phase) was extracted again with chloroform. The respective chloroform phases were combined, dried over anhydrous sodium sulfate, and then concentrated. The residue was purified by silica gel column chromatography (methanol/chloroform = 1/10) to obtain the desired compound (0.29 g) as a brown oil.

Example 66 (Illustrative Compound No. 709)

Synthesis of 6-[3-[N-[2-(4-aminophenoxy)ethyl]-N-isopropylamino]propyl]-1,3-dimethyl-2,4(1H,3H)-pyrimidinedione.

The 1,3-dimethyl-6-[3-[N-[2-(4-nitrophenoxy)ethyl]-N-isopropylamino]propyl]-2,4(1H,3H)pyrimidinedione (0.55 g) synthesized in Example 27 was dissolved in methanol (30 ml), and Pd/C (0.1 g) was added to the resulting solution under an atmosphere of nitrogen. Using a hydrogenation apparatus, this mixture was hydrogenated at room temperature. After completion of the hydrogen absorption, the Pd/C was separated by filtration. The filtrate was concentrated *in vacuo* to obtain the desired compound (0.58 g) as a white solid.

Example 67 (Illustrative Compound No. 707)

Synthesis of 6-[3-[N-[2-(4-aminophenoxy)ethyl]-N-ethylamino]propyl]-1,3-dimethyl-1H,3H) pyrimidinedione.

The 6-[3-[N-ethyl-N-[2-(4-nitrophenoxy)ethyl]amino]propyl]-1,3-dimethyl-2,4(1H,3H)pyrimidinedione synthesized in Example 28 was hydrogenated in the same manner as in Example 66 to obtain the desired compound.

Example 68 (Illustrative Compound No. 706)

Synthesis of 6-[3-[N-[2-(4-aminophenoxy)ethyl]-N-methylamino]propyl]-1,3-dimethyl-2,4(1H,3H)pyrimidinedione.

The 6-[3-[N-methyl-N-[2-(4-nitrophenoxy)ethyl]amino]propyl]-1,3-dimethyl-2,4(1H,3H)pyrimidinedione synthesized in Example 29 was hydrogenated in the same manner as in Example 66 to obtain the desired compound.

Example 69 (Illustrative Compound No. 708)

Synthesis of 6-[3-[N-[2-(4-aminophenoxy)ethyl]-N-propylamino]propyl]-1,3-dimethyl-2,4(1H,3H)pyrimidinedione.

The 1,3-dimethyl-6-[3-[N-[2-(4-nitrophenoxy)ethyl]-N-propylamino]propyl]-2,4(1H,3H)pyrimidinedione synthesized in Example 30 was hydrogenated in the same manner as in Example 66 to obtain the desired compound.

Example 70 (Illustrative Compound No. 719)

Synthesis of 6-[3-[N-[3-(4-aminophenoxy)propyl]-N-ethylamino]propyl]-1,3-dimethyl-2,4(1H,3H)pyrimidinedione.

The 6-[3-[N-ethyl-N-[3-(4-nitrophenoxy)propyl]amino]propyl]-1,3-dimethyl-2,4(1H,3H)pyrimidinedione synthesized in Example 31 was hydrogenated in the same manner as in Example 66 to obtain the desired compound.

Example 71 (Illustrative Compound No. 701)

Synthesis of 6-[2-[N-[2-(4-aminophenoxy)ethyl]-N-ethylamino]ethyl]-1,3-dimethyl-2,4(1H,3H)pyrimidinedione.

The 6-[2-[N-ethyl-N-[2-(4-nitrophenoxy)ethyl]amino]-ethyl]-1,3-dimethyl-2,4(1H,3H)pyrimidinedione synthesized in Example 32 was hydrogenated in the same manner as in Example 66 to obtain the desired compound.

Example 72 (Illustrative Compound No. 700)

Synthesis of 6-[2-[N-[2-(4-aminophenoxy)ethyl]-N-methylamino]ethyl]-1,3-dimethyl-2,4(1H,3H)pyrimidinedione.

The 6-[2-[N-methyl-N-[2-(4-nitrophenoxy)ethyl]amino]ethyl]-1,3-dimethyl-2,4(1H,3H)pyrimidinedione synthesized in Example 33 was hydrogenated in the same manner as in Example 66 to obtain the desired compound.

Example 73 (Illustrative Compound No. 703)

Synthesis of 6-[2-[N-[2-(4-aminophenoxy)ethyl]-N-isopropylamino]ethyl]-1,3-dimethyl-2,4(1H,3H)pyrimidinedione.

The 6-[2-[N-isopropyl-N-[2-(4-nitrophenoxy)ethyl]amino]ethyl]-1,3-dimethyl-2,4(1H,3H)pyrimidinedione synthesized in Example 33 was hydrogenated in the same manner as in Example 66 to obtain the desired compound.

Example 74 (Illustrative Compound No. 702)

Synthesis of 6-[2-[N-[2-(4-aminophenoxy)ethyl]-N-propylamino]ethyl]-1,3-dimethyl-2,4(1H,3H)pyrimidinedione.

The 1,3-dimethyl-6-[2-[N-[2-(4-nitrophenoxy)ethyl]-N-propylamino]ethyl]-2,4(1H,3H)pyrimidinedione synthesized in Example 35 was hydrogenated in the same manner as in Example 66 to obtain the desired compound.

Example 75 (Illustrative Compound No. 705)

Synthesis of 6-[3-[N-[2-(4-aminophenoxy)ethyl]amino]propyl]-1,3-dimethyl-2,4 (1H,3H)pyrimidinedione.

The 1,3-dimethyl-6-[3-[N-[2-(4-nitrophenoxy)ethyl]amino]propyl]-2,4(1H,3H)pyrimidinedione synthesized in Example 36 was hydrogenated in the same manner as in Example 66 to obtain the desired compound.

Example 76 (Illustrative Compound No. 587)

Synthesis of 1,3-dimethyl-6-[3-[N-[2-(4-nitrophenyl)ethyl]-N-propylamino]propyl]-2,4(1H,3H)pyrimidinedione.

The 6-(3-chloropropyl)-1,3-dimethyl-2,4(1H,3H)pyrimidinedione (8.1 g) synthesized in Example 3, N-propyl-[2-(4-nitrophenyl)ethyl]amine (7 g) and triethylamine (4.1 g) were dissolved in dioxane (100 ml), and sodium iodide (5.5 g) was added to the resulting solution. This solution was heated under reflux for 15 hours.

The resulting reaction solution was concentrated in vacuo, and the residue was extracted with chloroform/2N hydrochloric acid. The aqueous phases obtained from the respective extraction treatments were combined and extracted three times with chloroform/2N aqueous sodium hydroxide. The organic phases obtained from the respective extraction treatments were combined, dried over anhydrous sodium sulfate, and then concentrated. The residue was purified by silica gel column chromatography (chloroform/methanol = 200/3) to obtain the desired compound (10.1 g) as a yellow oil.

Example 77 (Illustrative Compound No. 589)

Synthesis of 6-[3-[N-butyl-N-[2-(4-nitrophenyl)ethyl]amino]propyl]-1,3-dimethyl-2,4(1H,3H)pyrimidinedione.

The procedure of Example 76 was repeated, except that N-butyl-[2-(4-nitrophenyl)ethyl]amine was used in place of N-propyl-[2-(4-nitrophenyl)ethyl]amine. Thus, the desired compound was obtained as a yellow oil.

Example 78 (Illustrative Compound No. 588)

Synthesis of 6-[3-[N-isopropyl-N-[2-(4-nitrophenyl)ethyl]amino]propyl]-1,3-dimethyl-2,4(1H,3H)pyrimidinedione.

The procedure of Example 76 was repeated, except that N-isopropyl-[2-(4-nitrophenyl)ethyl]amine was used in place of N-propyl-[2-(4-nitrophenyl)ethyl]amine. Thus, the desired compound was obtained as a yellow oil.

Example 79 (Illustrative Compound No. 585)

Synthesis of 1,3-dimethyl-6-[3-[N-methyl-N-[2-(4-nitrophenyl)ethyl]amino]propyl]-2,4(1H,3H)pyrimidinedione.

The procedure of Example 76 was repeated, except that N-methyl-[2-(4-nitrophenyl)ethyl]amine was used in place of N-propyl-[2-(4-nitrophenyl)ethyl]amine. Thus, the desired compound was obtained as yellow crystals.

Example 80 (Illustrative Compound No. 539)

Synthesis of 1,3-dimethyl-6-[3-[N-[2-(3-nitrophenyl)ethyl]-N-propylamino]propyl]-2,4(1H,3H)pyrimidinedione.

The procedure of Example 76 was repeated, except that N-propyl-[2-(3-nitrophenyl)ethyl]amine was used in place of N-propyl-[2-(4-nitrophenyl)ethyl]amine. Thus, the desired compound was obtained as a yellow oil.

Example 81 (Illustrative Compound No. 538)

Synthesis of 1,3-dimethyl-6-[3-[N-[2-(2-nitrophenyl)ethyl]-N-propylamino]propyl]-2,4(1H,3H)pyrimidinedione.

The procedure of Example 76 was repeated, except that N-propyl-[2-(2-nitrophenyl)ethyl]amine was used in place of N-propyl-[2-(4-nitrophenyl)ethyl]amine. Thus, the desired compound was obtained as a yellow oil.

Example 82 (Illustrative Compound No. 580)

Synthesis of 6-[2-[N-ethyl-N-[2-(4-nitrophenyl)ethyl]amino]ethyl]-1,3-dimethyl-2,4(1H,3H)pyrimidinedione.

The 6-(2-hydroxyethyl)-1,3-dimethyl-2,4(1H,3H)pyrimidinedione (1.5 g) synthesized in Example 11, p-toluenesulfonyl chloride (1.6 g) and triethylamine (1.23 g) were dissolved in dichloromethane, and the resulting solution was stirred at room temperature for 8 hours. After the solvent was removed by vacuum distillation, the residue was dissolved in dioxane, and N-ethyl-[2-(4-nitrophenyl)ethyl]amine (2.37 g) and triethylamine (4 ml) were further added to the resulting solution. This solution was heated under reflux for 6 hours. After the solvent was removed by vacuum distillation, the residue was dissolved in chloroform, and 1N hydrochloric acid was added to the resulting solution. After extraction, the aqueous phase was separated, adjusted to pH 10-11 with an aqueous solution of sodium hydroxide, and then extracted with chloroform. The organic phases obtained from the respective extraction treatments were combined, dried over anhydrous sodium sulfate, and then concentrated in vacuo. The residue was purified by silica gel column chromatography (chloroform/methanol = 15/1) to obtain the desired compound (1.82 g) as a brown oil.

Example 83 (Illustrative Compound No. 1556)

Synthesis of 1,3-dimethyl-6-[3-[N-(2-phenylethyl)amino]propyl]-2,4(1H,3H)pyrimidinedione.

Phenetylamine hydrochloride (0.95 g) and the 6-(3-chloropropyl)-1,3-dimethyl-2,4(1H,3H)pyrimidinedione (1.08 g) synthesized in Example 3 were dissolved in ethanol (10 ml), and triethylamine (6 ml) and sodium iodide (0.9 g) were added to the resulting solution. This solution was heated under reflux for 14 hours. After the solvent was removed by vacuum distillation, the residue was dissolved in chloroform and the resulting solution was extracted by the addition of 1N hydrochloric acid. The aqueous phase was separated, adjusted to pH 11-12 with an aqueous solution of sodium hydroxide, and then extracted with chloroform. The organic phases obtained from the respective extraction treatments were combined, dried over anhydrous sodium sulfate, and then concentrated. The residue was purified by silica gel column chromatography (chloroform/methanol = 10/1) to obtain the desired compound (1.35 g) as yellow crystals.

Subsequently, this pyrimidinedione derivative was treated with hydrochloric acid/methanol according to conventional procedure to obtain the hydrochloride (m.p. 77.2-78.2°C) of the desired compound.

Example 84 (Illustrative Compound No. 586)

Synthesis of 6-[3-[N-ethyl-N-[2-(4-nitrophenyl)ethyl]amino]propyl]-1,3-dimethyl-2,4(1H,3H)pyrimidinedione.

4-Nitrophenetyl bromide (1.54 g) and the 6-(3-ethylaminopropyl)-1,3-dimethyl-2,4(1H,3H)pyrimidinedione (2.22 g) synthesized in Example 20 were dissolved in ethanol (15 ml), and triethylamine (2.8 ml) was added to the resulting solution. This solution was refluxed for 7 hours. After the solvent was removed by vacuum distillation, the residue was dissolved in chloroform (100 ml) and the resulting solution was washed with a 1N aqueous solution of sodium hydroxide and then with a saturated aqueous solution of sodium chloride. The washed organic phase was dried over anhydrous sodium sulfate and then concentrated in vacuo. The residue was purified by silica gel column chromatography (chloroform/methanol = 10/1) to obtain the desired compound (0.52 g) as yellow crystals.

Subsequently, this pyrimidinedione derivative was treated with hydrochloric acid/methanol according to conventional procedure to obtain the hydrochloride (m.p. 177.6-179°C) of the desired compound.

Example 85 (Illustrative Compound No. 584)

Synthesis of 1,3-dimethyl-6-[3-[N-[2-(4-nitrophenyl)ethyl]amino]propyl]-2,4(1H,3H)pyrimidinedione.

2-(4-Nitrophenyl)ethyl p-toluenesulfonate (2.2 g) and the 6-(3-aminopropyl)-1,3-dimethyl-2,4(1H,3H)pyrimidinedione (1.5 g) synthesized in Example 25 were added to dioxane (15 ml), and the resulting solution was refluxed with stirring for 20 hours. After the reaction solution was allowed to cool, the solvent was removed by vacuum distillation, the residue was dissolved in chloroform and the resulting solution was extracted by the addition of 1N hydrochloric acid. The aqueous phase was separated, adjusted to pH 10-11 with an aqueous solution of sodium hydroxide, and then extracted with chloroform. The organic phases obtained from the respective extraction treatments were combined, dried over anhydrous sodium sulfate, and then concentrated in vacuo. The residue was purified by silica gel column chromatography (chloroform/methanol = 10/1) to obtain the desired compound (1.01 g) as yellow crystals.

Example 86 (Illustrative Compound No. 247)

Synthesis of 1,3-diethyl-6-[3-[N-[2-(4-nitrophenyl)ethyl]-N-propylamino]propyl]-2,4(1H,3H)pyrimidinedione.

The 6-(3-chloropropyl)-1,3-diethyl-2,4(1H,3H)pyrimidinedione (2.45 g) synthesized in Example 5, N-propyl-[2-(4-nitrophenyl)ethyl]amine (2.29 g) and triethylamine (1.52 g) were dissolved in dioxane (15 ml), and sodium iodide (1.65 g) was added to the resulting solution. The resulting solution was heated under reflux for 3 hours. After the reaction solution was concentrated in vacuo, the residue was extracted by the addition of chloroform and a 1N aqueous solution of sodium hydroxide. The organic phase was separated, dried over anhydrous sodium sulfate, and then concentrated. The residue was purified by silica gel column chromatography (chloroform/methanol = 20/1) to obtain the desired compound (2.28 g) as a yellow oil.

Example 87 (Illustrative Compound No. 279)

Synthesis of 1,3-diisopropyl-6-[3-[N-propyl-N-[2-(4-nitrophenyl)ethyl]amino]propyl]-2,4(1H,3H)pyrimidinedione.

The procedure of Example 86 was repeated, except that the 6-(3-chloropropyl)-1,3-diisopropyl-2,4(1H,3H)pyrimidinedione synthesized in Example 6 was used in place of 6-(3-chloropropyl)-1,3-diethyl-2,4(1H,3H)pyrimidinedione. Thus, the desired compound was obtained as a yellow oil.

Example 88 (Illustrative Compound No. 1495)

Synthesis of 6-[3-[N-methoxyethyl-N-[2-(4-nitrophenyl)ethyl]amino]propyl]-1,3-dimethyl-2,4(1H,3H)pyrimidinedione.

2-(4-Nitrophenyl)ethyl p-toluenesulfonate (2.5 g), the 6-[3-(N-methoxyethylamino)propyl]-1,3-dimethyl-2,4(1H,3H)pyrimidinedione (1.65 g) synthesized in Example 22, and triethylamine (1.4 ml) were added to dioxane (50 ml), and the resulting solution was heated under reflux for 18 hours. After the solvent was removed by vacuum distillation, the residue was dissolved in chloroform and the resulting solution was extracted by the addition of 1N hydrochloric acid. The aqueous phase was separated, adjusted to pH 10-11 with an aqueous solution of sodium hydroxide, and then extracted with chloroform. The organic phases obtained from the respective extraction treatments were combined, dried over anhydrous sodium sulfate, and then concentrated in vacuo. The residue was purified by silica gel column chromatography (chloroform/methanol = 30/1) to obtain the desired compound (0.68 g) as a brown oil.

Example 89 (Illustrative Compound No. 1527)

Synthesis of 1,3-dimethyl-6-[3-[N-dimethylaminoethyl-N-[2-(4-nitrophenyl)ethyl]amino]propyl]-2,4(1H,3H)-pyrimidinedione.

The procedure of Example 76 was repeated, except that N-dimethylaminoethyl-[2-(4-nitrophenyl)ethyl]amine synthesized according to conventional procedure was used in place of N-propyl-[2-(4-nitrophenyl)ethyl]amine. Thus, the desired compound was obtained as a red oil.

Example 90 (Illustrative Compound No. 592)

Synthesis of 6-[2-[N-ethyl-N-[3-(4-nitrophenyl)propyl]amino]ethyl]-1,3-dimethyl-2,4(1H,3H)pyrimidinedione.

The procedure of Example 82 was repeated, except that N-ethyl-[3-(4-nitrophenyl)propyl]amine was used in place of N-ethyl-[2-(4-nitrophenyl)ethyl]amine. Thus, the desired compound was obtained as a pale-yellow oil.

Subsequently, this pyrimidinedione derivative was treated with hydrochloric acid/methanol according to conventional procedure to obtain the hydrochloride (m.p. 187.5-189.5°C) of the desired compound.

Example 91 (Illustrative Compound No. 598)

Synthesis of 6-[3-[N-ethyl-N-[3-(4-nitrophenyl)propyl]amino]propyl]-1,3-dimethyl-2,4(1H,3H)pyrimidinedione.

The 1,3-dimethyl-6-[3-(p-toluenesulfonyloxy)propyl]-2,4(1H,3H)pyrimidinedione (0.46 g) synthesized in Example 26 and N-ethyl-[3-(4-nitrophenyl)propyl]amine (0.41 g) were suspended in triethylamine (5 ml), and the resulting suspension was stirred at 60°C for 4.5 hours.

Then, the reaction mixture was concentrated in vacuo. The residue was purified by silica gel column chromatography (chloroform/methanol = 25/2) to obtain the desired compound (0.1 g).

Example 92 (Illustrative Compound No. 599)

Synthesis of 1,3-dimethyl-6-[3-[N-[3-(4-nitrophenyl)propyl]-N-propylamino]propyl]-2,4(1H,3H)pyrimidinedione.

The procedure of Example 76 was repeated, except that N-propyl-[3-(4-nitrophenyl)propyl]amine was used in place of N-propyl-[2-(4-nitrophenyl)ethyl]amine. Thus, the desired compound was obtained as a yellow oil.

Example 93 (Illustrative Compound No. 1028)

Synthesis of 6-[3-[N-[3-(4-chlorophenyl)propyl]-N-ethylamino]propyl]-1,3-dimethyl-2,4(1H,3H)pyrimidinedione.

3-(4-chlorophenyl)propyl p-toluenesulfonate (1.31 g) and the 6-(3-ethylaminopropyl)-1,3-dimethyl-2,4(1H,3H)-pyrimidinedione (1.59 g) synthesized in Example 21 were dissolved in dioxane (20 ml), and triethylamine (1.8 ml) was added to the resulting solution. This solution was refluxed with stirring for 24 hours. After the solvent was removed by vacuum distillation, the residue was dissolved in chloroform (100 ml) and the resulting solution was washed with a 1N aqueous solution of sodium hydroxide and then with a saturated aqueous solution of sodium chloride. The washed

organic phase was dried over anhydrous sodium sulfate and then concentrated in vacuo. The residue was purified by silica gel column chromatography (chloroform/methanol = 10/1) to obtain the desired compound (0.79 g) as a yellow oil.

Subsequently, this pyrimidinedione derivative was treated with hydrochloric acid/methanol according to conventional procedure to obtain the hydrochloride (m.p. 120-121°C) of the desired compound.

Example 94 (Illustrative Compound No. 1024)

Synthesis of 6-[3-[N-ethyl-N-[3-(3-fluorophenyl)propyl]amino]propyl]-1,3-dimethyl-2,4(1H,3H)pyrimidinedione.

The procedure of Example 93 was repeated, except that 3-(3-fluorophenyl)propyl p-toluenesulfonate was used in place of 3-(4-chlorophenyl)propyl p-toluenesulfonate. Thus, the desired compound was obtained as a yellow oil.

Subsequently, this pyrimidinedione derivative was treated with hydrochloric acid/methanol according to conventional procedure to obtain the hydrochloride (m.p. 92-93°C) of the desired compound.

Example 95 (Illustrative Compound No. 1036)

Synthesis of 6-[3-[N-ethyl-N-[3-(4-methoxyphenyl)propyl]amino]propyl]-1,3-dimethyl-2,4(1H,3H)pyrimidinedione.

The procedure of Example 93 was repeated, except that 3-(4-methoxyphenyl)propyl p-toluenesulfonate was used in place of 3-(4-chlorophenyl)propyl p-toluenesulfonate. Thus, the desired compound was obtained as a yellow oil.

Subsequently, this pyrimidinedione derivative was treated with hydrochloric acid/methanol according to conventional procedure to obtain the hydrochloride (m.p. 54-56°C) of the desired compound.

Example 96 (Illustrative Compound No. 1029)

Synthesis of 6-[3-[N-[3-(4-cyanophenyl)propyl]-N-ethylamino]propyl]-1,3-dimethyl-2,4(1H,3H)pyrimidinedione.

The procedure of Example 93 was repeated, except that 3-(4-cyanophenyl)propyl p-toluenesulfonate was used in place of 3-(4-chlorophenyl)propyl p-toluenesulfonate. Thus, the desired compound was obtained as a yellow oil.

Subsequently, this pyrimidinedione derivative was treated with hydrochloric acid/methanol according to conventional procedure to obtain the hydrochloride (m.p. 213-215°C) of the desired compound.

Example 97 (Illustrative Compound No. 1022)

Synthesis of 6-[3-[N-ethyl-N-(3-phenylpropyl)amino]propyl]-1,3-dimethyl-2,4(1H,3H)pyrimidinedione.

The procedure of Example 84 was repeated, except that 3-phenylpropyl bromide was used in place of 4-nitorphenetyl bromide. Thus, the desired compound was obtained as a yellow oil.

Subsequently, this pyrimidinedione derivative was treated with hydrochloric acid/methanol according to conventional procedure to obtain the hydrochloride (m.p. 66-67°C) of the desired compound.

Example 98 (Illustrative Compound No. 936)

Synthesis of 6-[2-[N-[3-(4-chlorophenyl)propyl]-N-ethylamino]ethyl]-1,3-dimethyl-2,4(1H,3H)pyrimidinedione.

The 6-(2-ethylaminoethyl)-1,3-dimethyl-2,4(1H,3H)-pyrimidinedione (0.38 g) synthesized in Example 23, 3-(4-chlorophenyl)propyl p-toluenesulfonate (0.59 g) and triethylamine (3 ml) were dissolved in dioxane (2 ml). The resulting solution was heated under reflux for 8 hours.

After being allowed to cooled, the reaction mixture was concentrated in vacuo. The residue was purified by silica gel column chromatography (chloroform/methanol = 100/1) to obtain the desired compound (0.11 g) as a colorless oil.

Example 99 (Illustrative Compound No. 932)

Synthesis of 6-[2-[N-ethyl-N-[3-(3-fluorophenyl)propyl]amino]ethyl]-1,3-dimethyl-2,4(1H,3H)pyrimidinedione.

The procedure of Example 98 was repeated, except that 3-(3-fluorophenyl)propyl p-toluenesulfonate was used in place of 3-(4-chlorophenyl)propyl p-toluenesulfonate. Thus, the desired compound was obtained as a colorless oil.

Example 100 (Illustrative Compound No. 684)

Synthesis of 6-[3-[N-[2-(4-aminophenyl)ethyl]-N-propylamino]propyl]-1,3-dimethyl-2,4(1H,3H)pyrimidinedione

The 1,3-dimethyl-6-[3-[N-[2-(4-nitrophenyl)ethyl]-N-propylamino]propyl]-2,4(1H,3H)pyrimidinedione (1.65 g) synthesized in Example 76 was hydrogenated at room temperature in methanol (80 ml) in the presence of Pd/C (purity 10%; 0.3 g). After completion of the reaction, the Pd/C was separated by filtration. The filtrate was concentrated in vacuo to obtain the desired compound (1.39 g) as a colorless amorphous solid.

Example 101 (Illustrative Compound No. 686)

Synthesis of 6-[3-[N-butyl-N-[2-(4-aminophenyl)ethyl]amino]propyl]-1,3-dimethyl-2,4(1H,3H)pyrimidinedione.

The 6-[3-[N-butyl-N-[2-(4-nitrophenyl)ethyl]amino]propyl]-1,3-dimethyl-2,4(1H,3H)pyrimidinedione synthesized in Example 77 was hydrogenated in the same manner as in Example 100 to obtain the desired compound.

Example 102 (Illustrative Compound No. 685)

Synthesis of 6-[3-[N-[2-(4-aminophenyl)ethyl]-N-isopropylamino]propyl]-1,3-dimethyl-2,4(1H,3H)pyrimidinedione.

The 6-[3-[N-isopropyl-N-[2-(4-nitrophenyl)ethyl]amino]propyl]-1,3-dimethyl-2,4(1H,3H)pyrimidinedione synthesized in Example 78 was hydrogenated in the same manner as in Example 100 to obtain the desired compound.

Example 103 (Illustrative Compound No. 682)

Synthesis of 1,3-dimethyl-6-[3-[N-[2-(4-aminophenyl)ethyl]-N-methylamino]propyl]-2,4(1H,3H)pyrimidinedione.

The 1,3-dimethyl-6-[3-[N-methyl-N-[2-(4-nitrophenyl)ethyl]amino]propyl]-2,4(1H,3H)pyrimidinedione synthesized in Example 79 was hydrogenated in the same manner as in Example 100 to obtain the desired compound.

Example 104 (Illustrative Compound No. 636)

Synthesis of 6-[3-[N-[2-(3-aminophenyl)ethyl]-N-propylamino]propyl]-1,3-dimethyl-2,4(1H,3H)pyrimidinedione.

The 1,3-dimethyl-6-[3-[N-[2-(3-nitrophenyl)ethyl]-N-propylamino]propyl]-2,4(1H,3H)pyrimidinedione synthesized in Example 80 was hydrogenated in the same manner as in Example 100 to obtain the desired compound.

Example 105 (Illustrative Compound No. 635)

Synthesis of 6-[3-[N-[2-(2-aminophenyl)ethyl]-N-propylamino]propyl]-1,3-dimethyl-2,4(1H,3H)pyrimidinedione.

The 1,3-dimethyl-6-[3-[N-[2-(2-nitrophenyl)ethyl]-N-propylamino]propyl]-2,4(1H,3H)pyrimidinedione synthesized in Example 81 was hydrogenated in the same manner as in Example 100 to obtain the desired compound.

Example 106 (Illustrative Compound No. 677)

Synthesis of 6-[2-[N-[2-(4-aminophenyl)ethyl]-N-ethylamino]ethyl]-1,3-dimethyl-2,4(1H,3H)pyrimidinedione.

The 6-[2-[N-ethyl-N-[2-(4-nitrophenyl)ethyl]amino]ethyl]-1,3-dimethyl-2,4(1H,3H)pyrimidinedione synthesized in Example 82 was hydrogenated in the same manner as in Example 100 to obtain the desired compound.

Example 107 (Illustrative Compound No. 683)

Synthesis of 6-[3-[N-[2-(4-aminophenyl)ethyl]-N-ethylamino]propyl]-1,3-dimethyl-2,4(1H,3H)pyrimidinedione.

The 6-[3-[N-ethyl-N-[2-(4-nitrophenyl)ethyl]amino]propyl]-1,3-dimethyl-2,4(1H,3H)pyrimidinedione synthesized in Example 84 was hydrogenated in the same manner as in Example 100 to obtain the desired compound.

Example 108 (Illustrative Compound No. 681)

Synthesis of 6-[3-[N-[2-(4-aminophenyl)ethyl]amino]propyl]-1,3-dimethyl-2,4(1H,3H)pyrimidinedione.

The 1,3-dimethyl-6-[3-[N-[2-(4-nitrophenyl)ethyl]amino]propyl]-2,4(1H,3H)pyrimidinedione synthesized in Example 85 was hydrogenated in the same manner as in Example 100 to obtain the desired compound.

Example 109 (Illustrative Compound No. 343)

Synthesis of 6-[3-[N-[2-(4-aminophenyl)ethyl]-N-propylamino]propyl]-1,3-dimethyl-2,4(1H,3H)pyrimidinedione.

The 1,3-diethyl-6-[3-[N-[2-(4-nitrophenyl)ethyl]-N-propylamino]propyl]-2,4(1H,3H)pyrimidinedione synthesized in Example 86 was hydrogenated in the same manner as in Example 100 to obtain the desired compound.

Example 110 (Illustrative Compound No. 375)

Synthesis of 6-[3-[N-[2-(4-aminophenyl)ethyl]-N-propylamino]propyl]-1,3-diisopropyl-2,4(1H,3H)pyrimidinedione.

The 1,3-diisopropyl-6-[3-[N-propyl-N-[2-(4-nitrophenyl)ethyl]amino]propyl]-2,4(1H,3H)pyrimidinedione synthesized in Example 87 was hydrogenated in the same manner as in Example 100 to obtain the desired compound.

Example 111 (Illustrative Compound No. 1496)

Synthesis of 6-[3-[N-[2-(4-aminophenyl)ethyl]-N-methoxyethylamino]propyl]-1,3-dimethyl-2,4(1H,3H)pyrimidinedione.

The 6-[3-[N-methoxyethyl-N-[2-(4-nitrophenyl)ethyl]amino]propyl]-1,3-dimethyl-2,4(1H,3H)pyrimidinedione synthesized in Example 88 was hydrogenated in the same manner as in Example 100 to obtain the desired compound.

Example 112 (Illustrative Compound No. 1528)

Synthesis of 1,3-dimethyl-6-[3-[N-[2-(4-aminophenyl)ethyl]-N-dimethylaminoethylamino]propyl]-2,4(1H,3H)pyrimidinedione.

The 1,3-dimethyl-6-[3-[N-dimethylaminoethyl-N-[2-(4-nitrophenyl)ethyl]amino]propyl]-2,4(1H,3H)pyrimidinedione synthesized in Example 89 was hydrogenated in the same manner as in Example 100 to obtain the desired compound.

Example 113 (Illustrative Compound No. 689)

Synthesis of 6-[2-[N-[3-(4-aminophenyl)propyl]-N-ethylamino]ethyl]-1,3-dimethyl-2,4(1H,3H)pyrimidinedione.

The 6-[2-[N-ethyl-N-[3-(4-nitrophenyl)propyl]amino]ethyl]-1,3-dimethyl-2,4(1H,3H)pyrimidinedione synthesized in Example 90 was hydrogenated in the same manner as in Example 100 to obtain the desired compound.

Example 114 (Illustrative Compound No. 695)

Synthesis of 6-[3-[N-[3-(4-aminophenyl)propyl]-N-ethylamino]propyl]-1,3-dimethyl-2,4(1H,3H)pyrimidinedione.

The 6-[3-[N-ethyl-N-[3-(4-nitrophenyl)propyl]amino]propyl]-1,3-dimethyl-2,4(1H,3H)pyrimidinedione synthesized in Example 91 was hydrogenated in the same manner as in Example 100 to obtain the desired compound.

Example 115 (Illustrative Compound No. 696)

Synthesis of 6-[3-[N-[3-(4-aminophenyl)propyl]-N-propylamino]propyl]-1,3-dimethyl-2,4(1H,3H)-pyrimidinedione.

The 1,3-dimethyl-6-[3-[N-[3-(4-nitrophenyl)propyl]-N-propylamino]propyl]-2,4(1H,3H)pyrimidinedione synthesized in Example 92 was hydrogenated in the same manner as in Example 100 to obtain the desired compound.

Example 116 (Illustrative Compound No. 35)

Synthesis of 6-[3-[N-[2-(4-methanesulfonylaminophenoxy)ethyl]-N-isopropylamino]propyl]-1,3-dimethyl-2,4(1H,3H)pyrimidinedione.

The 6-[3-[N-[2-(4-aminophenoxy)ethyl]-N-isopropylamino]propyl]-1,3-dimethyl-2,4(1H,3H)pyrimidinedione (0.58 g) synthesized in Example 66 was dissolved in pyridine (10 ml), and methanesulfonyl chloride (0.21 g) was added to the resulting solution under an atmosphere of nitrogen. This mixture was stirred at room temperature for 8 hours. After completion of the reaction, the reaction mixture was concentrated in vacuo and the residue was dissolved in chloroform. After the addition of a saturated aqueous solution of sodium bicarbonate, the resulting mixture was extracted four times with chloroform. The organic phases obtained from the respective extraction treatments were combined, dried over anhydrous sodium sulfate, and then concentrated. The residue was purified by silica gel column chromatography (chloroform/methanol = 50/1) to obtain the desired compound (0.46 g) as a colorless oil.

Subsequently, this pyrimidinedione derivative was treated with fumaric acid/methanol according to conventional procedure to obtain the fumarate (m.p. 62-67°C) of the desired compound.

Example 117 (Illustrative Compound No. 33)

Synthesis of 6-[3-[N-ethyl-N-[2-(4-methanesulfonylaminophenoxy)ethyl]amino]propyl]-1,3-dimethyl-2,4(1H,3H)pyrimidinedione.

The 6-[3-[N-[2-(4-aminophenoxy)ethyl]-N-ethylamino]propyl]-1,3-dimethyl-2,4(1H,3H)pyrimidinedione (0.4 g) synthesized in Example 67 was dissolved in dichloromethane (25 ml), and methanesulfonic acid anhydride (0.21 g) was added to the resulting solution at room temperature. This mixture was stirred at room temperature for an hour and allowed to stand overnight. After standing, the reaction mixture was washed with a saturated aqueous solution of sodium bicarbonate (20 ml) and the wash liquid (aqueous phase) was extracted three times with chloroform. The several organic phases were combined, dried over anhydrous sodium sulfate, and then concentrated. The residue was purified by silica gel column chromatography (methanol/chloroform = 1/10) to obtain the desired compound (0.27 g) as an oil.

Example 118 (Illustrative Compound No. 10)

Synthesis of 6-[3-[N-[2-(4-methanesulfonylaminophenyl)ethyl]-N-propylamino]propyl]-1,3-dimethyl-2,4(1H,3H)pyrimidinedione.

The 6-[3-[N-[2-(4-aminophenyl)ethyl]-N-propylamino]propyl]-1,3-dimethyl-2,4(1H,3H)pyrimidinedione (1.39 g) synthesized in Example 100 was dissolved in pyridine (20 ml), and methanesulfonyl chloride (0.53 g) was added to the resulting solution under an atmosphere of nitrogen. After this mixture was stirred at room temperature for 4 hours, the reaction mixture was concentrated in vacuo and the residue was dissolved in chloroform. After the addition of a saturated aqueous solution of sodium bicarbonate, the resulting mixture was extracted four times with chloroform. The organic phases obtained from the respective extraction treatments were combined, dried over anhydrous sodium sulfate, and then concentrated. The residue was purified by silica gel column chromatography (chloroform/methanol = 25/1) to obtain the desired compound (1.64 g) as a colorless oil.

Subsequently, this pyrimidinedione derivative was treated with fumaric acid/methanol according to conventional procedure to obtain the fumarate (m.p. 193.5-194°C) of the desired compound.

Example 119 (Illustrative Compound No. 7)

Synthesis of 6-[3-[N-[2-(4-methanesulfonylaminophenyl)ethyl]amino]propyl]-1,3-dimethyl-2,4(1H,3H)pyrimidinedione.

The 6-[3-[N-[2-(4-aminophenyl)ethyl]amino]propyl]-1,3-dimethyl-2,4(1H,3H)pyrimidinedione (0.84 g) synthesized in Example 108 and pyridine (1.5 ml) were added to dichloromethane (10 ml), and trimethylchlorosilane (0.25 ml) was added dropwise to the resulting solution under cooling with ice. After the resulting mixture was stirred for 2 hours, methanesulfonyl chloride (0.25 ml) was added dropwise thereto. The resulting mixture was stirred at room temperature for 3 hours and then allowed to stand overnight. After standing, the solvent was removed from the reaction mixture by vacuum distillation. The residue was dissolved in chloroform and the resulting solution was extracted by the addition of 1N hydrochloric acid. The aqueous phase was separated, adjusted to pH 7-8 by the addition of sodium hydroxide, and then extracted with chloroform. The organic phases obtained from the respective extraction treatments were combined, dried over anhydrous sodium sulfate, and then concentrated in vacuo. The residue was purified by silica gel column chromatography (chloroform/methanol/triethylamine = 50/5/1) to obtain the desired compound (0.52 g) as white powder.

Subsequently, this pyrimidinedione derivative was treated with hydrochloric acid/methanol according to conventional procedure to obtain the hydrochloride (m.p. 201-202°C) of the desired compound.

Example 120 (Illustrative Compound No. 32)

Synthesis of 6-[3-[N-methyl-N-[2-(4-methanesulfonylaminophenoxy)ethyl]amino]propyl]-1,3-dimethyl-2,4(1H,3H)-pyrimidinedione.

The 6-[3-[N-[2-(4-aminophenoxy)ethyl]-N-methylamino]propyl]-1,3-dimethyl-2,4(1H,3H)pyrimidinedione synthesized in Example 68 was methanesulfonated in the same manner as in Example 116 to obtain the desired compound.

Subsequently, this pyrimidinedione derivative was treated with hydrochloric acid/methanol according to conventional procedure to obtain the hydrochloride (m.p. 120-125°C) of the desired compound.

Example 121 (Illustrative Compound No. 34)

Synthesis of 6-[3-[N-n-propyl-N-[2-(4-methanesulfonylaminophenoxy)ethyl]amino]propyl]-1,3-dimethyl-2,4(1H,3H)pyrimidinedione.

The 6-[3-[N-[2-(4-aminophenoxy)ethyl]-N-propylamino]propyl]-1,3-dimethyl-2,4(1H,3H)pyrimidinedione synthesized in Example 69 was methanesulfonated in the same manner as in Example 116 to obtain the desired compound.

Example 122 (Illustrative Compound No. 45)

Synthesis of 6-[3-[N-ethyl-N-[3-(4-methanesulfonylaminophenoxy)propyl]amino]propyl]-1,3-dimethyl-2,4(1H,3H)-pyrimidinedione.

The 6-[3-[N-[3-(4-aminophenoxy)propyl]-N-ethylamino]propyl]-1,3-dimethyl-2,4(1H,3H)pyrimidinedione synthesized in Example 70 was methanesulfonated in the same manner as in Example 116 to obtain the desired compound.

Example 123 (Illustrative Compound No. 27)

Synthesis of 6-[2-[N-ethyl-N-[2-(4-methanesulfonylaminophenoxy)ethyl]amino]ethyl]-1,3-dimethyl-2,4(1H,3H)-pyrimidinedione.

The 6-[2-[N-[2-(4-aminophenoxy)ethyl]-N-ethylamino]ethyl]-1,3-dimethyl-2,4(1H,3H)pyrimidinedione synthesized in Example 71 was methanesulfonated in the same manner as in Example 116 to obtain the desired compound.

Subsequently, this pyrimidinedione derivative was treated with hydrochloric acid/methanol according to conventional procedure to obtain the hydrochloride (m.p. 195.5-197°C) of the desired compound.

Example 124 (Illustrative Compound No. 26)

Synthesis of 6-[2-[N-methyl-N-[2-(4-methanesulfonylaminophenoxy)ethyl]amino]ethyl]-1,3-dimethyl-2,4(1H,3H)-pyrimidinedione.

The 6-[2-[N-[2-(4-aminophenoxy)ethyl]-N-methylamino]ethyl]-1,3-dimethyl-2,4(1H,3H)pyrimidinedione synthesized in Example 72 was methanesulfonated in the same manner as in Example 116 to obtain the desired compound.

Subsequently, this pyrimidinedione derivative was treated with fumaric acid/methanol according to conventional procedure to obtain the fumarate (m.p. 162.5-163.5°C) of the desired compound.

Example 125 (Illustrative Compound No. 29)

Synthesis of 6-[2-[N-isopropyl-N-[2-(4-methanesulfonylaminophenoxy)ethyl]amino]ethyl]-1,3-dimethyl-2,4(1H, 3H)pyrimidinedione.

The 6-[2-[N-[2-(4-aminophenoxy)ethyl]-N-isopropylamino]ethyl]-1,3-dimethyl-2,4(1H,3H)pyrimidinedione synthesized in Example 73 was methanesulfonated in the same manner as in Example 116 to obtain the desired compound.

Example 126 (Illustrative Compound No. 28)

Synthesis of 6-[2-[N-n-propyl-N-[2-(4-methanesulfonylaminophenoxy)ethyl]amino]ethyl]-1,3-dimethyl-2,4(1H,3H)pyrimidinedione.

The 6-[2-[N-[2-(4-aminophenoxy)ethyl]-N-n-propylamino]ethyl]-1,3-dimethyl-2,4(1H,3H)pyrimidinedione synthesized in Example 74 was methanesulfonated in the same manner as in Example 116 to obtain the desired compound.

Example 127 (Illustrative Compound No. 31)

Synthesis of 6-[3-[N-2-(4-methanesulfonylaminophenoxy)propylamino]propyl]-1,3-dimethyl-2,4(1H,3H)pyrimidinedione.

The 6-[3-[N-[2-(4-aminophenoxy)ethyl]amino]propyl]-1,3-dimethyl-2,4(1H,3H)pyrimidinedione synthesized in Example 75 was methanesulfonated in the same manner as in Example 116 to obtain the desired compound.

Subsequently, this pyrimidinedione derivative was treated with fumaric acid/methanol according to conventional procedure to obtain the fumarate (m.p. 143-144°C) of 6-[3-[N-2-(4-methanesulfonylaminophenoxy)propylamino]propyl]-1,3-dimethyl-2,4(1H,3H)pyrimidinedione.

Example 128 (Illustrative Compound No. 12)

Synthesis of 6-[3-[N-n-butyl-N-[2-(4-methanesulfonylaminophenyl)ethyl]amino]propyl]-1,3-dimethyl-2,4(1H,3H)-pyrimidinedione.

The 6-[3-[N-n-butyl-N-[2-(4-aminophenyl)ethyl]amino]propyl]-1,3-dimethyl-2,4(1H,3H)pyrimidinedione synthesized in Example 101 was methanesulfonated in the same manner as in Example 116 to obtain the desired compound.

Example 129 (Illustrative Compound No. 11)

Synthesis of 6-[3-[N-isopropyl-N-[2-(4-methanesulfonylaminophenyl)ethyl]amino]propyl]-1,3-dimethyl-2,4(1H,3H)pyrimidinedione.

The 6-[3-[N-[2-(4-aminophenyl)ethyl]-N-isopropylamino]propyl]-1,3-dimethyl-2,4(1H,3H)pyrimidinedione synthesized in Example 102 was methanesulfonated in the same manner as in Example 116 to obtain the desired compound.

Subsequently, this pyrimidinedione derivative was treated with hydrochloric acid/methanol according to conventional procedure to obtain the hydrochloride (m.p. 153-155°C) of the desired compound.

Example 130 (Illustrative Compound No. 8)

Synthesis of 6-[3-[N-methyl-N-[2-(4-methanesulfonylaminophenyl)ethyl]amino]propyl]-1,3-dimethyl-2,4(1H,3H)pyrimidinedione.

The 1,3-dimethyl-6-[3-[N-[2-(4-aminophenyl)ethyl]-N-methylamino]propyl]-2,4(1H,3H)pyrimidinedione synthesized in Example 103 was methanesulfonated in the same manner as in Example 116 to obtain the desired compound.

Subsequently, this pyrimidinedione derivative was treated with fumaric acid/methanol according to conventional procedure to obtain the fumarate (m.p. 199-199.5°C) of the desired compound.

Example 131 (Illustrative Compound No. 442)

Synthesis of 6-[3-[N-n-propyl-N-[2-(3-methanesulfonylaminophenyl)ethyl]amino]propyl]-1,3-dimethyl-2,4(1H,3H)pyrimidinedione.

The 6-[3-[N-[2-(3-aminophenyl)ethyl]-N-propylamino]propyl]-1,3-dimethyl-2,4(1H,3H)pyrimidinedione synthesized in Example 104 was methanesulfonated in the same manner as in Example 116 to obtain the desired compound.

Example 132 (Illustrative Compound No. 3)

Synthesis of 6-[2-[N-ethyl-N-[2-(4-methanesulfonylaminophenyl)ethyl]amino]ethyl]-1,3-dimethyl-2,4(1H,3H)pyrimidinedione.

The 6-[2-[N-[2-(4-aminophenyl)ethyl]-N-ethylamino]ethyl]-1,3-dimethyl-2,4(1H,3H)pyrimidinedione synthesized in Example 106 was methanesulfonated in the same manner as in Example 116 to obtain the desired compound.

Example 133 (Illustrative Compound No. 9)

Synthesis of 6-[3-[N-ethyl-N-[3-(4-methanesulfonylaminophenyl)ethyl]amino]propyl]-1,3-dimethyl-2,4(1H,3H)-pyrimidinedione.

The 6-[3-[N-[2-(4-aminophenyl)ethyl]-N-ethylamino]propyl]-1,3-dimethyl-2,4(1H,3H)pyrimidinedione synthesized in Example 107 was methanesulfonated in the same manner as in Example 116 to obtain the desired compound.

Subsequently, this pyrimidinedione derivative was treated with hydrochloric acid/methanol according to conventional procedure to obtain the hydrochloride (m.p. 177-178°C) of the desired compound.

Example 134 (Illustrative Compound No. 58)

Synthesis of 6-[3-[N-n-propyl-N-[2-(4-methanesulfonylaminophenyl)ethyl]amino]propyl]-1,3-diethyl-2,4(1H,3H)-pyrimidinedione.

The 6-[3-[N-[2-(4-aminophenyl)ethyl]-N-propylamino]propyl]-1,3-diethyl-2,4(1H,3H)pyrimidinedione synthesized in Example 109 was methanesulfonated in the same manner as in Example 116 to obtain the desired compound.

Example 135 (Illustrative Compound No. 154)

Synthesis of 6-[3-[N-n-propyl-N-[2-(4-methanesulfonylaminophenyl)ethyl]amino]propyl]-1,3-diisopropyl-2,4(1H,3H)pyrimidinedione.

The 6-[3-[N-[2-(4-aminophenyl)ethyl]-N-n-propylamino] propyl]-1,3-diisopropyl-2,4(1H,3H)pyrimidinedione synthesized in Example 110 was methanesulfonated in the same manner as in Example 116 to obtain the desired compound.

Example 136 (Illustrative Compound No. 1529)

Synthesis of 6-[3-[N-(N',N'-dimethylaminoethyl)-N-[2-(4-methanesulfonylaminophenyl)ethyl]amino]propyl]-1,3-dimethyl-2,4(1H,3H)pyrimidinedione.

The 1,3-dimethyl-6-[3-[N-[2-(4-aminophenyl)ethyl]-N-dimethylaminoethylamino]propyl]-2,4(1H,3H)pyrimidinedione synthesized in Example 112 was methanesulfonated in the same manner as in Example 116 to obtain the desired compound.

Example 137 (Illustrative Compound No. 15)

Synthesis of 6-[2-[N-ethyl-N-[3-(4-methanesulfonylaminophenyl)propyl]amino]ethyl]-1,3-dimethyl-2,4(1H,3H)-pyrimidinedione.

The 6-[2-[N-[3-(4-aminophenyl)propyl]-N-ethylamino]ethyl]-1,3-dimethyl-2,4(1H,3H)pyrimidinedione synthesized in Example 113 was methanesulfonated in the same manner as in Example 116 to obtain the desired compound.

Example 138 (Illustrative Compound No. 21)

Synthesis of 6-[3-[N-ethyl-N-[3-(4-methanesulfonylaminophenyl)propyl]amino]propyl]-1,3-dimethyl-2,4(1H,3H)-pyrimidinedione.

The 6-[3-[N-[3-(4-aminophenyl)propyl]-N-ethylamino]propyl]-1,3-dimethyl-2,4(1H,3H)pyrimidinedione synthesized in Example 114 was methanesulfonated in the same manner as in Example 116 to obtain the desired compound.

Example 139 (Illustrative Compound No. 22)

Synthesis of 6-[3-[N-n-propyl-N-[3-(4-methanesulfonylaminophenyl)propyl]amino]propyl]-1,3-dimethyl-2,4(1H,3H)pyrimidinedione.

The 6-[3-[N-[3-(4-aminophenyl)propyl]-N-n-propylamino]propyl]-1,3-dimethyl-2,4(1H,3H)pyrimidinedione synthesized in Example 115 was methanesulfonated in the same manner as in Example 116 to obtain the desired compound.

Subsequently, this pyrimidinedione derivative was treated with hydrochloric acid/methanol according to conventional procedure to obtain the hydrochloride (m.p. 186-190°C) of the desired compound.

Example 140 (Illustrative Compound No. 490)

Synthesis of 6-[3-[N-[2-(4-ethanesulfonylaminophenyl)ethyl]-N-propylamino]propyl]-1,3-dimethyl-2,4(1H,3H)pyrimidinedione.

The 6-[3-[N-[2-(4-aminophenyl)ethyl]-N-propylamino]propyl]-1,3-dimethyl-2,4(1H,3H)pyrimidinedione (1.0 g) synthesized in Example 100 was added to pyridine (15 ml). Then, in an atmosphere of nitrogen, ethanesulfonyl chloride (0.54 g) was added dropwise to the resulting solution under cooling with ice. After 2 hours, the solution was warmed to room temperature and allowed to stand overnight. After standing, the reaction mixture was concentrated in vacuo and the residue was dissolved in chloroform. After the addition of a saturated aqueous solution of sodium bicarbonate, the resulting mixture was extracted three times with chloroform. The organic phases obtained from the respective extraction treatments were combined, dried over anhydrous sodium sulfate, and then concentrated. The residue was purified by silica gel column chromatography (chloroform/methanol = 50/1) to obtain the desired compound (1.14 g) as a colorless oil.

Subsequently, this pyrimidinedione derivative was treated with fumaric acid/methanol according to conventional procedure to obtain the fumarate (m.p. 109-110°C) of the desired compound.

Example 141 (Illustrative Compound No. 1465)

Synthesis of 6-[3-[N-[2-(4-benzenesulfonylaminophenyl)ethyl]-N-propylamino]propyl]-1,3-dimethyl-2,4(1H,3H)pyrimidinedione.

The desired compound was obtained by repeating the procedure of Example 140, except that benzenesulfonyl chloride was used in place of ethanesulfonyl chloride.

Subsequently, this pyrimidinedione derivative was treated with fumaric acid/methanol according to conventional procedure to obtain the fumarate (m.p. 174-176°C) of the desired compound.

The analytical results of the compounds in the titles of Examples 1-141 are shown in Table 7.

Formulation Example 1

Using the following components, tablets were prepared as described below.

| Components: | |
|---|---|
| 6-[3-[N-[2-(4-methanesulfonylaminophenyl)ethyl]-N-propylamino]propyl]-1,3-dimethyl-2,4(1H,3H)pyrimidinedione fumarate | 90 g |
| Citric acid | 1 g |
| Lactose | 40 g |
| Dibasic calcium phosphate | 73 g |
| PLURONIC F-68 | 33 g |
| Sodium laurylsulfate | 20 g |
| Polyvinylpyrrolidone | 15 g |
| Polyethylene glycol ("Carbowax 1500") | 4 g |
| Polyethylene glycol ("Carbowax 6000") | 46 g |
| Corn starch | 30 g |
| Dry magnesium stearate | 3 g |
| Ethanol | q.s. |

The above compound according to the present invention as the effective ingredient, citric acid, lactose, dibasic calcium phosphate, the "PLURONIC F-68" and sodium laurylsulfate were mixed.

The resulting mixture was sifted through a No. 60 screen. Using an ethanol solution containing polyvinylpyrrolidone, the "Carbowax 1500" and the "Carbowax 6000", the mixture so sifted was wet-granulated. Namely, the powder so-sifted was added with ethanol in such an amount as needed for the formulation of paste-like mass. The corn starch was added to the paste-like mass, followed by mixing until uniform granules were formed. The granular mixture was caused to pass through a No. 10 screen, placed in a tray, and then dried for 12-15 hours in an oven controlled at 100°C. The granules so dried were sifted through a No. 16 screen, to which dry magnesium stearate were added, followed by mixing. The mixture so obtained was then compressed into cores of a desired shape by a tableting machine.

The cores were treated with a varnish, on which talc was sprinkled to prevent absorption of moisture. Around each core, an undercoat was applied. The varnish was coated again as many times as needed for internal use. To form the resulting tablet into a fully round shape with a smooth surface, an additional undercoat and a smoothcoat were applied further. Color coating was then conducted until a desired coating was obtained. After drying, the tablets as coated were polished into tablets of uniform gloss.

Formulation Example 2

Using the following components, tablets of uniform gloss were prepared in the same manner as in Formulation Example 1:

| Components: | |
|---|---|
| 6-[3-[N-[2-(2-acethyl-4-fluorophenoxy)ethyl]-N-methylamino]propyl]-1,3-dimethyl-2,4(1H,3H)pyrimidinedione hydrochloride | 80 g |
| Citric acid | 1 g |
| Lactose | 35 g |
| Dibasic calcium phosphate | 70 g |
| PLURONIC F-68 | 31 g |
| Sodium laurylsulfate | 22 g |
| Polyvinylpyrrolidone | 13 g |
| Polyethylene glycol ("Carbowax 1500") | 5 g |
| Polyethylene glycol ("Carbowax 6000") | 45 g |
| Corn starch | 30 g |
| Dry magnesium stearate | 3 g |
| Ethanol | q.s. |

Formulation Example 3

Using the following components, an injection was prepared as described below.

| Components: | |
|---|---|
| 6-[3-[N-[2-(4-acethylphenoxy)ethyl]-N-propylamino]propyl]-1,3-dimethyl-2,4(1H,3H)pyrimidinedione hydrochloride | 4 g |
| Polyethylene glycol (molecular weight: 4000) | 0.4 g |
| Sodium chloride | 1.0 g |
| Polyoxyethylene-sorbithan monooleate | 0.6 g |
| Sodium metabisulfate | 0.1 g |
| Methylparaben | 0.2 g |
| Distilled water for injection | 10.0 ml |

Methylparaben, sodium metabisulfate and sodium chloride were dissolved under stirring at 80°C in about a half of the above amount of the distilled water for injection. The solution so obtained was cooled down to 40°C, followed by the dissolution of the above compound according to the present invention as the effective ingredient and then polyethylene glycol and polyoxyethylene-sorbitan monooleate in the solution. The residual distilled water for injection was added to the resulting solution to give the final volume. The mixture so obtained was subjected to bacterial filtration through appropriate a filter paper to sterilized the same, whereby an injection was formulated.

Formulation Example 4

Using the following components, an injection was prepared in the same manner as in Formulation Example 3:

| Components: | |
|---|---|
| 6-[3-[N-ethyl-N-[2-(4-nitrophenyl)ethyl]amino] propyl]-1,3-dimethyl-2,4(1H,3H)pyrimidinedione | 5 g |
| Polyethylene glycol (molecular weight: 4000) | 0.3 g |
| Sodium chloride | 1.1 g |
| Polyoxyethylene-sorbithan monooleate | 0.6 g |
| Sodium metabisulfate | 0.1 g |
| Methylparaben | 0.2 g |
| Distilled water for injection | 10.0 ml |

Stability Test

In order to confirm the stability of the compounds of the present invention in an acid aqueous solution, stability was tested on the fumarate of 6-[3-[N-[2-(4-methanesulfonylaminophenyl)ethyl]-N-propylamino]propyl]-1,3-dimethyl-2,4(1H,3H)pyrimidinedione (the compound obtained in Example 118; hereinafter referred to as compound A) which is a compound in accordance with the present invention, and 1,3-dimethyl-6-{2-[N-ethyl-3-phenylpropylamino]ethylamino}2,4(1H,3H)-pyrimidinedione (hereinafter referred to as compound B) which is a pyrimidinedione derivative having a substituted amino group at the 6-position of the pyrimidinedione ring as described in European Publication No. 454,498 A2 and was used for purposes of comparison.

Solution 1 (pH 1.2) described in the Japaense Pharmacopoeia was prepared by adding 7.0 ml of hydrochloric acid and water to 2.0 g of sodium chloride so as to give the final volume of 1,000 ml.

Compounds A and B were dissolved in Solution 1 at a concentration of 2 mg/ml and the resulting solutions were stirred at 37°C. Thus, changes with time in the percentages of the remaining compounds A and B were examined by HPLC. Changes with time in the percentages of the remaining compounds A and B are shown in Table 1.

Table 1

| (Stability test) | | |
|---|---|---|
| Time | Percentage of remaining compound A | Percentage of remaining compound B |
| 0 hr | 100% | 100% |
| 3 hr | 100% | 96% |
| 6 hr | 99% | 93% |

The compound of the present invention was stable even after 6 hours' heating. Compound B showed a decrease of 7% after 6 hours' heating.

Pharmacological Test 1

(1) Effect on myocardial action potential duration ($APD_{75}$)

After a mongrel adult dog was anesthetized by the intravenous administration of 30 mg/kg of pentobarbital, the heart was excised therefrom. Then, the right Purkinje fiber was isolated in a nutrient fluid (i.e., Tyrode's solution). The isolated right Purkinje fiber was fixed in a constant-temperature bath (20 ml) at 37°C and perfused with Tyrode's solution. In this state, the myocardial action potential duration ($APD_{75}$) was measured before and after the administration of each test compound. Then, the percent prolongation of myocardial action potential duration (T) after the administration of each test compound was calculated according to the following equation.

$$T(\%) = \frac{B - A}{A} \times 100$$

where

A: $APD_{75}$ before administration
B: $APD_{75}$ after administration

$APD_{75}$ was measured as follows: A field stimulus of 1 Hz was given to the right Purkinje fiber, and any change in action potential was detected by a glass microelectrode (10-20 MΩ) penetrated into a Purkinje fiber and monitored on an oscilloscope via an amplifier. The waveform on the oscilloscope was analyzed by means of a computer, and the duration beween the point of action potential generation adn the point of 75% repolarization was defined as $APD_{75}$. The test compounds shown in Table 2 were separately added to the perfusing nutrient fluid, and after 20 minutes' incubation was recorded. This test was carried out according to the method of Sato et al. [S. Sato, K. Hashimoto; Arzneimittel Forschung, 34(1), 3a, 376-380(1984)].

The results thus obtained are shown in Table 2.

Table 2

| (pharmacological test) | | | |
|---|---|---|---|
| Test compound (Example No.) | T (%) Dose (x $10^{-6}$ M) | | |
| | 1 | 10 | 100 |
| 28 (hydrochloride) | 14 | 35 | 114 |
| 38 (hydrochloride) | 6 | 16 | 22 |
| 39 (hydrochloride) | 14 | 53 | 49 |
| 47 (hydrochloride) | 13 | 53 | 107 |
| 53 (hydrochloride) | 8 | 24 | 49 |
| 60 (hydrochloride) | 13 | 34 | 47 |
| 61 (hydrochloride) | 3 | 18 | 35 |
| 76 (hydrochloride) | 52 | 114 | N.T. |
| 84 (hydrochloride) | 23 | 72 | 96 |
| 116 (fumarate) | 9 | 43 | 77 |
| 118 (fumarate) | 16 | 54 | 81 |
| 121 (hydrochloride) | 17 | 68 | 103 |
| 122 (hydrochloride) | 10 | 14 | 33 |
| 123 (hydrochloride) | 12 | 41 | 64 |
| 126 (hydrochloride) | 14 | 46 | 85 |
| 138 (hydrochloride) | 6 | 22 | 49 |
| N.T.: Not tested | | | |

(2) Effect on ventricular muscle refractory period

According to the method described below, the refractory period was measured before and after the intravenous administration of each test compound, and the percent prolongation of refractory period (ERP) was calculated according to the following equation.

$$\text{ERP prolongation (\%)} = \frac{W - Y}{Y} \times 100$$

where

W: Refractory period after administration
Y: Refractory period before administration

After a mongrel adult dog was anesthetized by the intravenous (i.v.) administration of 30 mg/kg of pentobarbital, the chest was opened on the right side. Then, a set of silver-silver chloride electrodes spaced by 3 mm was sewn on the right ventricle, and electrical stimuli were given at an interval of 400 msec. and electrical stimuli (a duration of 4 msec, and a current intensity equal to twice the threshold) were given at an interval of 400 msec. Thereafter, a small amount of alcohol was injected into a sinus artery in order to destroy the pacemaker activity, and the ventricular refractory period (ERP) was measured under ventricular pacing. Specifically, one train comprised a series of stimuli having an interval of 400 msec, and the interval between trains was usually 400 msec. During measurement of the refractory period, this interval was shortened by decrements of 10 msec, and the interval between trains at which the response to the first stimulus of the train disappeared was regarded as the refractory period. These electrical stimuli were given by programmed stimulation using a heart stimulation apparatus (Diamedical Co., Ltd.; DHM-226-3). The results thus obtained are shown in Table 3.

Table 3

| (pharmacological test) | | | |
|---|---|---|---|
| Test compound (Example No.) | ERP (%) Dose (i.v., mg/kg) | | |
| | 0.1 | 0.3 | 1.0 |
| 116 (fumarate) | 6.3 | 12.5 | 25.0 |
| 118 (fumarate) | 6.7 | 20.0 | 33.3 |
| 121 (hydrochloride) | 6.2 | 20.0 | 33.3 |
| 126 (hydrochloride) | N.T. | 5.9 | 11.8 |
| N.T.: Not tested | | | |

Toxicity Test

The test compounds were separately administered to mice (ddy strain, male). The administration of each compound was carried out intraperitoneally (i.p.) in a dose of 100 mg/kg. Twenty-four hours after the administration, the mortality rate of the mice (in groups of three) was examined. The results thus obtained are shown in Table 4.

Table 4

| (toxicity test) | |
|---|---|
| Test compound (Example No.) | Mortality rate (%) 100 mg/kg (i.p.) |
| 47 (hydrochloride) | 0 |
| 53 (hydrochloride) | 0 |
| 60 (hydrochloride) | 0 |
| 116 (fumarate) | 0 |
| 118 (fumarate) | 0 |
| 121 (hydrochloride) | 0 |

Table 5

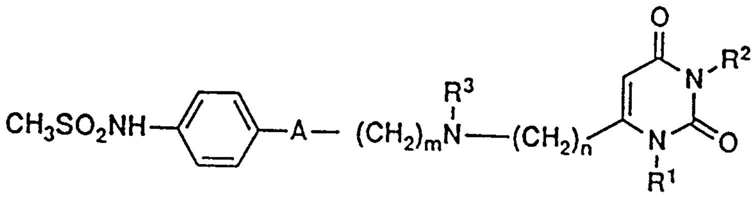

| Compound No. | A | m | n | $R^3$ | $R^2$ | $R^1$ |
|---|---|---|---|---|---|---|
| 1 | $CH_2$ | 1 | 2 | H | Me | Me |
| 2 | $CH_2$ | 1 | 2 | Me | Me | Me |
| 3 | $CH_2$ | 1 | 2 | Et | Me | Me |
| 4 | $CH_2$ | 1 | 2 | n-Pr | Me | Me |
| 5 | $CH_2$ | 1 | 4 | i-Pr | Me | Me |
| 6 | $CH_2$ | 1 | 2 | n-Bu | Me | Me |
| 7 | $CH_2$ | 1 | 3 | H | Me | Me |
| 8 | $CH_2$ | 1 | 3 | Me | Me | Me |
| 9 | $CH_2$ | 1 | 3 | Et | Me | Me |
| 10 | $CH_2$ | 1 | 3 | n-Pr | Me | Me |
| 11 | $CH_2$ | 1 | 3 | i-Pr | Me | Me |
| 12 | $CH_2$ | 1 | 3 | n-Bu | Me | Me |
| 13 | $CH_2$ | 2 | 2 | n-Hex | Me | Me |
| 14 | $CH_2$ | 2 | 4 | Me | Me | Me |
| 15 | $CH_2$ | 2 | 2 | Et | Me | Me |
| 16 | $CH_2$ | 2 | 2 | c-Pr | Me | Me |
| 17 | $CH_2$ | 2 | 2 | 1,2-diMePr | Me | Me |
| 18 | $CH_2$ | 2 | 2 | 2-MePr | Me | Me |
| 19 | $CH_2$ | 2 | 3 | H | Et | Et |
| 20 | $CH_2$ | 2 | 4 | Me | Me | Me |

Table 5 (Cont'd)

| Compound No. | A | m | n | $R^3$ | $R^2$ | $R^1$ |
|---|---|---|---|---|---|---|
| 21 | $CH_2$ | 2 | 3 | Et | Me | Me |
| 22 | $CH_2$ | 2 | 3 | n-Pr | Me | Me |
| 23 | $CH_2$ | 2 | 3 | i-Pr | Me | Me |
| 24 | $CH_2$ | 2 | 3 | n-Bu | Me | Me |
| 25 | O | 2 | 2 | 4-MePen | Me | Me |
| 26 | O | 2 | 2 | Me | Me | Me |
| 27 | O | 2 | 2 | Et | Me | Me |
| 28 | O | 2 | 2 | n-Pr | Me | Me |
| 29 | O | 2 | 2 | i-Pr | Me | Me |
| 30 | O | 2 | 4 | 1-MePr | Me | Me |
| 31 | O | 2 | 3 | H | Me | Me |
| 32 | O | 2 | 3 | Me | Me | Me |
| 33 | O | 2 | 3 | Et | Me | Me |
| 34 | O | 2 | 3 | n-Pr | Me | Me |
| 35 | O | 2 | 3 | i-Pr | Me | Me |
| 36 | O | 2 | 3 | t-Bu | Me | Me |
| 37 | O | 3 | 2 | H | Me | Me |
| 38 | O | 3 | 4 | Me | Me | Me |
| 39 | O | 3 | 2 | Et | Me | Me |
| 40 | O | 3 | 4 | n-Pr | Me | Me |
| 41 | O | 3 | 2 | n-Pen | Me | Me |
| 42 | O | 4 | 2 | n-Bu | Me | Me |
| 43 | O | 3 | 3 | c-Hex | Me | Me |
| 44 | O | 3 | 3 | Me | Me | Me |

Table 5 (Cont'd)

| Compound No. | A | m | n | $R^3$ | $R^2$ | $R^1$ |
|---|---|---|---|---|---|---|
| 45 | O | 3 | 3 | Et | Me | Me |
| 46 | O | 3 | 3 | n-Pr | Me | Me |
| 47 | O | 3 | 3 | 2,2-diMePr | Me | Me |
| 48 | O | 4 | 3 | n-Bu | Me | Me |
| 49 | $CH_2$ | 1 | 2 | H | Et | Et |
| 50 | $CH_2$ | 1 | 2 | Me | n-Hex | n-Hex |
| 51 | $CH_2$ | 1 | 4 | Et | Et | Et |
| 52 | $CH_2$ | 1 | 2 | n-Pr | Et | Et |
| 53 | $CH_2$ | 1 | 2 | c-Pen | Et | Et |
| 54 | $CH_2$ | 1 | 2 | 2-MePr | Et | Et |
| 55 | $CH_2$ | 1 | 4 | H | Et | Et |
| 56 | $CH_2$ | 1 | 3 | Me | Et | Et |
| 57 | $CH_2$ | 1 | 3 | Et | Et | Et |
| 58 | $CH_2$ | 1 | 3 | n-Pr | Et | Et |
| 59 | $CH_2$ | 1 | 3 | i-Pr | Et | Et |
| 60 | $CH_2$ | 1 | 3 | n-Bu | Et | Et |
| 61 | $CH_2$ | 2 | 2 | H | Et | Et |
| 62 | $CH_2$ | 2 | 2 | Me | t-Bu | t-Bu |
| 63 | $CH_2$ | 2 | 4 | Et | Et | Et |
| 64 | $CH_2$ | 2 | 2 | n-Pr | Et | Et |
| 65 | $CH_2$ | 2 | 2 | c-Pen | Et | Et |
| 66 | $CH_2$ | 2 | 2 | t-Bu | Et | Et |
| 67 | $CH_2$ | 2 | 3 | 3-MeBu | Et | Et |
| 68 | $CH_2$ | 2 | 4 | Me | Et | Et |

Table 5 (Cont'd)

| Compound No. | A | m | n | $R^3$ | $R^2$ | $R^1$ |
|---|---|---|---|---|---|---|
| 69 | $CH_2$ | 2 | 4 | Et | Et | Et |
| 70 | $CH_2$ | 2 | 3 | n-Pr | Et | Et |
| 71 | $CH_2$ | 2 | 3 | 1,1-diMePr | Et | Et |
| 72 | $CH_2$ | 2 | 3 | n-Bu | Et | Et |
| 73 | O | 2 | 2 | H | Et | Et |
| 74 | O | 2 | 4 | Me | Et | Et |
| 75 | O | 2 | 2 | Et | Et | Et |
| 76 | O | 2 | 4 | n-Pr | Et | Et |
| 77 | O | 2 | 2 | i-Pr | Et | Et |
| 78 | O | 2 | 2 | n-Bu | Et | Et |
| 79 | O | 2 | 3 | 2-MePr | Et | Et |
| 80 | O | 2 | 3 | Me | Et | Et |
| 81 | O | 2 | 3 | Et | Et | Et |
| 82 | O | 2 | 3 | c-Pr | Et | Et |
| 83 | O | 2 | 4 | i-Pr | Et | Et |
| 84 | O | 2 | 3 | n-Bu | Et | Et |
| 85 | O | 3 | 2 | H | Et | Et |
| 86 | O | 3 | 2 | Me | Et | Et |
| 87 | O | 3 | 2 | Et | Et | Et |
| 88 | O | 3 | 4 | n-Pr | Et | Et |
| 89 | O | 4 | 2 | i-Pr | Et | Et |
| 90 | O | 3 | 2 | n-Bu | Et | Et |
| 91 | O | 3 | 3 | H | Et | Et |
| 92 | O | 4 | 3 | Me | Et | Et |

Table 5 (Cont'd)

| Compound No. | A | m | n | $R^3$ | $R^2$ | $R^1$ |
|---|---|---|---|---|---|---|
| 93 | O | 3 | 3 | Et | Et | Et |
| 94 | O | 3 | 3 | n-Pr | Et | Et |
| 95 | O | 3 | 3 | 2,2-diMePr | Et | Et |
| 96 | O | 3 | 3 | 1-MePr | Et | Et |
| 97 | $CH_2$ | 1 | 2 | H | n-Pr | n-Pr |
| 98 | $CH_2$ | 1 | 2 | Me | i-Pr | i-Pr |
| 99 | $CH_2$ | 1 | 4 | Et | n-Pr | n-Pr |
| 100 | $CH_2$ | 1 | 2 | n-Pr | c-Pr | c-Pr |
| 101 | $CH_2$ | 1 | 2 | i-Pr | i-Pr | i-Pr |
| 102 | $CH_2$ | 1 | 4 | n-Bu | n-Pr | n-Pr |
| 103 | $CH_2$ | 1 | 3 | H | n-Pen | n-Pen |
| 104 | $CH_2$ | 1 | 3 | Me | n-Pr | n-Pr |
| 105 | $CH_2$ | 1 | 3 | Et | n-Pr | n-Pr |
| 106 | $CH_2$ | 1 | 4 | n-Pr | n-Pr | n-Pr |
| 107 | $CH_2$ | 1 | 3 | i-Pr | n-Pr | n-Pr |
| 108 | $CH_2$ | 1 | 3 | n-Bu | i-Pr | i-Pr |
| 109 | $CH_2$ | 2 | 2 | H | n-Pr | n-Pr |
| 110 | $CH_2$ | 2 | 2 | Me | c-Pen | c-Pen |
| 111 | $CH_2$ | 2 | 4 | Et | n-Pr | n-Pr |
| 112 | $CH_2$ | 2 | 2 | n-Pr | n-Pr | n-Pr |
| 113 | $CH_2$ | 2 | 4 | i-Pr | n-Pr | n-Pr |
| 114 | $CH_2$ | 2 | 2 | n-Bu | n-Pr | n-Pr |
| 115 | $CH_2$ | 2 | 3 | H | i-Pr | i-Pr |
| 116 | $CH_2$ | 2 | 4 | Me | n-Pr | n-Pr |

Table 5 (Cont'd)

| Compound No. | A | m | n | $R^3$ | $R^2$ | $R^1$ |
|---|---|---|---|---|---|---|
| 117 | $CH_2$ | 2 | 3 | Et | 3-MeBu | 3-MeBu |
| 118 | $CH_2$ | 2 | 3 | n-Pr | n-Pr | n-Pr |
| 119 | $CH_2$ | 2 | 4 | i-Pr | n-Pr | n-Pr |
| 120 | $CH_2$ | 2 | 3 | n-Bu | n-Pr | n-Pr |
| 121 | O | 2 | 2 | H | n-Pr | n-Pr |
| 122 | O | 2 | 2 | Me | i-Pr | i-Pr |
| 123 | O | 2 | 2 | Et | n-Pr | n-Pr |
| 124 | O | 2 | 4 | n-Pr | n-Pr | n-Pr |
| 125 | O | 2 | 2 | i-Pr | 2-MeBu | 2-MeBu |
| 126 | O | 2 | 2 | n-Bu | n-Pr | n-Pr |
| 127 | O | 2 | 3 | H | n-Pr | n-Pr |
| 128 | O | 2 | 3 | Me | n-Pr | n-Pr |
| 129 | O | 2 | 4 | Et | i-Pr | i-Pr |
| 130 | O | 2 | 4 | n-Pr | n-Pr | n-Pr |
| 131 | O | 2 | 3 | i-Pr | n-Pr | n-Pr |
| 132 | O | 2 | 3 | n-Bu | 1,2-diMePr | 1,2-diMePr |
| 133 | O | 3 | 2 | H | n-Pr | n-Pr |
| 134 | O | 3 | 2 | Me | n-Pr | n-Pr |
| 135 | O | 3 | 4 | Et | n-Pr | n-Pr |
| 136 | O | 3 | 2 | n-Pr | n-Pr | n-Pr |
| 137 | O | 3 | 2 | i-Pr | n-Pr | n-Pr |
| 138 | O | 3 | 4 | n-Bu | n-Pr | n-Pr |
| 139 | O | 3 | 4 | H | n-Pr | n-Pr |
| 140 | O | 3 | 3 | Me | n-Pr | n-Pr |

Table 5 (Cont'd)

| Compound No. | A | m | n | $R^3$ | $R^2$ | $R^1$ |
|---|---|---|---|---|---|---|
| 141 | O | 3 | 4 | Et | n-Pr | n-Pr |
| 142 | O | 3 | 3 | n-Pr | 1,1-diMePr | 1,1-diMePr |
| 143 | O | 3 | 3 | i-Pr | i-Pr | i-Pr |
| 144 | O | 3 | 3 | n-Bu | 2,2-diMePr | 2,2-diMePr |
| 145 | $CH_2$ | 1 | 2 | H | n-Bu | n-Bu |
| 146 | $CH_2$ | 1 | 2 | Me | c-Bu | c-Bu |
| 147 | $CH_2$ | 1 | 2 | Et | 2-MePr | 2-MePr |
| 148 | $CH_2$ | 1 | 2 | n-Pr | n-Hex | n-Hex |
| 149 | $CH_2$ | 1 | 2 | n-Pen | c-Hex | c-Hex |
| 150 | $CH_2$ | 1 | 2 | n-Bu | 4-MePen | 4-MePen |
| 151 | $CH_2$ | 1 | 4 | H | 3-MePen | 3-MePen |
| 152 | $CH_2$ | 1 | 3 | Me | 2-MePen | 2-MePen |
| 153 | $CH_2$ | 1 | 3 | Et | 1-MePr | 1-MePr |
| 154 | $CH_2$ | 1 | 3 | n-Pr | i-Pr | i-Pr |
| 155 | $CH_2$ | 1 | 3 | i-Pr | c-Bu | c-Bu |
| 156 | $CH_2$ | 1 | 3 | c-Bu | 1-MePen | 1-MePen |
| 157 | $CH_2$ | 2 | 2 | H | 1,2,2-triMePr | 1,2,2-triMePr |
| 158 | $CH_2$ | 2 | 2 | Me | 1,1-diMeBu | 1,1-diMeBu |
| 159 | $CH_2$ | 2 | 2 | Et | 1,1,2-triMePr | 1,1,2-triMePr |
| 160 | $CH_2$ | 2 | 2 | n-Pr | 2,2-diMeBu | 2,2-diMeBu |
| 161 | $CH_2$ | 2 | 2 | i-Pr | 1,3-diMeBu | 1,3-diMeBu |
| 162 | $CH_2$ | 2 | 2 | n-Bu | 2,3-diMeBu | 2,3-diMeBu |
| 163 | $CH_2$ | 2 | 3 | H | n-Bu | n-Bu |
| 164 | $CH_2$ | 2 | 3 | Me | n-Hex | n-Hex |

Table 5 (Cont'd)

| Compound No. | A | m | n | $R^3$ | $R^2$ | $R^1$ |
|---|---|---|---|---|---|---|
| 165 | $CH_2$ | 2 | 3 | Et | t-Bu | t-Bu |
| 166 | $CH_2$ | 2 | 3 | n-Pr | c-Hex | c-Hex |
| 167 | $CH_2$ | 2 | 3 | i-Pr | 2-MePr | 2-MePr |
| 168 | $CH_2$ | 2 | 4 | n-Bu | 1-MePr | 1-MePr |
| 169 | O | 2 | 2 | n-Hex | t-Bu | t-Bu |
| 170 | O | 2 | 2 | Me | n-Hex | n-Hex |
| 171 | O | 2 | 2 | Et | i-Pr | i-Pr |
| 172 | O | 2 | 2 | n-Pr | n-Bu | n-Bu |
| 173 | O | 2 | 2 | i-Pr | 2-MePr | 2-MePr |
| 174 | O | 2 | 2 | t-Bu | 1-MePr | 1-MePr |
| 175 | O | 2 | 3 | c-Hex | t-Bu | t-Bu |
| 176 | O | 2 | 4 | Me | 4-MePen | 4-MePen |
| 177 | O | 2 | 3 | Et | 3-MePen | 3-MePen |
| 178 | O | 2 | 3 | n-Pr | 2-MePen | 2-MePen |
| 179 | O | 2 | 4 | i-Pr | 1-MePen | 1-MePen |
| 180 | O | 2 | 3 | n-Bu | 1,2,2-triMePr | 1,2,2-triMePr |
| 181 | O | 3 | 2 | H | 1,1-diMeBu | 1,1-diMeBu |
| 182 | O | 3 | 4 | Me | 1,1,2-triMePr | 1,1,2-triMePr |
| 183 | O | 3 | 2 | Et | 1,1-diMeBu | 1,1-diMeBu |
| 184 | O | 3 | 2 | n-Pr | 1,3-diMeBu | 1,3-diMeBu |
| 185 | O | 4 | 2 | i-Pr | 2,3-diMeBu | 2,3-diMeBu |
| 186 | O | 3 | 2 | n-Bu | c-Hex | c-Hex |
| 187 | O | 3 | 3 | H | c-Bu | c-Bu |
| 188 | O | 3 | 3 | Me | n-Bu | n-Bu |

Table 5 (Cont'd)

| Compound No. | A | m | n | $R^3$ | $R^2$ | $R^1$ |
|---|---|---|---|---|---|---|
| 189 | O | 3 | 3 | Et | t-Bu | t-Bu |
| 190 | O | 3 | 3 | n-Pr | 2-MePr | 2-MePr |
| 191 | O | 3 | 3 | c-Pen | i-Pr | i-Pr |
| 192 | O | 4 | 3 | n-Bu | n-Bu | n-Bu |
| 193 | $CH_2$ | 1 | 2 | H | Me | H |
| 194 | $CH_2$ | 1 | 2 | Me | Me | Et |
| 195 | $CH_2$ | 1 | 2 | Et | Me | n-Pr |
| 196 | $CH_2$ | 1 | 2 | n-Pr | Me | t-Bu |
| 197 | $CH_2$ | 1 | 2 | i-Pr | Me | n-Pen |
| 198 | $CH_2$ | 1 | 2 | n-Bu | Me | n-Hex |
| 199 | $CH_2$ | 1 | 4 | H | H | Me |
| 200 | $CH_2$ | 1 | 3 | Me | Et | Me |
| 201 | $CH_2$ | 1 | 3 | Et | i-Pr | Me |
| 202 | $CH_2$ | 1 | 4 | n-Pr | n-Bu | Me |
| 203 | $CH_2$ | 1 | 3 | i-Pr | c-Pen | Me |
| 204 | $CH_2$ | 1 | 3 | n-Bu | c-Hex | Me |
| 205 | $CH_2$ | 2 | 2 | H | Et | H |
| 206 | $CH_2$ | 2 | 2 | Me | Et | i-Pr |
| 207 | $CH_2$ | 2 | 2 | Et | Et | 2-MePr |
| 208 | $CH_2$ | 2 | 2 | n-Pr | Et | 3-MeBu |
| 209 | $CH_2$ | 2 | 2 | i-Pr | Et | n-Hex |
| 210 | $CH_2$ | 2 | 2 | n-Bu | H | Et |
| 211 | $CH_2$ | 2 | 3 | H | n-Pr | Et |
| 212 | $CH_2$ | 2 | 4 | Me | 1-MePr | Et |

Table 5 (Cont'd)

| Compound No. | A | m | n | $R^3$ | $R^2$ | $R^1$ |
|---|---|---|---|---|---|---|
| 213 | $CH_2$ | 2 | 3 | Et | 2-MeBu | Et |
| 214 | $CH_2$ | 2 | 3 | n-Pr | 1,2-diMePr | Et |
| 215 | $CH_2$ | 2 | 3 | i-Pr | 1,1-diMePr | Et |
| 216 | $CH_2$ | 2 | 4 | n-Bu | c-Hex | Et |
| 217 | O | 2 | 2 | H | n-Pr | H |
| 218 | O | 2 | 2 | Me | i-Pr | n-Bu |
| 219 | O | 2 | 2 | Et | c-Pr | n-Pen |
| 220 | O | 2 | 2 | n-Pr | n-Pr | c-Hex |
| 221 | O | 2 | 2 | i-Pr | H | i-Pr |
| 222 | O | 2 | 2 | n-Bu | 2-MePr | c-Pr |
| 223 | O | 2 | 3 | H | c-Hex | n-Pr |
| 224 | O | 2 | 3 | Me | 1-MePr | H |
| 225 | O | 2 | 4 | Et | t-Bu | n-Pen |
| 226 | O | 2 | 3 | n-Pr | c-Bu | 4-MePen |
| 227 | O | 2 | 4 | i-Pr | H | t-Bu |
| 228 | O | 2 | 3 | n-Bu | 2,2-diMePr | n-Bu |
| 229 | O | 4 | 2 | H | 3-MePen | 2-MePr |
| 230 | O | 3 | 2 | Me | 2,2-diMePr | H |
| 231 | O | 3 | 2 | Et | c-Pen | 1,1-diMeBu |
| 232 | O | 3 | 2 | n-Pr | 1,1,2-triMePr | H |
| 233 | O | 3 | 2 | i-Pr | 1,2-diMeBu | n-Pen |
| 234 | O | 3 | 2 | n-Bu | H | 3-MeBu |
| 235 | O | 3 | 3 | H | H | 1,3-diMeBu |

Table 5 (Cont'd)

| Compound No. | A | m | n | $R^3$ | $R^2$ | $R^1$ |
|---|---|---|---|---|---|---|
| 236 | O | 4 | 3 | Me | H | H |
| 237 | O | 3 | 3 | Et | Me | Et |
| 238 | O | 3 | 3 | n-Pr | Et | Me |
| 239 | O | 4 | 4 | i-Pr | Me | n-Pr |
| 240 | O | 3 | 3 | n-Bu | i-Pr | Me |

Table 6

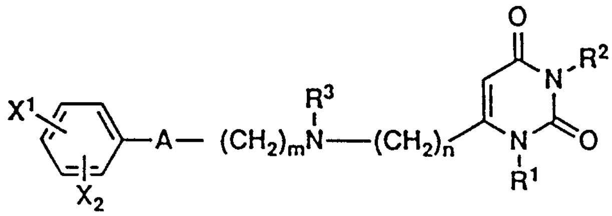

| Comp. No. | A | m | n | $R^3$ | $X^1$ | $X^2$ | $R^2$ | $R^1$ |
|---|---|---|---|---|---|---|---|---|
| 241 | $CH_2$ | 1 | 2 | Me | (4)-$NO_2$ | H | Et | Et |
| 242 | $CH_2$ | 1 | 2 | H | (4)-$NO_2$ | H | Et | Et |
| 243 | $CH_2$ | 1 | 2 | n-Pr | (4)-$NO_2$ | H | Et | Et |
| 244 | $CH_2$ | 1 | 2 | n-Pen | (4)-$NO_2$ | H | Et | Et |
| 245 | $CH_2$ | 1 | 3 | Me | (4)-$NO_2$ | H | Et | Et |
| 246 | $CH_2$ | 1 | 4 | Et | (4)-$NO_2$ | H | Et | Et |
| 247 | $CH_2$ | 1 | 3 | n-Pr | (4)-$NO_2$ | H | Et | Et |
| 248 | $CH_2$ | 1 | 3 | n-He | (4)-$NO_2$ | H | Et | Et |
| 249 | $CH_2$ | 2 | 2 | Me | (4)-$NO_2$ | H | Et | Et |
| 250 | $CH_2$ | 2 | 2 | Et | (4)-$NO_2$ | H | Et | Et |
| 251 | $CH_2$ | 2 | 2 | i-Pr | (4)-$NO_2$ | H | Et | Et |
| 252 | $CH_2$ | 2 | 2 | n-Bu | (4)-$NO_2$ | H | Et | Et |
| 253 | $CH_2$ | 2 | 4 | Me | (4)-$NO_2$ | H | Et | Et |
| 254 | $CH_2$ | 2 | 3 | Et | (4)-$NO_2$ | H | Et | Et |
| 255 | $CH_2$ | 2 | 3 | c-Pr | (4)-$NO_2$ | H | Et | Et |
| 256 | $CH_2$ | 2 | 3 | t-Bu | (4)-$NO_2$ | H | Et | Et |
| 257 | O | 2 | 2 | Me | (4)-$NO_2$ | H | Et | Et |
| 258 | O | 2 | 2 | Et | (4)-$NO_2$ | H | Et | Et |
| 259 | O | 2 | 2 | n-Pr | (4)-$NO_2$ | H | Et | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 260 | O | 2 | 2 | c-Pen | (4)-$NO_2$ | H | Et | Et |
| 261 | O | 2 | 3 | Me | (4)-$NO_2$ | H | Et | Et |
| 262 | O | 2 | 3 | Et | (4)-$NO_2$ | H | Et | Et |
| 263 | O | 2 | 4 | n-Pr | (4)-$NO_2$ | H | Et | Et |
| 264 | O | 2 | 3 | c-Hex | (4)-$NO_2$ | H | Et | Et |
| 265 | O | 4 | 2 | Me | (4)-$NO_2$ | H | Et | Et |
| 266 | O | 3 | 2 | H | (4)-$NO_2$ | H | Et | Et |
| 267 | O | 3 | 2 | i-Pr | (4)-$NO_2$ | H | Et | Et |
| 268 | O | 3 | 2 | 1-MePr | (4)-$NO_2$ | H | Et | Et |
| 269 | O | 3 | 3 | Me | (4)-$NO_2$ | H | Et | Et |
| 270 | O | 4 | 3 | Et | (4)-$NO_2$ | H | Et | Et |
| 271 | O | 3 | 3 | n-Pr | (4)-$NO_2$ | H | Et | Et |
| 272 | O | 3 | 4 | 2-MePr | (4)-$NO_2$ | H | Et | Et |
| 273 | $CH_2$ | 1 | 2 | Me | (4)-$NO_2$ | H | n-Pr | n-Pr |
| 274 | $CH_2$ | 1 | 2 | Et | (4)-$NO_2$ | H | i-Pr | i-Pr |
| 275 | $CH_2$ | 1 | 2 | i-Pr | (4)-$NO_2$ | H | c-Pr | c-Pr |
| 276 | $CH_2$ | 1 | 2 | n-Hex | (4)-$NO_2$ | H | n-Pr | n-Pr |
| 277 | $CH_2$ | 1 | 3 | Me | (4)-$NO_2$ | H | i-Pr | n-Pr |
| 278 | $CH_2$ | 1 | 4 | Et | (4)-$NO_2$ | H | i-Pr | i-Pr |
| 279 | $CH_2$ | 1 | 3 | n-Pr | (4)-$NO_2$ | H | i-Pr | i-Pr |
| 280 | $CH_2$ | 1 | 3 | c-Hex | (4)-$NO_2$ | H | n-Pr | n-Pr |
| 281 | $CH_2$ | 2 | 2 | Me | (4)-$NO_2$ | H | n-Pr | i-Pr |
| 282 | $CH_2$ | 2 | 2 | Et | (4)-$NO_2$ | H | i-Pr | i-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 283 | $CH_2$ | 2 | 2 | n-Pr | (4)-$NO_2$ | H | n-Pr | n-Pr |
| 284 | $CH_2$ | 2 | 2 | t-Bu | (4)-$NO_2$ | H | n-Pr | n-Pr |
| 285 | $CH_2$ | 2 | 4 | Me | (4)-$NO_2$ | H | i-Pr | i-Pr |
| 286 | $CH_2$ | 2 | 3 | Et | (4)-$NO_2$ | H | n-Pr | n-Pr |
| 287 | $CH_2$ | 2 | 3 | i-Pr | (4)-$NO_2$ | H | n-Pr | n-Pr |
| 288 | $CH_2$ | 2 | 3 | n-Pen | (4)-$NO_2$ | H | n-Pr | n-Pr |
| 289 | O | 2 | 2 | Me | (4)-$NO_2$ | H | i-Pr | i-Pr |
| 290 | O | 2 | 2 | H | (4)-$NO_2$ | H | n-Pr | n-Pr |
| 291 | O | 2 | 2 | n-Pr | (4)-$NO_2$ | H | n-Pr | n-Pr |
| 292 | O | 2 | 2 | n-Hex | (4)-$NO_2$ | H | n-Pr | n-Pr |
| 293 | O | 2 | 3 | Me | (4)-$NO_2$ | H | n-Pr | n-Pr |
| 294 | O | 2 | 3 | Et | (4)-$NO_2$ | H | i-Pr | i-Pr |
| 295 | O | 2 | 4 | n-Pr | (4)-$NO_2$ | H | n-Pr | n-Pr |
| 296 | O | 2 | 3 | 2-MePr | (4)-$NO_2$ | H | n-Pr | n-Pr |
| 297 | O | 3 | 2 | Me | (4)-$NO_2$ | H | n-Pr | n-Pr |
| 298 | O | 3 | 2 | Et | (4)-$NO_2$ | H | n-Pr | n-Pr |
| 299 | O | 4 | 2 | n-Pr | (4)-$NO_2$ | H | i-Pr | i-Pr |
| 300 | O | 3 | 2 | c-Hex | (4)-$NO_2$ | H | n-Pr | n-Pr |
| 301 | O | 3 | 3 | Me | (4)-$NO_2$ | H | n-Pr | n-Pr |
| 302 | O | 4 | 3 | Et | (4)-$NO_2$ | H | n-Pr | n-Pr |
| 303 | O | 3 | 3 | n-Pr | (4)-$NO_2$ | H | n-Pr | n-Pr |
| 304 | O | 3 | 3 | c-Bu | (4)-$NO_2$ | H | n-Pr | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 305 | $CH_2$ | 1 | 2 | Me | (4)-$NO_2$ | H | n-Bu | n-Bu |
| 306 | $CH_2$ | 1 | 2 | Et | (4)-$NO_2$ | H | t-Bu | t-Bu |
| 307 | $CH_2$ | 1 | 2 | n-Pr | (4)-$NO_2$ | H | c-Bu | c-Bu |
| 308 | $CH_2$ | 1 | 2 | n-Pen | (4)-$NO_2$ | H | 2-MePr | 2-MePr |
| 309 | $CH_2$ | 1 | 3 | Me | (4)-$NO_2$ | H | n-Pen | n-Pen |
| 310 | $CH_2$ | 1 | 4 | Et | (4)-$NO_2$ | H | c-Pen | c-Pen |
| 311 | $CH_2$ | 1 | 3 | n-Pr | (4)-$NO_2$ | H | n-Hex | n-Hex |
| 312 | $CH_2$ | 1 | 3 | n-Hex | (4)-$NO_2$ | H | c-Hex | c-Hex |
| 313 | $CH_2$ | 2 | 2 | Me | (4)-$NO_2$ | H | 1-MePr | 1-MePr |
| 314 | $CH_2$ | 2 | 2 | Et | (4)-$NO_2$ | H | n-Bu | n-Bu |
| 315 | $CH_2$ | 2 | 2 | n-Pr | (4)-$NO_2$ | H | t-Bu | t-Bu |
| 316 | $CH_2$ | 2 | 2 | t-Bu | (4)-$NO_2$ | H | c-Hex | c-Hex |
| 317 | $CH_2$ | 2 | 3 | Me | (4)-$NO_2$ | H | n-Pen | n-Pen |
| 318 | $CH_2$ | 2 | 4 | Et | (4)-$NO_2$ | H | t-Bu | t-Bu |
| 319 | $CH_2$ | 2 | 3 | n-Pr | (4)-$NO_2$ | H | 2-MePr | 2-MePr |
| 320 | $CH_2$ | 2 | 3 | 1-MePr | (4)-$NO_2$ | H | c-Bu | c-Bu |
| 321 | O | 2 | 2 | Me | (4)-$NO_2$ | H | c-Pen | c-Pen |
| 322 | O | 2 | 2 | Et | (4)-$NO_2$ | H | n-Pen | n-Pen |
| 323 | O | 2 | 2 | n-Pr | (4)-$NO_2$ | H | n-Bu | Me |
| 324 | O | 2 | 2 | n-Bu | (4)-$NO_2$ | H | Et | n-Hex |
| 325 | O | 2 | 4 | Me | (4)-$NO_2$ | H | n-Pr | t-Bu |
| 326 | O | 2 | 3 | H | (4)-$NO_2$ | H | Me | Et |
| 327 | O | 2 | 3 | n-Pr | (4)-$NO_2$ | H | Et | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 328 | O | 2 | 3 | n-He | (4)-$NO_2$ | H | Me | t-Bu |
| 329 | O | 3 | 2 | Me | (4)-$NO_2$ | H | n-Bu | n-Hex |
| 330 | O | 3 | 2 | Et | (4)-$NO_2$ | H | n-Hex | n-Bu |
| 331 | O | 4 | 2 | i-Pr | (4)-$NO_2$ | H | c-Pen | Me |
| 332 | O | 3 | 2 | t-Bu | (4)-$NO_2$ | H | Me | c-Pen |
| 333 | O | 3 | 3 | Me | (4)-$NO_2$ | H | Me | n-Hex |
| 334 | O | 4 | 4 | Et | (4)-$NO_2$ | H | n-Hex | Me |
| 335 | O | 3 | 3 | n-Pr | (4)-$NO_2$ | H | c-Hex | Et |
| 336 | O | 3 | 3 | n-Pe | (4)-$NO_2$ | H | Et | c-Hex |
| 337 | $CH_2$ | 1 | 2 | Me | (4)-$NH_2$ | H | Et | Et |
| 338 | $CH_2$ | 1 | 2 | Et | (4)-$NH_2$ | H | Et | Et |
| 339 | $CH_2$ | 1 | 2 | n-Pr | (4)-$NH_2$ | H | Et | Et |
| 340 | $CH_2$ | 1 | 2 | i-Pr | (4)-$NH_2$ | H | Et | Et |
| 341 | $CH_2$ | 1 | 4 | Me | (4)-$NH_2$ | H | Et | Et |
| 342 | $CH_2$ | 1 | 3 | Et | (4)-$NH_2$ | H | Et | Et |
| 343 | $CH_2$ | 1 | 3 | n-Pr | (4)-$NH_2$ | H | Et | Et |
| 344 | $CH_2$ | 1 | 3 | i-Pr | (4)-$NH_2$ | H | Et | Et |
| 345 | $CH_2$ | 2 | 2 | Me | (4)-$NH_2$ | H | Et | Et |
| 346 | $CH_2$ | 2 | 2 | Et | (4)-$NH_2$ | H | Et | Et |
| 347 | $CH_2$ | 2 | 2 | n-Pr | (4)-$NH_2$ | H | Et | Et |
| 348 | $CH_2$ | 2 | 2 | i-Pr | (4)-$NH_2$ | H | Et | Et |
| 349 | $CH_2$ | 2 | 3 | Me | (4)-$NH_2$ | H | Et | Et |
| 350 | $CH_2$ | 2 | 3 | Et | (4)-$NH_2$ | H | Et | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 351 | $CH_2$ | 2 | 3 | n-Pr | (4)-$NO_2$ | H | Et | Et |
| 352 | $CH_2$ | 2 | 4 | i-Pr | (4)-$NO_2$ | H | Et | Et |
| 353 | O | 2 | 2 | Me | (4)-$NH_2$ | H | Et | Et |
| 354 | O | 2 | 2 | Et | (4)-$NH_2$ | H | Et | Et |
| 355 | O | 2 | 2 | n-Pr | (4)-$NH_2$ | H | Et | Et |
| 356 | O | 2 | 2 | i-Pr | (4)-$NH_2$ | H | Et | Et |
| 357 | O | 2 | 3 | Me | (4)-$NH_2$ | H | Et | Et |
| 358 | O | 2 | 3 | Et | (4)-$NH_2$ | H | Et | Et |
| 359 | O | 2 | 4 | n-Pr | (4)-$NH_2$ | H | Et | Et |
| 360 | O | 2 | 3 | i-Pr | (4)-$NH_2$ | H | Et | Et |
| 361 | O | 3 | 2 | Me | (4)-$NH_2$ | H | Et | Et |
| 362 | O | 4 | 2 | Et | (4)-$NH_2$ | H | Et | Et |
| 363 | O | 3 | 2 | n-Pr | (4)-$NH_2$ | H | Et | Et |
| 364 | O | 3 | 2 | i-Pr | (4)-$NH_2$ | H | Et | Et |
| 365 | O | 4 | 4 | Me | (4)-$NH_2$ | H | Et | Et |
| 366 | O | 3 | 3 | Et | (4)-$NH_2$ | H | Et | Et |
| 367 | O | 3 | 3 | n-Pr | (4)-$NH_2$ | H | Et | Et |
| 368 | O | 3 | 3 | i-Pr | (4)-$NH_2$ | H | Et | Et |
| 369 | $CH_2$ | 1 | 2 | Me | (4)-$NH_2$ | H | n-Pr | n-Pr |
| 370 | $CH_2$ | 1 | 2 | Et | (4)-$NH_2$ | H | i-Pr | i-Pr |
| 371 | $CH_2$ | 1 | 2 | n-Pr | (4)-$NH_2$ | H | c-Pr | c-Pr |
| 372 | $CH_2$ | 1 | 2 | i-Pr | (4)-$NH_2$ | H | n-Pr | n-Pr |
| 373 | $CH_2$ | 1 | 3 | Me | (4)-$NH_2$ | H | i-Pr | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 374 | $CH_2$ | 1 | 3 | Et | (4)-$NH_2$ | H | i-Pr | i-Pr |
| 375 | $CH_2$ | 1 | 3 | n-Pr | (4)-$NH_2$ | H | i-Pr | i-Pr |
| 376 | $CH_2$ | 1 | 4 | i-Pr | (4)-$NH_2$ | H | n-Pr | n-Pr |
| 377 | $CH_2$ | 2 | 2 | Me | (4)-$NH_2$ | H | n-Pr | i-Pr |
| 378 | $CH_2$ | 2 | 2 | Et | (4)-$NH_2$ | H | i-Pr | i-Pr |
| 379 | $CH_2$ | 2 | 2 | n-Pr | (4)-$NH_2$ | H | n-Pr | n-Pr |
| 380 | $CH_2$ | 2 | 2 | i-Pr | (4)-$NH_2$ | H | n-Pr | n-Pr |
| 381 | $CH_2$ | 2 | 3 | Me | (4)-$NH_2$ | H | i-Pr | i-Pr |
| 382 | $CH_2$ | 2 | 3 | Et | (4)-$NH_2$ | H | n-Pr | n-Pr |
| 383 | $CH_2$ | 2 | 4 | n-Pr | (4)-$NH_2$ | H | n-Pr | n-Pr |
| 384 | $CH_2$ | 2 | 3 | i-Pr | (4)-$NH_2$ | H | n-Pr | n-Pr |
| 385 | O | 2 | 2 | Me | (4)-$NH_2$ | H | i-Pr | i-Pr |
| 386 | O | 2 | 2 | Et | (4)-$NH_2$ | H | n-Pr | n-Pr |
| 387 | O | 2 | 2 | n-Pr | (4)-$NH_2$ | H | n-Pr | n-Pr |
| 388 | O | 2 | 2 | i-Pr | (4)-$NH_2$ | H | n-Pr | n-Pr |
| 389 | O | 2 | 4 | Me | (4)-$NH_2$ | H | n-Pr | n-Pr |
| 390 | O | 2 | 3 | Et | (4)-$NH_2$ | H | i-Pr | i-Pr |
| 391 | O | 2 | 3 | n-Pr | (4)-$NH_2$ | H | n-Pr | n-Pr |
| 392 | O | 2 | 3 | i-Pr | (4)-$NH_2$ | H | n-Pr | n-Pr |
| 393 | O | 3 | 2 | Me | (4)-$NH_2$ | H | n-Pr | n-Pr |
| 394 | O | 4 | 2 | Et | (4)-$NH_2$ | H | n-Pr | n-Pr |
| 395 | O | 3 | 2 | n-Pr | (4)-$NH_2$ | H | i-Pr | i-Pr |
| 396 | O | 3 | 2 | i-Pr | (4)-$NH_2$ | H | n-Pr | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 397 | O | 3 | 3 | Me | (4)-$NH_2$ | H | n-Pr | n-Pr |
| 398 | O | 3 | 3 | Et | (4)-$NH_2$ | H | n-Pr | n-Pr |
| 399 | O | 4 | 4 | n-Pr | (4)-$NH_2$ | H | n-Pr | n-Pr |
| 400 | O | 3 | 3 | i-Pr | (4)-$NH_2$ | H | n-Pr | n-Pr |
| 401 | $CH_2$ | 1 | 2 | Me | (4)-$NH_2$ | H | n-Bu | n-Bu |
| 402 | $CH_2$ | 1 | 2 | Et | (4)-$NH_2$ | H | t-Bu | t-Bu |
| 403 | $CH_2$ | 1 | 2 | n-Pr | (4)-$NH_2$ | H | c-Bu | c-Bu |
| 404 | $CH_2$ | 1 | 2 | i-Pr | (4)-$NH_2$ | H | 2-MePr | 2-MePr |
| 405 | $CH_2$ | 1 | 3 | Me | (4)-$NH_2$ | H | n-Pen | n-Pen |
| 406 | $CH_2$ | 1 | 3 | Et | (4)-$NH_2$ | H | i-Pr | i-Pr |
| 407 | $CH_2$ | 1 | 4 | n-Pr | (4)-$NH_2$ | H | c-Pen | c-Pen |
| 408 | $CH_2$ | 1 | 3 | i-Pr | (4)-$NH_2$ | H | n-Hex | n-Hex |
| 409 | $CH_2$ | 2 | 2 | Me | (4)-$NH_2$ | H | c-Hex | c-Hex |
| 410 | $CH_2$ | 2 | 2 | Et | (4)-$NH_2$ | H | 1-MePr | 1-MePr |
| 411 | $CH_2$ | 2 | 2 | n-Pr | (4)-$NH_2$ | H | n-Bu | n-Bu |
| 412 | $CH_2$ | 2 | 2 | i-Pr | (4)-$NH_2$ | H | t-Bu | t-Bu |
| 413 | $CH_2$ | 2 | 4 | Me | (4)-$NH_2$ | H | c-Hex | c-Hex |
| 414 | $CH_2$ | 2 | 3 | Et | (4)-$NH_2$ | H | n-Pen | n-Pen |
| 415 | $CH_2$ | 2 | 3 | n-Pr | (4)-$NH_2$ | H | t-Bu | t-Bu |
| 416 | $CH_2$ | 2 | 3 | i-Pr | (4)-$NH_2$ | H | 2-MePr | 2-MePr |
| 417 | O | 2 | 2 | Me | (4)-$NH_2$ | H | c-Bu | c-Bu |
| 418 | O | 2 | 2 | Et | (4)-$NH_2$ | H | c-Pen | c-Pen |

Table 6 (Cont'd)

| Comp. No. | A | m | n | $R^3$ | $X^1$ | $X^2$ | $R^2$ | $R^1$ |
|---|---|---|---|---|---|---|---|---|
| 419 | O | 2 | 2 | n-Pr | (4)-$NH_2$ | H | n-Pen | n-Pen |
| 420 | O | 2 | 2 | i-Pr | (4)-$NH_2$ | H | n-Bu | Me |
| 421 | O | 2 | 3 | Me | (4)-$NH_2$ | H | Et | n-Hex |
| 422 | O | 2 | 4 | Et | (4)-$NH_2$ | H | n-Pr | t-Bu |
| 423 | O | 2 | 3 | n-Pr | (4)-$NH_2$ | H | Me | Et |
| 424 | O | 2 | 3 | i-Pr | (4)-$NH_2$ | H | Et | Me |
| 425 | O | 4 | 2 | Me | (4)-$NH_2$ | H | Me | t-Bu |
| 426 | O | 3 | 2 | Et | (4)-$NH_2$ | H | n-Bu | n-Hex |
| 427 | O | 3 | 2 | n-Pr | (4)-$NH_2$ | H | n-Hex | n-Bu |
| 428 | O | 3 | 2 | i-Pr | (4)-$NH_2$ | H | c-Pen | Me |
| 429 | O | 4 | 4 | Me | (4)-$NH_2$ | H | Me | c-Pen |
| 430 | O | 3 | 3 | Et | (4)-$NH_2$ | H | Me | n-Hex |
| 431 | O | 3 | 3 | n-Pr | (4)-$NH_2$ | H | n-Hex | Me |
| 432 | O | 4 | 4 | i-Pr | (4)-$NH_2$ | H | c-Hex | Et |
| 433 | $CH_2$ | 1 | 2 | H | (2)$CH_3SO_2NH$- | H | H | H |
| 434 | $CH_2$ | 1 | 2 | Me | (3)$CH_3SO_2NH$- | H | Me | Me |
| 435 | $CH_2$ | 1 | 2 | Et | (2)$CH_3SO_2NH$- | H | Et | Et |
| 436 | $CH_2$ | 1 | 4 | n-Pr | (3)$CH_3SO_2NH$- | H | n-Pr | n-Pr |
| 437 | $CH_2$ | 1 | 2 | i-Pr | (2)$CH_3SO_2NH$- | H | t-Bu | t-Bu |
| 438 | $CH_2$ | 1 | 2 | n-Bu | (3)$CH_3SO_2NH$- | H | c-Pen | c-Pen |
| 439 | $CH_2$ | 1 | 3 | H | (3)$CH_3SO_2NH$- | H | n-Hex | n-Hex |
| 440 | $CH_2$ | 1 | 4 | Me | (2)$CH_3SO_2NH$- | H | Me | Et |
| 441 | $CH_2$ | 1 | 3 | Et | (3)$CH_3SO_2NH$- | H | Et | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 442 | $CH_2$ | 1 | 3 | n-Pr | (3)$CH_3SO_2NH$- | H | Me | Me |
| 443 | $CH_2$ | 1 | 3 | i-Pr | (3)$CH_3SO_2NH$- | H | i-Pr | i-Pr |
| 444 | $CH_2$ | 1 | 4 | n-Bu | (2)$CH_3SO_2NH$- | H | 2-MePr | 2-MePr |
| 445 | $CH_2$ | 2 | 2 | H | (2)$CH_3SO_2NH$- | H | n-Pen | n-Pen |
| 446 | $CH_2$ | 2 | 2 | Me | (3)$CH_3SO_2NH$- | H | 4-MePen | 4-MePen |
| 447 | $CH_2$ | 2 | 2 | Et | (2)$CH_3SO_2NH$- | H | Me | n-Pr |
| 448 | $CH_2$ | 2 | 4 | n-Pr | (3)$CH_3SO_2NH$- | H | n-Pr | Me |
| 449 | $CH_2$ | 2 | 2 | i-Pr | (2)$CH_3SO_2NH$- | H | Et | Et |
| 450 | $CH_2$ | 2 | 2 | n-Bu | (3)$CH_3SO_2NH$- | H | n-Pr | n-Pr |
| 451 | $CH_2$ | 2 | 3 | H | (3)$CH_3SO_2NH$- | H | t-Bu | t-Bu |
| 452 | $CH_2$ | 2 | 3 | Me | (2)$CH_3SO_2NH$- | H | 1,1-diMePr | 1,1-diMePr |
| 453 | $CH_2$ | 2 | 4 | Et | (3)$CH_3SO_2NH$- | H | n-Hex | n-Hex |
| 454 | $CH_2$ | 2 | 3 | n-Pr | (2)$CH_3SO_2NH$- | H | Me | n-Bu |
| 455 | $CH_2$ | 2 | 3 | i-Pr | (3)$CH_3SO_2NH$- | H | n-Bu | Me |
| 456 | $CH_2$ | 2 | 4 | n-Bu | (2)$CH_3SO_2NH$- | H | Et | Et |
| 457 | O | 2 | 2 | H | (2)$CH_3SO_2NH$- | H | 1-MePr | 1-MePr |
| 458 | O | 2 | 2 | Me | (3)$CH_3SO_2NH$- | H | 2,2-diMePr | 2,2-diMePr |
| 459 | O | 2 | 2 | Et | (2)$CH_3SO_2NH$- | H | 1-MePen | 1-MePen |
| 460 | O | 2 | 2 | n-Pr | (3)$CH_3SO_2NH$- | H | Me | n-Pen |
| 461 | O | 2 | 2 | i-Pr | (2)$CH_3SO_2NH$- | H | n-Pen | Me |
| 462 | O | 2 | 2 | n-Bu | (3)$CH_3SO_2NH$- | H | Me | Me |
| 463 | O | 2 | 4 | H | (3)$CH_3SO_2NH$- | H | Et | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 464 | O | 2 | 3 | Me | (2)$CH_3SO_2NH$- | H | i-Pr | i-Pr |
| 465 | O | 2 | 4 | Et | (3)$CH_3SO_2NH$- | H | c-Bu | c-Bu |
| 466 | O | 2 | 3 | n-Pr | (2)$CH_3SO_2NH$- | H | n-Pen | n-Pen |
| 467 | O | 2 | 3 | i-Pr | (3)$CH_3SO_2NH$- | H | 2-MePen | 2-MePen |
| 468 | O | 2 | 3 | n-Bu | (2)$CH_3SO_2NH$- | H | Me | Me |
| 469 | O | 3 | 2 | H | (3)$CH_3SO_2NH$- | H | Et | Et |
| 470 | O | 3 | 2 | Me | (2)$CH_3SO_2NH$- | H | n-Pr | n-Pr |
| 471 | O | 3 | 2 | Et | (3)$CH_3SO_2NH$- | H | i-Pr | i-Pr |
| 472 | O | 3 | 2 | n-Pr | (2)$CH_3SO_2NH$- | H | 1-MePr | 1-MePr |
| 473 | O | 3 | 2 | i-Pr | (3)$CH_3SO_2NH$- | H | Me | Me |
| 474 | O | 3 | 2 | n-Bu | (2)$CH_3SO_2NH$- | H | Et | Et |
| 475 | O | 3 | 3 | H | (2)$CH_3SO_2NH$- | H | n-Pr | n-Pr |
| 476 | O | 3 | 4 | Me | (3)$CH_3SO_2NH$- | H | t-Bu | t-Bu |
| 477 | O | 3 | 3 | Et | (2)$CH_3SO_2NH$- | H | c-Pen | c-Pen |
| 478 | O | 4 | 3 | n-Pr | (3)$CH_3SO_2NH$- | H | n-Hex | n-Hex |
| 479 | O | 3 | 3 | i-Pr | (2)$CH_3SO_2NH$- | H | Me | Et |
| 480 | O | 4 | 3 | n-Bu | (3)$CH_3SO_2NH$- | H | Et | Me |
| 481 | $CH_2$ | 1 | 2 | H | (4)n-Pr$SO_2NH$- | H | Me | Me |
| 482 | $CH_2$ | 1 | 2 | Me | (4)n-Bu$SO_2NH$- | H | i-Pr | i-Pr |
| 483 | $CH_2$ | 1 | 2 | Et | (4)n-Pen$SO_2NH$- | H | 2-MePr | 2-MePr |
| 484 | $CH_2$ | 1 | 2 | n-Pr | (4)n-Hex$SO_2NH$- | H | n-Pen | n-Pen |
| 485 | $CH_2$ | 1 | 2 | i-Pr | (4)Et$SO_2NH$- | H | 4-MePen | 4-MePen |

Table 6 (Cont'd)

| Comp. No. | A | m | n | $R^3$ | $X^1$ | $X^2$ | $R^2$ | $R^1$ |
|---|---|---|---|---|---|---|---|---|
| 486 | $CH_2$ | 1 | 2 | n-Bu | (4)c-Hex$SO_2$NH- | H | Me | n-Pr |
| 487 | $CH_2$ | 1 | 4 | H | (4)c-Bu$SO_2$NH- | H | n-Pr | Me |
| 488 | $CH_2$ | 1 | 3 | Me | (4)c-Pr$SO_2$NH- | H | Et | Et |
| 489 | $CH_2$ | 1 | 3 | Et | (4)t-Bu$SO_2$NH- | H | n-Pr | n-Pr |
| 490 | $CH_2$ | 1 | 3 | n-Pr | (4)Et$SO_2$NH- | H | Me | Me |
| 491 | $CH_2$ | 1 | 4 | i-Pr | (4)(2-MePr)-$SO_2$NH- | H | Et | Et |
| 492 | $CH_2$ | 1 | 3 | n-Bu | (4)(1-MePr)-$SO_2$NH- | H | i-Pr | i-Pr |
| 493 | $CH_2$ | 2 | 2 | H | (4)i-Pr$SO_2$NH- | H | n-Bu | n-Bu |
| 494 | $CH_2$ | 2 | 2 | Me | (4)n-Pr$SO_2$NH- | H | 3-MeBu | 3-MeBu |
| 495 | $CH_2$ | 2 | 2 | Et | (4)Et$SO_2$NH- | H | 3-MePen | 3-MePen |
| 496 | $CH_2$ | 2 | 2 | n-Pr | (4)n-Pen$SO_2$NH- | H | Me | Me |
| 497 | $CH_2$ | 2 | 2 | i-Pr | (4)n-Hex$SO_2$NH- | H | Et | Et |
| 498 | $CH_2$ | 2 | 2 | n-Bu | (4)n-Bu$SO_2$NH- | H | i-Pr | i-Pr |
| 499 | $CH_2$ | 2 | 3 | H | (4)c-Hex$SO_2$NH- | H | 2-MePr | 2-MePr |
| 500 | $CH_2$ | 2 | 3 | Me | (4)Et$SO_2$NH- | H | Me | Me |
| 501 | $CH_2$ | 2 | 4 | Et | (4)c-Pen$SO_2$NH- | H | Et | Et |
| 502 | $CH_2$ | 2 | 3 | n-Pr | (4)i-Pr$SO_2$NH- | H | n-Pr | n-Pr |
| 503 | $CH_2$ | 2 | 3 | i-Pr | (4)Et$SO_2$NH- | H | t-Bu | t-Bu |
| 504 | $CH_2$ | 2 | 3 | n-Bu | (4)n-Bu$SO_2$NH- | H | c-Pen | c-Pen |
| 505 | O | 2 | 2 | H | (4)n-Hex$SO_2$NH- | H | n-Hex | n-Hex |
| 506 | O | 2 | 2 | Me | (4)c-Hex$SO_2$NH- | H | Me | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 507 | O | 2 | 2 | Et | (4)n-$BuSO_2NH$- | H | Et | Me |
| 508 | O | 2 | 2 | n-Pr | (4)$EtSO_2NH$- | H | Me | Me |
| 509 | O | 2 | 2 | i-Pr | (4)2-MePr-$SO_2NH$- | H | i-Pr | i-Pr |
| 510 | O | 2 | 2 | n-Bu | (4)i-$PrSO_2NH$- | H | 2-MePr | 2-MePr |
| 511 | O | 2 | 4 | H | (4)$EtSO_2NH$- | H | Me | Et |
| 512 | O | 2 | 3 | Me | (4)1-MePr-$SO_2NH$- | H | Et | Me |
| 513 | O | 2 | 3 | Et | (4)t-$BuSO_2NH$- | H | n-Pr | n-Pr |
| 514 | O | 2 | 3 | n-Pr | (4)n-$PenSO_2NH$- | H | t-Bu | t-Bu |
| 515 | O | 2 | 3 | i-Pr | (4)$EtSO_2NH$- | H | 3-MeBu | 3-MeBu |
| 516 | O | 2 | 4 | n-Bu | (4)c-$BuSO_2NH$- | H | n-Hex | n-Hex |
| 517 | O | 3 | 2 | H | (4)c-$PenSO_2NH$- | H | Me | Me |
| 518 | O | 4 | 2 | Me | (4)c-$HexSO_2NH$- | H | Et | Et |
| 519 | O | 3 | 2 | Et | (4)$EtSO_2NH$- | H | n-Pr | n-Pr |
| 520 | O | 3 | 2 | n-Pr | (4)t-$BuSO_2NH$- | H | t-Bu | t-Bu |
| 521 | O | 3 | 2 | i-Pr | (4)n-$HexSO_2NH$- | H | c-Pen | c-Pen |
| 522 | O | 3 | 2 | n-Bu | (4)i-$PrSO_2NH$- | H | n-Hex | n-Hex |
| 523 | O | 3 | 3 | H | (4)2-$MePrSO_2NH$- | H | Me | Et |
| 524 | O | 3 | 3 | Me | (4)n-$PenSO_2NH$- | H | Et | Me |
| 525 | O | 4 | 3 | Et | (4)$EtSO_2NH$- | H | Me | Me |
| 526 | O | 4 | 4 | n-Pr | (4)$EtSO_2NH$- | H | i-Pr | i-Pr |
| 527 | O | 3 | 3 | i-Pr | (4)$EtSO_2NH$- | H | 2-MePr | 2-MePr |
| 528 | O | 3 | 3 | n-Bu | (4)$EtSO_2NH$- | H | 1,1,2-triMePr | 1,1,2-triMePr |

Table 6 (Cont'd)

| Comp. No. | A | m | n | $R^3$ | $X^1$ | $X^2$ | $R^2$ | $R^1$ |
|---|---|---|---|---|---|---|---|---|
| 529 | $CH_2$ | 1 | 2 | H | (2)-$NO_2$ | H | Me | Et |
| 530 | $CH_2$ | 1 | 2 | Me | (3)-$NO_2$ | H | Et | Me |
| 531 | $CH_2$ | 1 | 2 | Et | (3)-$NO_2$ | H | n-Pr | n-Pr |
| 532 | $CH_2$ | 1 | 2 | n-Pr | (3)-$NO_2$ | H | c-Bu | c-Bu |
| 533 | $CH_2$ | 1 | 2 | i-Pr | (3)-$NO_2$ | H | 1-MeBu | 1-MeBu |
| 534 | $CH_2$ | 1 | 2 | n-Bu | (2)-$NO_2$ | H | 1-MePen | 1-MePen |
| 535 | $CH_2$ | 1 | 3 | H | (3)-$NO_2$ | H | Me | Et |
| 536 | $CH_2$ | 1 | 4 | Me | (3)-$NO_2$ | H | Et | Me |
| 537 | $CH_2$ | 1 | 3 | Et | (2)-$NO_2$ | H | i-Pr | i-Pr |
| 538 | $CH_2$ | 1 | 3 | n-Pr | (2)-$NO_2$ | H | Me | Me |
| 539 | $CH_2$ | 1 | 3 | n-Pr | (3)-$NO_2$ | H | Me | Me |
| 540 | $CH_2$ | 1 | 3 | i-Pr | (3)-$NO_2$ | H | n-Pr | n-Pr |
| 541 | $CH_2$ | 1 | 4 | n-Bu | (3)-$NO_2$ | H | t-Bu | t-Bu |
| 542 | $CH_2$ | 2 | 2 | H | (2)-$NO_2$ | H | 2-MeBu | 2-MeBu |
| 543 | $CH_2$ | 2 | 2 | Me | (2)-$NO_2$ | H | c-Hex | c-Hex |
| 544 | $CH_2$ | 2 | 2 | Et | (3)-$NO_2$ | H | Me | Me |
| 545 | $CH_2$ | 2 | 2 | n-Pr | (3)-$NO_2$ | H | Et | Et |
| 546 | $CH_2$ | 2 | 2 | i-Pr | (3)-$NO_2$ | H | n-Pr | n-Pr |
| 547 | $CH_2$ | 2 | 2 | n-Bu | (2)-$NO_2$ | H | t-Bu | t-Bu |
| 548 | $CH_2$ | 2 | 3 | H | (2)-$NO_2$ | H | c-Pen | c-Pen |
| 549 | $CH_2$ | 2 | 4 | Me | (3)-$NO_2$ | H | n-Hex | n-Hex |
| 550 | $CH_2$ | 2 | 3 | Et | (2)-$NO_2$ | H | Me | Et |
| 551 | $CH_2$ | 2 | 3 | n-Pr | (3)-$NO_2$ | H | Et | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 552 | $CH_2$ | 2 | 3 | i-Pr | (2)-$NO_2$ | H | n-Pr | n-Pr |
| 553 | $CH_2$ | 2 | 3 | n-Bu | (3)-$NO_2$ | H | i-Pr | i-Pr |
| 554 | O | 2 | 2 | H | (3)-$NO_2$ | H | n-Bu | n-Bu |
| 555 | O | 2 | 2 | Me | (3)-$NO_2$ | H | t-Bu | t-Bu |
| 556 | O | 2 | 2 | Et | (2)-$NO_2$ | H | c-Bu | c-Bu |
| 557 | O | 2 | 2 | n-Pr | (3)-$NO_2$ | H | 2-MePr | 2-MePr |
| 558 | O | 2 | 2 | i-Pr | (3)-$NO_2$ | H | 1-MePr | 1-MePr |
| 559 | O | 2 | 2 | n-Bu | (2)-$NO_2$ | H | n-Pen | n-Pen |
| 560 | O | 2 | 3 | H | (2)-$NO_2$ | H | c-Pen | c-Pen |
| 561 | O | 2 | 4 | Me | (3)-$NO_2$ | H | 3-MeBu | 3-MeBu |
| 562 | O | 2 | 3 | Et | (3)-$NO_2$ | H | 2-MeBu | 2-MeBu |
| 563 | O | 2 | 3 | n-Pr | (3)-$NO_2$ | H | 1-MeBu | 1-MeBu |
| 564 | O | 2 | 3 | i-Pr | (2)-$NO_2$ | H | 1,1-diMePr | 1,1-diMePr |
| 565 | O | 2 | 3 | n-Bu | (2)-$NO_2$ | H | 1,2-diMePr | 1,2-diMePr |
| 566 | O | 3 | 2 | H | (2)-$NO_2$ | H | 2,2-diMePr | 2,2-diMePr |
| 567 | O | 3 | 2 | Me | (2)-$NO_2$ | H | n-Hex | n-Hex |
| 568 | O | 3 | 2 | Et | (2)-$NO_2$ | H | c-Hex | c-Hex |
| 569 | O | 4 | 2 | n-Pr | (2)-$NO_2$ | H | 4-MePen | 4-MePen |
| 570 | O | 3 | 2 | i-Pr | (3)-$NO_2$ | H | 3-MePen | 3-MePen |
| 571 | O | 3 | 2 | n-Bu | (3)-$NO_2$ | H | 2-MePen | 2-MePen |
| 572 | O | 3 | 3 | H | (3)-$NO_2$ | H | 1-MePen | 1-MePen |
| 573 | O | 4 | 3 | Me | (2)-$NO_2$ | H | 1,2,2-triMePr | 1,2,2-triMePr |

Table 6 (Cont'd)

| Comp. No. | A | m | n | $R^3$ | $X^1$ | $X^2$ | $R^2$ | $R^1$ |
|---|---|---|---|---|---|---|---|---|
| 574 | O | 3 | 4 | Et | (3)-$NO_2$ | H | 1,1,2-triMePr | 1,1,2-triMePr |
| 575 | O | 3 | 3 | n-Pr | (3)-$NO_2$ | H | 1,1-diMeBu | 1,1-diMeBu |
| 576 | O | 3 | 3 | i-Pr | (2)-$NO_2$ | H | 2,2-diMeBu | 2,2-diMeBu |
| 577 | O | 3 | 3 | n-Bu | (2)-$NO_2$ | H | 1,3-diMeBu | 1,3-diMeBu |
| 578 | $CH_2$ | 1 | 2 | H | (4)-$NO_2$ | H | 2,3-diMeBu | 2,3-diMeBu |
| 579 | $CH_2$ | 1 | 2 | Me | (4)-$NO_2$ | H | Me | Me |
| 580 | $CH_2$ | 1 | 2 | Et | (4)-$NO_2$ | H | Me | Me |
| 581 | $CH_2$ | 1 | 2 | n-Pr | (4)-$NO_2$ | H | Me | Et |
| 582 | $CH_2$ | 1 | 2 | i-Pr | (4)-$NO_2$ | H | Et | Me |
| 583 | $CH_2$ | 1 | 2 | n-Bu | (4)-$NO_2$ | H | Et | Et |
| 584 | $CH_2$ | 1 | 3 | H | (4)-$NO_2$ | H | Me | Me |
| 585 | $CH_2$ | 1 | 3 | Me | (4)-$NO_2$ | H | Me | Me |
| 586 | $CH_2$ | 1 | 3 | Et | (4)-$NO_2$ | H | Me | Me |
| 587 | $CH_2$ | 1 | 3 | n-Pr | (4)-$NO_2$ | H | Me | Me |
| 588 | $CH_2$ | 1 | 3 | i-Pr | (4)-$NO_2$ | H | Me | Me |
| 589 | $CH_2$ | 1 | 3 | n-Bu | (4)-$NO_2$ | H | Me | Me |
| 590 | $CH_2$ | 2 | 2 | H | (4)-$NO_2$ | H | n-Pr | n-Pr |
| 591 | $CH_2$ | 2 | 2 | Me | (4)-$NO_2$ | H | 2-MePr | 2-MePr |
| 592 | $CH_2$ | 2 | 2 | Et | (4)-$NO_2$ | H | Me | Me |
| 593 | $CH_2$ | 2 | 2 | n-Pr | (4)-$NO_2$ | H | Et | Et |
| 594 | $CH_2$ | 2 | 2 | i-Pr | (4)-$NO_2$ | H | Me | i-Pr |
| 595 | $CH_2$ | 2 | 2 | n-Bu | (4)-$NO_2$ | H | i-Pr | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 596 | $CH_2$ | 2 | 4 | H | (4)-$NO_2$ | H | n-Bu | n-Bu |
| 597 | $CH_2$ | 2 | 3 | Me | (4)-$NO_2$ | H | n-Pen | n-Pen |
| 598 | $CH_2$ | 2 | 3 | Et | (4)-$NO_2$ | H | Me | Me |
| 599 | $CH_2$ | 2 | 3 | n-Pr | (4)-$NO_2$ | H | Me | Me |
| 600 | $CH_2$ | 2 | 3 | i-Pr | (4)-$NO_2$ | H | Et | Et |
| 601 | $CH_2$ | 2 | 4 | n-Bu | (4)-$NO_2$ | H | n-Bu | n-Bu |
| 602 | O | 2 | 2 | H | (4)-$NO_2$ | H | i-Pr | i-Pr |
| 603 | O | 2 | 2 | Me | (4)-$NO_2$ | H | Me | Me |
| 604 | O | 2 | 2 | Et | (4)-$NO_2$ | H | Me | Me |
| 605 | O | 2 | 2 | n-Pr | (4)-$NO_2$ | H | Me | Me |
| 606 | O | 2 | 2 | i-Pr | (4)-$NO_2$ | H | Me | Me |
| 607 | O | 2 | 2 | n-Bu | (4)-$NO_2$ | H | 3-MeBu | 3-MeBu |
| 608 | O | 2 | 3 | H | (4)-$NO_2$ | H | Me | Me |
| 609 | O | 2 | 3 | Me | (4)-$NO_2$ | H | Me | Me |
| 610 | O | 2 | 3 | Et | (4)-$NO_2$ | H | Me | Me |
| 611 | O | 2 | 3 | n-Pr | (4)-$NO_2$ | H | Me | Me |
| 612 | O | 2 | 3 | i-Pr | (4)-$NO_2$ | H | Me | Me |
| 613 | O | 2 | 3 | n-Bu | (4)-$NO_2$ | H | Et | Et |
| 614 | O | 3 | 2 | H | (4)-$NO_2$ | H | n-Pr | n-Pr |
| 615 | O | 4 | 2 | Me | (4)-$NO_2$ | H | 2-MePr | 2-MePr |
| 616 | O | 3 | 2 | Et | (4)-$NO_2$ | H | 1-MeBu | 1-MeBu |
| 617 | O | 3 | 2 | n-Pr | (4)-$NO_2$ | H | 2,3-diMeBu | 2,3-diMeBu |

Table 6 (Cont'd)

| Comp. No. | A | m | n | $R^3$ | $X^1$ | $X^2$ | $R^2$ | $R^1$ |
|---|---|---|---|---|---|---|---|---|
| 618 | O | 3 | 2 | i-Pr | (4)-$NO_2$ | H | Me | Me |
| 619 | O | 3 | 2 | n-Bu | (4)-$NO_2$ | H | Et | Et |
| 620 | O | 3 | 4 | H | (4)-$NO_2$ | H | n-Pr | n-Pr |
| 621 | O | 3 | 3 | Me | (4)-$NO_2$ | H | n-Bu | n-Bu |
| 622 | O | 3 | 3 | Et | (4)-$NO_2$ | H | Me | Me |
| 623 | O | 3 | 3 | n-Pr | (4)-$NO_2$ | H | Et | Et |
| 624 | O | 3 | 3 | i-Pr | (4)-$NO_2$ | H | Et | Et |
| 625 | O | 4 | 4 | n-Bu | (4)-$NO_2$ | H | Me | Me |
| 626 | $CH_2$ | 1 | 2 | H | (2)-$NH_2$ | H | n-Pr | n-Pr |
| 627 | $CH_2$ | 1 | 2 | Me | (3)-$NH_2$ | H | i-Pr | i-Pr |
| 628 | $CH_2$ | 1 | 2 | Et | (2)-$NH_2$ | H | c-Pr | c-Pr |
| 629 | $CH_2$ | 1 | 2 | n-Pr | (3)-$NH_2$ | H | n-Bu | n-Bu |
| 630 | $CH_2$ | 1 | 2 | i-Pr | (2)-$NH_2$ | H | t-Bu | t-Bu |
| 631 | $CH_2$ | 1 | 2 | n-Bu | (3)-$NH_2$ | H | c-Bu | c-Bu |
| 632 | $CH_2$ | 1 | 3 | H | (3)-$NH_2$ | H | 2-MePr | 2-MePr |
| 633 | $CH_2$ | 1 | 4 | Me | (3)-$NH_2$ | H | 1-MePr | 1-MePr |
| 634 | $CH_2$ | 1 | 3 | Et | (3)-$NH_2$ | H | n-Pr | n-Pr |
| 635 | $CH_2$ | 1 | 3 | n-Pr | (2)-$NH_2$ | H | Me | Me |
| 636 | $CH_2$ | 1 | 3 | n-Pr | (3)-$NH_2$ | H | Me | Me |
| 637 | $CH_2$ | 1 | 3 | i-Pr | (2)-$NH_2$ | H | n-Pr | n-Pr |
| 638 | $CH_2$ | 1 | 4 | n-Bu | (2)-$NH_2$ | H | t-Bu | t-Bu |
| 639 | $CH_2$ | 2 | 2 | H | (2)-$NH_2$ | H | 2-MeBu | 2-MeBu |

Table 6 (Cont'd)

| Comp. No. | A | m | n | $R^3$ | $X^1$ | $X^2$ | $R^2$ | $R^1$ |
|---|---|---|---|---|---|---|---|---|
| 640 | $CH_2$ | 2 | 2 | Me | (3)-$NH_2$ | H | c-Hex | c-Hex |
| 641 | $CH_2$ | 2 | 2 | Et | (2)-$NH_2$ | H | Me | Me |
| 642 | $CH_2$ | 2 | 2 | n-Pr | (3)-$NH_2$ | H | Et | Et |
| 643 | $CH_2$ | 2 | 2 | i-Pr | (2)-$NH_2$ | H | n-Pr | n-Pr |
| 644 | $CH_2$ | 2 | 2 | n-Bu | (3)-$NH_2$ | H | t-Bu | t-Bu |
| 645 | $CH_2$ | 2 | 3 | H | (2)-$NH_2$ | H | c-Pen | c-Pen |
| 646 | $CH_2$ | 2 | 3 | Me | (2)-$NH_2$ | H | n-Hex | n-Hex |
| 647 | $CH_2$ | 2 | 3 | Et | (3)-$NH_2$ | H | Me | Et |
| 648 | $CH_2$ | 2 | 3 | n-Pr | (2)-$NH_2$ | H | Et | Me |
| 649 | $CH_2$ | 2 | 4 | i-Pr | (3)-$NH_2$ | H | i-Pr | i-Pr |
| 650 | $CH_2$ | 2 | 3 | n-Bu | (2)-$NH_2$ | H | n-Bu | n-Bu |
| 651 | O | 2 | 2 | H | (3)-$NH_2$ | H | n-Pen | n-Pen |
| 652 | O | 2 | 2 | Me | (2)-$NH_2$ | H | c-Hex | c-Hex |
| 653 | O | 2 | 2 | Et | (2)-$NH_2$ | H | Me | Me |
| 654 | O | 2 | 2 | n-Pr | (3)-$NH_2$ | H | Et | Et |
| 655 | O | 2 | 2 | i-Pr | (3)-$NH_2$ | H | n-Pr | n-Pr |
| 656 | O | 2 | 2 | n-Bu | (3)-$NH_2$ | H | n-Bu | n-Bu |
| 657 | O | 2 | 4 | H | (2)-$NH_2$ | H | H | H |
| 658 | O | 2 | 3 | Me | (2)-$NH_2$ | H | Me | Me |
| 659 | O | 2 | 3 | Et | (2)-$NH_2$ | H | Et | Et |
| 660 | O | 2 | 3 | n-Pr | (3)-$NH_2$ | H | n-Pr | n-Pr |
| 661 | O | 2 | 3 | i-Pr | (2)-$NH_2$ | H | t-Bu | t-Bu |
| 662 | O | 2 | 3 | n-Bu | (3)-$NH_2$ | H | c-Pen | c-Pen |

Table 6 (Cont'd)

| Comp. No. | A | m | n | $R^3$ | $X^1$ | $X^2$ | $R^2$ | $R^1$ |
|---|---|---|---|---|---|---|---|---|
| 663 | O | 3 | 2 | H | (2)-$NH_2$ | H | n-Hex | n-Hex |
| 664 | O | 3 | 2 | Me | (2)-$NH_2$ | H | Me | Et |
| 665 | O | 4 | 2 | Et | (2)-$NH_2$ | H | Et | Me |
| 666 | O | 3 | 2 | n-Pr | (3)-$NH_2$ | H | Me | Me |
| 667 | O | 3 | 2 | i-Pr | (2)-$NH_2$ | H | i-Pr | i-Pr |
| 668 | O | 3 | 2 | n-Bu | (3)-$NH_2$ | H | 2-MePr | 2-MePr |
| 669 | O | 3 | 3 | H | (3)-$NH_2$ | H | n-Pen | n-Pen |
| 670 | O | 3 | 3 | Me | (2)-$NH_2$ | H | 4-MePen | 4-MePen |
| 671 | O | 3 | 3 | Et | (3)-$NH_2$ | H | Me | n-Pr |
| 672 | O | 3 | 3 | n-Pr | (3)-$NH_2$ | H | n-Pr | Me |
| 673 | O | 4 | 3 | i-Pr | (2)-$NH_2$ | H | Et | Et |
| 674 | O | 3 | 3 | n-Bu | (3)-$NH_2$ | H | n-Pr | n-Pr |
| 675 | $CH_2$ | 1 | 2 | H | (4)-$NH_2$ | H | t-Bu | t-Bu |
| 676 | $CH_2$ | 1 | 2 | Me | (4)-$NH_2$ | H | n-Bu | n-Bu |
| 677 | $CH_2$ | 1 | 2 | Et | (4)-$NH_2$ | H | Me | Me |
| 678 | $CH_2$ | 1 | 2 | n-Pr | (4)-$NH_2$ | H | Me | t-Bu |
| 679 | $CH_2$ | 1 | 2 | i-Pr | (4)-$NH_2$ | H | t-Bu | Me |
| 680 | $CH_2$ | 1 | 2 | n-Bu | (4)-$NH_2$ | H | Et | Et |
| 681 | $CH_2$ | 1 | 3 | H | (4)-$NH_2$ | H | Me | Me |
| 682 | $CH_2$ | 1 | 3 | Me | (4)-$NH_2$ | H | Me | Me |
| 683 | $CH_2$ | 1 | 3 | Et | (4)-$NH_2$ | H | Me | Me |
| 684 | $CH_2$ | 1 | 3 | n-Pr | (4)-$NH_2$ | H | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 685 | $CH_2$ | 1 | 3 | i-Pr | (4)-$NH_2$ | H | Me | Me |
| 686 | $CH_2$ | 1 | 3 | n-Bu | (4)-$NH_2$ | H | Me | Me |
| 687 | $CH_2$ | 2 | 2 | H | (4)-$NH_2$ | H | Me | 2-MePr |
| 688 | $CH_2$ | 2 | 2 | Me | (4)-$NH_2$ | H | 2-MePr | Me |
| 689 | $CH_2$ | 2 | 2 | Et | (4)-$NH_2$ | H | Me | Me |
| 690 | $CH_2$ | 2 | 2 | n-Pr | (4)-$NH_2$ | H | Et | Et |
| 691 | $CH_2$ | 2 | 2 | i-Pr | (4)-$NH_2$ | H | n-Pr | n-Pr |
| 692 | $CH_2$ | 2 | 2 | n-Bu | (4)-$NH_2$ | H | i-Pr | i-Pr |
| 693 | $CH_2$ | 2 | 4 | H | (4)-$NH_2$ | H | c-Pr | c-Pr |
| 694 | $CH_2$ | 2 | 3 | Me | (4)-$NH_2$ | H | n-Bu | n-Bu |
| 695 | $CH_2$ | 2 | 3 | Et | (4)-$NH_2$ | H | Me | Me |
| 696 | $CH_2$ | 2 | 3 | n-Pr | (4)-$NH_2$ | H | Me | Me |
| 697 | $CH_2$ | 2 | 3 | i-Pr | (4)-$NH_2$ | H | Me | 1-MePr |
| 698 | $CH_2$ | 2 | 4 | n-Bu | (4)-$NH_2$ | H | 1-MePr | Me |
| 699 | O | 2 | 2 | H | (4)-$NH_2$ | H | n-Bu | n-Bu |
| 700 | O | 2 | 2 | Me | (4)-$NH_2$ | H | Me | Me |
| 701 | O | 2 | 2 | Et | (4)-$NH_2$ | H | Me | Me |
| 702 | O | 2 | 2 | n-Pr | (4)-$NH_2$ | H | Me | Me |
| 703 | O | 2 | 2 | i-Pr | (4)-$NH_2$ | H | Me | Me |
| 704 | O | 2 | 2 | n-Bu | (4)-$NH_2$ | H | Et | Et |
| 705 | O | 2 | 3 | H | (4)-$NH_2$ | H | Me | Me |
| 706 | O | 2 | 3 | Me | (4)-$NH_2$ | H | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 707 | O | 2 | 3 | Et | (4)-$NH_2$ | H | Me | Me |
| 708 | O | 2 | 3 | n-Pr | (4)-$NH_2$ | H | Me | Me |
| 709 | O | 2 | 3 | i-Pr | (4)-$NH_2$ | H | Me | Me |
| 710 | O | 2 | 4 | n-Bu | (4)-$NH_2$ | H | Et | Et |
| 711 | O | 3 | 2 | H | (4)-$NH_2$ | H | n-Pr | n-Pr |
| 712 | O | 4 | 2 | Me | (4)-$NH_2$ | H | i-Pr | i-Pr |
| 713 | O | 3 | 2 | Et | (4)-$NH_2$ | H | n-Bu | n-Bu |
| 714 | O | 3 | 2 | n-Pr | (4)-$NH_2$ | H | t-Bu | t-Bu |
| 715 | O | 4 | 2 | i-Pr | (4)-$NH_2$ | H | 2-MePr | 2-MePr |
| 716 | O | 3 | 2 | n-Bu | (4)-$NH_2$ | H | 1-MePr | 1-MePr |
| 717 | O | 3 | 3 | H | (4)-$NH_2$ | H | n-Pen | n-Pen |
| 718 | O | 4 | 4 | Me | (4)-$NH_2$ | H | c-Pen | c-Pen |
| 719 | O | 3 | 3 | Et | (4)-$NH_2$ | H | Me | Me |
| 720 | O | 3 | 3 | n-Pr | (4)-$NH_2$ | H | Et | Et |
| 721 | O | 3 | 3 | i-Pr | (4)-$NH_2$ | H | n-Pr | n-Pr |
| 722 | O | 3 | 3 | n-Bu | (4)-$NH_2$ | H | 3-MeBu | 3-MeBu |
| 723 | $CH_2$ | 1 | 2 | Me | H | H | n-Hex | n-Hex |
| 724 | $CH_2$ | 1 | 2 | Me | (2) F | H | Me | Me |
| 725 | $CH_2$ | 1 | 2 | Me | (3) F | H | Et | Et |
| 726 | $CH_2$ | 1 | 2 | Me | (4) F | H | n-Pr | n-Pr |
| 727 | $CH_2$ | 1 | 2 | Me | (2) Cl | H | i-Pr | i-Pr |
| 728 | $CH_2$ | 1 | 2 | Me | (3) Cl | H | n-Bu | n-Bu |
| 729 | $CH_2$ | 1 | 2 | Me | (4) Cl | H | t-Bu | t-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 730 | $CH_2$ | 1 | 2 | Me | (4)-CN | H | Me | Et |
| 731 | $CH_2$ | 1 | 2 | Me | (2) Ac | H | Et | Me |
| 732 | $CH_2$ | 1 | 2 | Me | (3)EtCO- | H | Me | Me |
| 733 | $CH_2$ | 1 | 2 | Me | (4)n-PrCO- | H | i-Pr | i-Pr |
| 734 | $CH_2$ | 1 | 2 | Me | (4)PhCO- | H | 2-MePr | 2-MePr |
| 735 | $CH_2$ | 1 | 2 | Me | (2)MeO- | H | n-Pen | n-Pen |
| 736 | $CH_2$ | 1 | 2 | Me | (3)EtO- | H | 4-MePen | 4-MePen |
| 737 | $CH_2$ | 1 | 2 | Me | (4)MeS- | H | Me | n-Pr |
| 738 | $CH_2$ | 1 | 2 | Me | (4)-$CF_3$ | H | n-Pr | Me |
| 739 | $CH_2$ | 1 | 2 | Me | (4)$CF_3O$- | H | Et | Et |
| 740 | $CH_2$ | 1 | 2 | Me | (4)1-imidazol | H | n-Pr | n-Pr |
| 741 | $CH_2$ | 1 | 2 | Me | (4)EtCONH- | H | t-Bu | t-Bu |
| 742 | $CH_2$ | 1 | 2 | Me | (2) Ac | (4) Cl | n-Bu | n-Bu |
| 743 | $CH_2$ | 1 | 2 | Me | (2) Ac | (4) F | Me | Et |
| 744 | $CH_2$ | 1 | 2 | Me | (2) Cl | (4) Ac | Et | Me |
| 745 | $CH_2$ | 1 | 2 | Me | (2) F | (4) Ac | Me | Me |
| 746 | $CH_2$ | 1 | 2 | Et | H | H | i-Pr | i-Pr |
| 747 | $CH_2$ | 1 | 2 | Et | (2) F | H | 2-MePr | 2-MePr |
| 748 | $CH_2$ | 1 | 2 | Et | (3) F | H | 3-MeBu | 3-MeBu |
| 749 | $CH_2$ | 1 | 2 | Et | (4) F | H | 3-MePen | 3-MePen |
| 750 | $CH_2$ | 1 | 2 | Et | (2) Cl | H | Me | Me |
| 751 | $CH_2$ | 1 | 2 | Et | (3) Cl | H | Et | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 752 | $CH_2$ | 1 | 2 | Et | (4) Cl | H | n-Pr | n-Pr |
| 753 | $CH_2$ | 1 | 2 | Et | (4)-CN | H | c-Bu | c-Bu |
| 754 | $CH_2$ | 1 | 2 | Et | (2) Ac | H | 1-MeBu | 1-MeBu |
| 755 | $CH_2$ | 1 | 2 | Et | (3) Ac | H | 1,1-diMeBu | 1,1-diMeBu |
| 756 | $CH_2$ | 1 | 2 | Et | (4)PenCO- | H | Me | Et |
| 757 | $CH_2$ | 1 | 2 | Et | (4)PhCO- | H | Et | Me |
| 758 | $CH_2$ | 1 | 2 | Et | (2)n-PrO- | H | n-Pr | n-Pr |
| 759 | $CH_2$ | 1 | 2 | Et | (3)MeS- | H | i-Pr | i-Pr |
| 760 | $CH_2$ | 1 | 2 | Et | (4)MeO- | H | n-Bu | n-Bu |
| 761 | $CH_2$ | 1 | 2 | Et | (4)-$CF_3$ | H | t-Bu | t-Bu |
| 762 | $CH_2$ | 1 | 2 | Et | (4)$CF_3O$- | H | 2-MeBu | 2-MeBu |
| 763 | $CH_2$ | 1 | 2 | Et | (4)1-imidazol | H | 1-MePen | 1-MePen |
| 764 | $CH_2$ | 1 | 2 | Et | (3)PrCONH- | H | 2,3-diMeBu | 2,3-diMeBu |
| 765 | $CH_2$ | 1 | 2 | Et | (2) Ac | (4) Cl | Me | Et |
| 766 | $CH_2$ | 1 | 2 | Et | (2)i-PrCO- | (4) F | Et | Me |
| 767 | $CH_2$ | 1 | 2 | Et | (2) Cl | (4) Ac | i-Pr | i-Pr |
| 768 | $CH_2$ | 1 | 2 | Et | (2) F | (4) Ac | 1-MePr | 1-MePr |
| 769 | $CH_2$ | 1 | 2 | n-Pr | H | H | H | H |
| 770 | $CH_2$ | 1 | 2 | n-Pr | (2) F | H | Me | Me |
| 771 | $CH_2$ | 1 | 2 | n-Pr | (3) F | H | Et | Et |
| 772 | $CH_2$ | 1 | 2 | n-Pr | (4) F | H | n-Pr | n-Pr |
| 773 | $CH_2$ | 1 | 2 | n-Pr | (2) Cl | H | t-Bu | t-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 774 | $CH_2$ | 1 | 2 | n-Pr | (3) Cl | H | c-Pen | c-Pen |
| 775 | $CH_2$ | 1 | 2 | n-Pr | (4) Cl | H | n-Hex | n-Hex |
| 776 | $CH_2$ | 1 | 2 | n-Pr | (4)-CN | H | Me | Et |
| 777 | $CH_2$ | 1 | 2 | n-Pr | (2)2-MePrCO- | H | Et | Me |
| 778 | $CH_2$ | 1 | 2 | n-Pr | (3) Ac | H | Me | Me |
| 779 | $CH_2$ | 1 | 2 | n-Pr | (4) Ac | H | i-Pr | i-Pr |
| 780 | $CH_2$ | 1 | 2 | n-Pr | (4)PhCO- | H | 2-MePr | 2-MePr |
| 781 | $CH_2$ | 1 | 2 | n-Pr | (2)MeS- | H | n-Pen | n-Pen |
| 782 | $CH_2$ | 1 | 2 | n-Pr | (3)MeO- | H | 4-MePen | 4-MePen |
| 783 | $CH_2$ | 1 | 2 | n-Pr | (4)i-PrO- | H | Me | n-Pr |
| 784 | $CH_2$ | 1 | 2 | n-Pr | (4)-$CF_3$ | H | n-Pr | Me |
| 785 | $CH_2$ | 1 | 2 | n-Pr | (4)-$OCF_3$ | H | Et | Et |
| 786 | $CH_2$ | 1 | 2 | n-Pr | (4)1-imidazol | H | n-Pr | n-Pr |
| 787 | $CH_2$ | 1 | 2 | n-Pr | (4)n-BuCONH- | H | t-Bu | t-Bu |
| 788 | $CH_2$ | 1 | 2 | n-Pr | (2)t-BuCO- | (4) Cl | 1-MeBu | 1-MeBu |
| 789 | $CH_2$ | 1 | 2 | n-Pr | (2) Ac | (4) F | n-Hex | n-Hex |
| 790 | $CH_2$ | 1 | 2 | n-Pr | (2) Cl | (4) Ac | Me | Me |
| 791 | $CH_2$ | 1 | 2 | n-Pr | (2) F | (4)EtCO- | Et | Et |
| 792 | $CH_2$ | 1 | 2 | i-Pr | H | H | Me | Et |
| 793 | $CH_2$ | 1 | 2 | i-Pr | (2) F | H | Et | Me |
| 794 | $CH_2$ | 1 | 2 | i-Pr | (3) F | H | Me | Me |
| 795 | $CH_2$ | 1 | 2 | i-Pr | (4) F | H | i-Pr | i-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 796 | $CH_2$ | 1 | 2 | i-Pr | (2) Cl | H | 2-MePr | 2-MePr |
| 797 | $CH_2$ | 1 | 2 | i-Pr | (3) Cl | H | n-Pen | n-Pen |
| 798 | $CH_2$ | 1 | 2 | i-Pr | (4) Cl | H | 4-MePen | 4-MePen |
| 799 | $CH_2$ | 1 | 2 | i-Pr | (4)-CN | H | Me | n-Pr |
| 800 | $CH_2$ | 1 | 2 | i-Pr | (2) Ac | H | n-Pr | Me |
| 801 | $CH_2$ | 1 | 2 | i-Pr | (3)PrCO- | H | Et | Et |
| 802 | $CH_2$ | 1 | 2 | i-Pr | (4) Ac | H | n-Pr | n-Pr |
| 803 | $CH_2$ | 1 | 2 | i-Pr | (4)PhCO- | H | t-Bu | t-Bu |
| 804 | $CH_2$ | 1 | 2 | i-Pr | (2)EtS- | H | n-Bu | n-Bu |
| 805 | $CH_2$ | 1 | 2 | i-Pr | (3)t-BuO- | H | Me | Et |
| 806 | $CH_2$ | 1 | 2 | i-Pr | (4)MeO- | H | Et | Me |
| 807 | $CH_2$ | 1 | 2 | i-Pr | (4)-$CF_3$ | H | Me | Me |
| 808 | $CH_2$ | 1 | 2 | i-Pr | (4)$CF_3O$- | H | i-Pr | i-Pr |
| 809 | $CH_2$ | 1 | 2 | i-Pr | (4)1-imidazol | H | 2-MePr | 2-MePr |
| 810 | $CH_2$ | 1 | 2 | i-Pr | (2)n-PenNH- | H | 3-MeBu | 3-MeBu |
| 811 | $CH_2$ | 1 | 2 | i-Pr | (2)-n-Bu | (4) Cl | 3-MePen | 3-MePen |
| 812 | $CH_2$ | 1 | 2 | i-Pr | (2) Ac | (4) F | Me | Me |
| 813 | $CH_2$ | 1 | 2 | i-Pr | (2) Cl | (4) Ac | Et | Et |
| 814 | $CH_2$ | 1 | 2 | i-Pr | (2) F | (4) Ac | n-Pr | n-Pr |
| 815 | $CH_2$ | 1 | 4 | Me | H | H | Me | Me |
| 816 | $CH_2$ | 1 | 3 | Me | (2) F | H | i-Pr | i-Pr |
| 817 | $CH_2$ | 1 | 3 | Me | (3) F | H | 2-MePr | 2-MePr |

Table 6 (Cont'd)

| Comp. No. | A | m | n | $R^3$ | $X^1$ | $X^2$ | $R^2$ | $R^1$ |
|---|---|---|---|---|---|---|---|---|
| 818 | $CH_2$ | 1 | 3 | Me | (4) F | H | n-Pen | n-Pen |
| 819 | $CH_2$ | 1 | 3 | Me | (2) Cl | H | 4-MePen | 4-MePen |
| 820 | $CH_2$ | 1 | 4 | Me | (3) Cl | H | Me | n-Pr |
| 821 | $CH_2$ | 1 | 3 | Me | (4) Cl | H | n-Pr | Me |
| 822 | $CH_2$ | 1 | 3 | Me | (4)-CN | H | Et | Et |
| 823 | $CH_2$ | 1 | 3 | Me | (2) Ac | H | n-Pr | n-Pr |
| 824 | $CH_2$ | 1 | 4 | Me | (3) Ac | H | t-Bu | t-Bu |
| 825 | $CH_2$ | 1 | 3 | Me | (4)PenCO- | H | 1-MeBu | 1-MeBu |
| 826 | $CH_2$ | 1 | 3 | Me | (4)PhCO- | H | n-Hex | n-Hex |
| 827 | $CH_2$ | 1 | 3 | Me | (2)2-MePrS- | H | Me | Me |
| 828 | $CH_2$ | 1 | 3 | Me | (3)MeO- | H | Et | Et |
| 829 | $CH_2$ | 1 | 3 | Me | (4)n-BuO- | H | n-Pr | n-Pr |
| 830 | $CH_2$ | 1 | 3 | Me | (4)-$CF_3$ | H | i-Pr | i-Pr |
| 831 | $CH_2$ | 1 | 4 | Me | (4)$CF_3O$- | H | n-Bu | n-Bu |
| 832 | $CH_2$ | 1 | 3 | Me | (4)1-imidazol | H | t-Bu | t-Bu |
| 833 | $CH_2$ | 1 | 3 | Me | (4)AcNH- | H | Me | Et |
| 834 | $CH_2$ | 1 | 3 | Me | (2)i-PrCO- | (4) Cl | Et | Me |
| 835 | $CH_2$ | 1 | 3 | Me | (2) Ac | (4) F | Me | Me |
| 836 | $CH_2$ | 1 | 3 | Me | (2) Cl | (4) Ac | i-Pr | i-Pr |
| 837 | $CH_2$ | 1 | 4 | Me | (2) F | (4) Ac | 2-MePr | 2-MePr |
| 838 | $CH_2$ | 1 | 3 | Et | H | H | 3-MeBu | 3-MeBu |
| 839 | $CH_2$ | 1 | 3 | Et | (2) F | H | 3-MePen | 3-MePen |

Table 6 (Cont'd)

| Comp. No. | A | m | n | $R^3$ | $X^1$ | $X^2$ | $R^2$ | $R^1$ |
|---|---|---|---|---|---|---|---|---|
| 840 | $CH_2$ | 1 | 3 | Et | (3) F | H | Me | Me |
| 841 | $CH_2$ | 1 | 3 | Et | (4) F | H | Et | Et |
| 842 | $CH_2$ | 1 | 3 | Et | (2) Cl | H | n-Pr | n-Pr |
| 843 | $CH_2$ | 1 | 3 | Et | (3) Cl | H | Me | Me |
| 844 | $CH_2$ | 1 | 3 | Et | (4) Cl | H | i-Pr | i-Pr |
| 845 | $CH_2$ | 1 | 4 | Et | (4)-CN | H | 2-MePr | 2-MePr |
| 846 | $CH_2$ | 1 | 3 | Et | (2) Ac | H | n-Pen | n-Pen |
| 847 | $CH_2$ | 1 | 3 | Et | (3)2-MePrCO- | H | 4-MePen | 4-MePen |
| 848 | $CH_2$ | 1 | 3 | Et | (4) Ac | H | Me | c-Pr |
| 849 | $CH_2$ | 1 | 3 | Et | (4)PhCO- | H | c-Pr | Me |
| 850 | $CH_2$ | 1 | 3 | Et | (2)n-PenO- | H | Me | Et |
| 851 | $CH_2$ | 1 | 3 | Et | (3)MeO- | H | Et | Me |
| 852 | $CH_2$ | 1 | 3 | Et | (4)n-HexO- | H | Me | Me |
| 853 | $CH_2$ | 1 | 3 | Et | (4)-$CF_3$ | H | i-Pr | i-Pr |
| 854 | $CH_2$ | 1 | 4 | Et | (4)$CF_3O$- | H | 2-MePr | 2-MePr |
| 855 | $CH_2$ | 1 | 3 | Et | (4)1-imidazol | H | n-Pen | n-Pen |
| 856 | $CH_2$ | 1 | 3 | Et | (3)i-PrCO- | H | 4-MePen | 4-MePen |
| 857 | $CH_2$ | 1 | 3 | Et | (2) Ac | (4) Cl | Me | n-Pr |
| 858 | $CH_2$ | 1 | 3 | Et | (2) Ac | (4) F | n-Pr | Me |
| 859 | $CH_2$ | 1 | 4 | Et | (2) Cl | (4)t-BuCO- | Et | Et |
| 860 | $CH_2$ | 1 | 3 | Et | (2) F | (4)EtCO- | n-Pr | n-Pr |
| 861 | $CH_2$ | 1 | 3 | n-Pr | H | H | t-Bu | t-Bu |
| 862 | $CH_2$ | 1 | 4 | n-Pr | (2) F | H | 1-MeBu | 1-MeBu |

Table 6 (Cont'd)

| Comp. No. | A | m | n | $R^3$ | $X^1$ | $X^2$ | $R^2$ | $R^1$ |
|---|---|---|---|---|---|---|---|---|
| 863 | $CH_2$ | 1 | 3 | n-Pr | (3) F | H | n-Hex | n-Hex |
| 864 | $CH_2$ | 1 | 3 | n-Pr | (4) F | H | Me | Me |
| 865 | $CH_2$ | 1 | 3 | n-Pr | (2) Cl | H | Et | Et |
| 866 | $CH_2$ | 1 | 3 | n-Pr | (3) Cl | H | Me | Et |
| 867 | $CH_2$ | 1 | 4 | n-Pr | (4) Cl | H | Et | Me |
| 868 | $CH_2$ | 1 | 3 | n-Pr | (4)-CN | H | n-Pr | n-Pr |
| 869 | $CH_2$ | 1 | 3 | n-Pr | (2) Ac | H | i-Pr | i-Pr |
| 870 | $CH_2$ | 1 | 3 | n-Pr | (3) Ac | H | n-Bu | n-Bu |
| 871 | $CH_2$ | 1 | 4 | n-Pr | (4)PrCO- | H | t-Bu | t-Bu |
| 872 | $CH_2$ | 1 | 3 | n-Pr | (4)PhCO- | H | 2-MeBu | 2-MeBu |
| 873 | $CH_2$ | 1 | 3 | n-Pr | (2)MeO- | H | 1-MePen | 1-MePen |
| 874 | $CH_2$ | 1 | 3 | n-Pr | (3)n-PrS- | H | 2,3-diMeBu | 2,3-diMeBu |
| 875 | $CH_2$ | 1 | 4 | n-Pr | (4)c-PrO- | H | Me | Et |
| 876 | $CH_2$ | 1 | 3 | n-Pr | (4)-$CF_3$ | H | Et | Me |
| 877 | $CH_2$ | 1 | 3 | n-Pr | (4)$CF_3O$- | H | i-Pr | i-Pr |
| 878 | $CH_2$ | 1 | 3 | n-Pr | (4)1-imidazol | H | 1-MePr | 1-MePr |
| 879 | $CH_2$ | 1 | 4 | n-Pr | (4)t-BuCONH- | H | H | H |
| 880 | $CH_2$ | 1 | 3 | n-Pr | (2) Ac | (4) Cl | Me | Me |
| 881 | $CH_2$ | 1 | 3 | n-Pr | (2) Ac | (4) F | i-Pr | i-Pr |
| 882 | $CH_2$ | 1 | 3 | n-Pr | (2) Cl | (4)BuCO- | 2-MePr | 2-MePr |
| 883 | $CH_2$ | 1 | 3 | n-Pr | (2) F | (4) Ac | n-Pen | n-Pen |
| 884 | $CH_2$ | 1 | 3 | i-Pr | H | H | 4-MePen | 4-MePen |
| 885 | $CH_2$ | 1 | 3 | i-Pr | (2) F | H | Me | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 886 | $CH_2$ | 1 | 3 | i-Pr | (3) F | H | n-Pr | Me |
| 887 | $CH_2$ | 1 | 3 | i-Pr | (4) F | H | Et | Et |
| 888 | $CH_2$ | 1 | 4 | i-Pr | (2) Cl | H | n-Pr | n-Pr |
| 889 | $CH_2$ | 1 | 3 | i-Pr | (3) Cl | H | t-Bu | t-Bu |
| 890 | $CH_2$ | 1 | 3 | i-Pr | (4) Cl | H | n-Bu | n-Bu |
| 891 | $CH_2$ | 1 | 3 | i-Pr | (4)-CN | H | Me | Et |
| 892 | $CH_2$ | 1 | 4 | i-Pr | (2)n-PenCO- | H | Et | Me |
| 893 | $CH_2$ | 1 | 3 | i-Pr | (3) Ac | H | Me | Me |
| 894 | $CH_2$ | 1 | 3 | i-Pr | (4)EtCO- | H | i-Pr | i-Pr |
| 895 | $CH_2$ | 1 | 4 | i-Pr | (4)PhCO- | H | 2-MePr | 2-MePr |
| 896 | $CH_2$ | 1 | 3 | i-Pr | (2)i-PrS- | H | 3-MeBu | 3-MeBu |
| 897 | $CH_2$ | 1 | 3 | i-Pr | (3)MeO- | H | 3-MePen | 3-MePen |
| 898 | $CH_2$ | 1 | 3 | i-Pr | (4)c-HexO- | H | Me | Me |
| 899 | $CH_2$ | 1 | 3 | i-Pr | (4)-$CF_3$ | H | Et | Et |
| 900 | $CH_2$ | 1 | 3 | i-Pr | (4)$CF_3O$- | H | Me | Et |
| 901 | $CH_2$ | 1 | 3 | i-Pr | (4)1-imidazol | H | Et | Me |
| 902 | $CH_2$ | 1 | 4 | i-Pr | (4)AcNH- | H | n-Pr | n-Pr |
| 903 | $CH_2$ | 1 | 3 | i-Pr | (2) Ac | (4) Cl | i-Pr | i-Pr |
| 904 | $CH_2$ | 1 | 3 | i-Pr | (2)i-PrCO- | (4) F | n-Bu | n-Bu |
| 905 | $CH_2$ | 1 | 3 | i-Pr | (2) Cl | (4)n-PenCO- | t-Bu | t-Bu |
| 906 | $CH_2$ | 1 | 3 | i-Pr | (2) F | (4) Ac | 2-MeBu | 2-MeBu |
| 907 | $CH_2$ | 2 | 2 | Me | H | H | 1-MePen | 1-MePen |

Table 6 (Cont'd)

| Comp. No. | A | m | n | $R^3$ | $X^1$ | $X^2$ | $R^2$ | $R^1$ |
|---|---|---|---|---|---|---|---|---|
| 908 | $CH_2$ | 2 | 2 | Me | (2) F | H | 2,3-diMeBu | 2,3-diMeBu |
| 909 | $CH_2$ | 2 | 2 | Me | (3) F | H | Me | Et |
| 910 | $CH_2$ | 2 | 2 | Me | (4) F | H | Et | Me |
| 911 | $CH_2$ | 2 | 2 | Me | (2) Cl | H | i-Pr | i-Pr |
| 912 | $CH_2$ | 2 | 2 | Me | (3) Cl | H | 1-MePr | 1-MePr |
| 913 | $CH_2$ | 2 | 2 | Me | (4) Cl | H | H | H |
| 914 | $CH_2$ | 2 | 2 | Me | (4)-CN | H | Me | Me |
| 915 | $CH_2$ | 2 | 2 | Me | (2) Ac | H | i-Pr | i-Pr |
| 916 | $CH_2$ | 2 | 2 | Me | (3)2-MePrCO- | H | 2-MePr | 2-MePr |
| 917 | $CH_2$ | 2 | 2 | Me | (4) Ac | H | n-Pen | n-Pen |
| 918 | $CH_2$ | 2 | 2 | Me | (4)PhCO- | H | 4-MePen | 4-MePen |
| 919 | $CH_2$ | 2 | 2 | Me | (2)MeO- | H | Me | n-Pr |
| 920 | $CH_2$ | 2 | 2 | Me | (1)MePrO- | H | Me | Me |
| 921 | $CH_2$ | 2 | 2 | Me | (4)n-PenS- | H | Et | Et |
| 922 | $CH_2$ | 2 | 2 | Me | (4)-$CF_3$ | H | n-Pr | n-Pr |
| 923 | $CH_2$ | 2 | 2 | Me | (4)$CF_3O$- | H | i-Pr | i-Pr |
| 924 | $CH_2$ | 2 | 2 | Me | (4)1-imidazol | H | 2-MePr | 2-MePr |
| 925 | $CH_2$ | 2 | 2 | Me | (2)2-MePrCONH- | H | 1-MePr | 1-MePr |
| 926 | $CH_2$ | 2 | 2 | Me | (2) Ac | (4) Cl | Me | Me |
| 927 | $CH_2$ | 2 | 2 | Me | (2) Ac | (4) F | Et | Et |
| 928 | $CH_2$ | 2 | 2 | Me | (2) Cl | (4) Ac | i-Pr | i-Pr |
| 929 | $CH_2$ | 2 | 2 | Me | (2) F | (4) Ac | c-Pr | c-Pr |
| 930 | $CH_2$ | 2 | 2 | Et | H | H | n-Bu | n-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 931 | $CH_2$ | 2 | 2 | Et | (2) F | H | t-Bu | t-Bu |
| 932 | $CH_2$ | 2 | 2 | Et | (3) F | H | Me | Me |
| 933 | $CH_2$ | 2 | 2 | Et | (4) F | H | n-Pen | n-Pen |
| 934 | $CH_2$ | 2 | 2 | Et | (2) Cl | H | n-Pr | n-Pr |
| 935 | $CH_2$ | 2 | 2 | Et | (3) Cl | H | Et | Et |
| 936 | $CH_2$ | 2 | 2 | Et | (4) Cl | H | Me | Me |
| 937 | $CH_2$ | 2 | 2 | Et | (4)-CN | H | Me | n-Pr |
| 938 | $CH_2$ | 2 | 2 | Et | (2) Ac | H | n-Pr | Me |
| 939 | $CH_2$ | 2 | 2 | Et | (3)EtCO- | H | Et | Et |
| 940 | $CH_2$ | 2 | 2 | Et | (4) Ac | H | n-Pr | n-Pr |
| 941 | $CH_2$ | 2 | 2 | Et | (4)PhCO- | H | t-Bu | t-Bu |
| 942 | $CH_2$ | 2 | 2 | Et | (2)MeO- | H | 1-MeBu | 1-MeBu |
| 943 | $CH_2$ | 2 | 2 | Et | (3)c-HexS- | H | n-Hex | n-Hex |
| 944 | $CH_2$ | 2 | 2 | Et | (4)c-BuO- | H | Me | Me |
| 945 | $CH_2$ | 2 | 2 | Et | (4)-$CF_3$ | H | Et | Et |
| 946 | $CH_2$ | 2 | 2 | Et | (4)$CF_3O$- | H | n-Pr | n-Pr |
| 947 | $CH_2$ | 2 | 2 | Et | (4)1-imidazol | H | i-Pr | i-Pr |
| 948 | $CH_2$ | 2 | 2 | Et | (3)EtCONH- | H | n-Bu | n-Bu |
| 949 | $CH_2$ | 2 | 2 | Et | (2)n-PrCO- | (4) Cl | t-Bu | t-Bu |
| 950 | $CH_2$ | 2 | 2 | Et | (2) Ac | (4) F | Me | Et |
| 951 | $CH_2$ | 2 | 2 | Et | (2) Cl | (4) Ac | Et | Me |
| 952 | $CH_2$ | 2 | 2 | Et | (2) F | (4) Ac | Me | Me |
| 953 | $CH_2$ | 2 | 2 | n-Pr | H | H | i-Pr | i-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 954 | $CH_2$ | 2 | 2 | n-Pr | (2) F | H | Me | Me |
| 955 | $CH_2$ | 2 | 2 | n-Pr | (3) F | H | Me | n-Pr |
| 956 | $CH_2$ | 2 | 2 | n-Pr | (4) F | H | n-Pr | Me |
| 957 | $CH_2$ | 2 | 2 | n-Pr | (2) Cl | H | Et | Et |
| 958 | $CH_2$ | 2 | 2 | n-Pr | (3) Cl | H | n-Pr | n-Pr |
| 959 | $CH_2$ | 2 | 2 | n-Pr | (4) Cl | H | t-Bu | t-Bu |
| 960 | $CH_2$ | 2 | 2 | n-Pr | (4)-CN | H | 1-MeBu | 1-MeBu |
| 961 | $CH_2$ | 2 | 2 | n-Pr | (2)n-PenCO- | H | n-Hex | n-Hex |
| 962 | $CH_2$ | 2 | 2 | n-Pr | (3) Ac | H | Me | Me |
| 963 | $CH_2$ | 2 | 2 | n-Pr | (4) Ac | H | Et | Et |
| 964 | $CH_2$ | 2 | 2 | n-Pr | (4)PhCO- | H | n-Pr | n-Pr |
| 965 | $CH_2$ | 2 | 2 | n-Pr | (2)MeO- | H | i-Pr | i-Pr |
| 966 | $CH_2$ | 2 | 2 | n-Pr | (3)MeO- | H | n-Bu | n-Bu |
| 967 | $CH_2$ | 2 | 2 | n-Pr | (4)c-PenS- | H | Me | Me |
| 968 | $CH_2$ | 2 | 2 | n-Pr | (4)-$CF_3$ | H | Et | Et |
| 969 | $CH_2$ | 2 | 2 | n-Pr | (4)$CF_3O$- | H | n-Pr | n-Pr |
| 970 | $CH_2$ | 2 | 2 | n-Pr | (4)1-imidazol | H | t-Bu | t-Bu |
| 971 | $CH_2$ | 2 | 2 | n-Pr | (3)n-PrCONH- | H | Me | Me |
| 972 | $CH_2$ | 2 | 2 | n-Pr | (2)EtCO- | (4) Cl | n-Hex | n-Hex |
| 973 | $CH_2$ | 2 | 2 | n-Pr | (2) Ac | (4) F | Me | Me |
| 974 | $CH_2$ | 2 | 2 | n-Pr | (2) Cl | (4) Ac | Et | Et |
| 975 | $CH_2$ | 2 | 2 | n-Pr | (2) F | (4)n-PrCO- | n-Pr | n-Pr |
| 976 | $CH_2$ | 2 | 2 | i-Pr | H | H | i-Pr | i-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 977 | $CH_2$ | 2 | 2 | i-Pr | (2) F | H | n-Bu | n-Bu |
| 978 | $CH_2$ | 2 | 2 | i-Pr | (3) F | H | 3-MeBu | 3-MeBu |
| 979 | $CH_2$ | 2 | 2 | i-Pr | (4) F | H | 1-MePen | 1-MePen |
| 980 | $CH_2$ | 2 | 2 | i-Pr | (2) Cl | H | 1,2,2-triMePr | 1,2,2-triMePr |
| 981 | $CH_2$ | 2 | 2 | i-Pr | (3) Cl | H | 1,1,2-triMePr | 1,1,2-triMePr |
| 982 | $CH_2$ | 2 | 2 | i-Pr | (4) Cl | H | Me | Me |
| 983 | $CH_2$ | 2 | 2 | i-Pr | (4)-CN | H | Et | Et |
| 984 | $CH_2$ | 2 | 2 | i-Pr | (2)n-BuCO- | H | n-Pr | n-Pr |
| 985 | $CH_2$ | 2 | 2 | i-Pr | (3) Ac | H | i-Pr | i-Pr |
| 986 | $CH_2$ | 2 | 2 | i-Pr | (4) Ac | H | 2-MePr | 2-MePr |
| 987 | $CH_2$ | 2 | 2 | i-Pr | (4)PhCO- | H | 1-MePr | 1-MePr |
| 988 | $CH_2$ | 2 | 2 | i-Pr | (2)MeO- | H | Me | Me |
| 989 | $CH_2$ | 2 | 2 | i-Pr | (3)n-BuS- | H | Et | Et |
| 990 | $CH_2$ | 2 | 2 | i-Pr | (4)MeO- | H | i-Pr | i-Pr |
| 991 | $CH_2$ | 2 | 2 | i-Pr | (4)-$CF_3$ | H | c-Pr | c-Pr |
| 992 | $CH_2$ | 2 | 2 | i-Pr | (4)$CF_3O$- | H | n-Bu | n-Bu |
| 993 | $CH_2$ | 2 | 2 | i-Pr | (4)1-imidazol | H | t-Bu | t-Bu |
| 994 | $CH_2$ | 2 | 2 | i-Pr | (3)AcNH- | H | Me | Me |
| 995 | $CH_2$ | 2 | 2 | i-Pr | (2) Ac | (4) Cl | Et | Et |
| 996 | $CH_2$ | 2 | 2 | i-Pr | (2) Ac | (4) F | n-Pr | n-Pr |
| 997 | $CH_2$ | 2 | 2 | i-Pr | (2) Cl | (4)i-PrCO- | t-Bu | t-Bu |
| 998 | $CH_2$ | 2 | 2 | i-Pr | (2) F | (4) Ac | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 999 | $CH_2$ | 2 | 3 | Me | H | H | n-Hex | n-Hex |
| 1000 | $CH_2$ | 2 | 4 | Me | (2) F | H | Me | Me |
| 1001 | $CH_2$ | 2 | 3 | Me | (3) F | H | Et | Et |
| 1002 | $CH_2$ | 2 | 3 | Me | (4) F | H | n-Pr | n-Pr |
| 1003 | $CH_2$ | 2 | 3 | Me | (2) Cl | H | i-Pr | i-Pr |
| 1004 | $CH_2$ | 2 | 3 | Me | (3) Cl | H | n-Bu | n-Bu |
| 1005 | $CH_2$ | 2 | 3 | Me | (4) Cl | H | 3-MeBu | 3-MeBu |
| 1006 | $CH_2$ | 2 | 3 | Me | (4)-CN | H | 1-MePen | 1-MePen |
| 1007 | $CH_2$ | 2 | 4 | Me | (2) Ac | H | Me | Me |
| 1008 | $CH_2$ | 2 | 3 | Me | (3)n-Pr | H | Et | Et |
| 1009 | $CH_2$ | 2 | 3 | Me | (4) Ac | H | n-Pr | n-Pr |
| 1010 | $CH_2$ | 2 | 3 | Me | (4)PhCO- | H | t-Bu | t-Bu |
| 1011 | $CH_2$ | 2 | 3 | Me | (2)MeO- | H | Me | Me |
| 1012 | $CH_2$ | 2 | 4 | Me | (3)MeO- | H | n-Hex | n-Hex |
| 1013 | $CH_2$ | 2 | 3 | Me | (4)n-HexO- | H | Me | Me |
| 1014 | $CH_2$ | 2 | 3 | Me | (4)-$CF_3$ | H | Et | Et |
| 1015 | $CH_2$ | 2 | 4 | Me | (4)$CF_3O$- | H | n-Pr | n-Pr |
| 1016 | $CH_2$ | 2 | 3 | Me | (4)1-imidazol | H | i-Pr | i-Pr |
| 1017 | $CH_2$ | 2 | 3 | Me | (2)AcNH- | H | n-Bu | n-Bu |
| 1018 | $CH_2$ | 2 | 4 | Me | (2)i-PrCO- | (4) Cl | 3-MeBu | 3-MeBu |
| 1019 | $CH_2$ | 2 | 3 | Me | (2) Ac | (4) F | 1,1-diMePr | 1,1-diMePr |
| 1020 | $CH_2$ | 2 | 3 | Me | (2) Cl | (4) Ac | Me | c-Bu |
| 1021 | $CH_2$ | 2 | 3 | Me | (2) F | (4) Ac | c-Bu | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1022 | $CH_2$ | 2 | 3 | Et | H | H | Me | Me |
| 1023 | $CH_2$ | 2 | 3 | Et | (2) F | H | Et | Et |
| 1024 | $CH_2$ | 2 | 3 | Et | (3) F | H | Me | Me |
| 1025 | $CH_2$ | 2 | 3 | Et | (4) F | H | Me | 1-MePr |
| 1026 | $CH_2$ | 2 | 3 | Et | (2) Cl | H | 1-MePr | Me |
| 1027 | $CH_2$ | 2 | 4 | Et | (3) Cl | H | Me | Me |
| 1028 | $CH_2$ | 2 | 3 | Et | (4) Cl | H | Me | Me |
| 1029 | $CH_2$ | 2 | 3 | Et | (4)-CN | H | Me | Me |
| 1030 | $CH_2$ | 2 | 3 | Et | (2) Ac | H | Me | Me |
| 1031 | $CH_2$ | 2 | 4 | Et | (3) Ac | H | Et | Et |
| 1032 | $CH_2$ | 2 | 3 | Et | (4)n-PrCO- | H | n-Pr | n-Pr |
| 1033 | $CH_2$ | 2 | 3 | Et | (4)PhCO- | H | n-Bu | n-Bu |
| 1034 | $CH_2$ | 2 | 4 | Et | (2)t-BuO- | H | Et | Et |
| 1035 | $CH_2$ | 2 | 3 | Et | (3)MeO- | H | Me | Me |
| 1036 | $CH_2$ | 2 | 3 | Et | (4)MeO- | H | Me | Me |
| 1037 | $CH_2$ | 2 | 3 | Et | (4)-$CF_3$ | H | n-Pr | n-Pr |
| 1038 | $CH_2$ | 2 | 3 | Et | (4)$CF_3O$- | H | Me | Me |
| 1039 | $CH_2$ | 2 | 3 | Et | (4)1-imidazol | H | Et | Et |
| 1040 | $CH_2$ | 2 | 4 | Et | (4)n-PrCONH- | H | n-Pr | n-Pr |
| 1041 | $CH_2$ | 2 | 3 | Et | (2) Ac | (4) Cl | i-Pr | i-Pr |
| 1042 | $CH_2$ | 2 | 3 | Et | (2)EtCO- | (4) F | n-Bu | n-Bu |
| 1043 | $CH_2$ | 2 | 3 | Et | (2) Cl | (4) Ac | 3-MeBu | 3-MeBu |
| 1044 | $CH_2$ | 2 | 4 | Et | (2) F | (4) Ac | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1045 | $CH_2$ | 2 | 3 | n-Pr | H | H | Et | Et |
| 1046 | $CH_2$ | 2 | 3 | n-Pr | (2) F | H | n-Pr | n-Pr |
| 1047 | $CH_2$ | 2 | 3 | n-Pr | (3) F | H | t-Bu | t-Bu |
| 1048 | $CH_2$ | 2 | 4 | n-Pr | (4) F | H | Me | Me |
| 1049 | $CH_2$ | 2 | 3 | n-Pr | (2) Cl | H | n-Hex | n-Hex |
| 1050 | $CH_2$ | 2 | 3 | n-Pr | (3) Cl | H | Me | Me |
| 1051 | $CH_2$ | 2 | 3 | n-Pr | (4) Cl | H | Et | Et |
| 1052 | $CH_2$ | 2 | 4 | n-Pr | (4)-CN | H | n-Pr | n-Pr |
| 1053 | $CH_2$ | 2 | 3 | n-Pr | (2) Ac | H | i-Pr | i-Pr |
| 1054 | $CH_2$ | 2 | 3 | n-Pr | (3) Ac | H | n-Bu | n-Bu |
| 1055 | $CH_2$ | 2 | 3 | n-Pr | (4) Ac | H | 3-MeBu | 3-MeBu |
| 1056 | $CH_2$ | 2 | 4 | n-Pr | (4)PhCO- | H | 1-MePen | 1-MePen |
| 1057 | $CH_2$ | 2 | 3 | n-Pr | (2)MeO- | H | Me | Me |
| 1058 | $CH_2$ | 2 | 3 | n-Pr | (3)MeO- | H | Et | Et |
| 1059 | $CH_2$ | 2 | 3 | n-Pr | (4)c-PrS- | H | n-Pr | n-Pr |
| 1060 | $CH_2$ | 2 | 4 | n-Pr | (4)-$CF_3$ | H | t-Bu | t-Bu |
| 1061 | $CH_2$ | 2 | 3 | n-Pr | (4)-$CF_3O$ | H | Me | Me |
| 1062 | $CH_2$ | 2 | 3 | n-Pr | (4)1-imidazol | H | Et | Et |
| 1063 | $CH_2$ | 2 | 3 | n-Pr | (3)2-MePrCONH- | H | n-Pr | n-Pr |
| 1064 | $CH_2$ | 2 | 3 | n-Pr | (2)n-PrCO- | (4) Cl | i-Pr | i-Pr |
| 1065 | $CH_2$ | 2 | 3 | n-Pr | (2) Ac | (4) F | 2-MePr | 2-MePr |
| 1066 | $CH_2$ | 2 | 3 | n-Pr | (2) Cl | (4)n-PrCO- | 1-MePr | 1-MePr |
| 1067 | $CH_2$ | 2 | 3 | n-Pr | (2) F | (4) Ac | 1,1-diMePr | 1,1-diMePr |

Table 6 (Cont'd)

| Comp. No. | A | m | n | $R^3$ | $X^1$ | $X^2$ | $R^2$ | $R^1$ |
|---|---|---|---|---|---|---|---|---|
| 1068 | $CH_2$ | 2 | 4 | i-Pr | H | H | Me | c-Bu |
| 1069 | $CH_2$ | 2 | 3 | i-Pr | (2) F | H | c-Bu | Me |
| 1070 | $CH_2$ | 2 | 3 | i-Pr | (3) F | H | Me | Me |
| 1071 | $CH_2$ | 2 | 3 | i-Pr | (4) F | H | Et | Et |
| 1072 | $CH_2$ | 2 | 3 | i-Pr | (2) Cl | H | Me | Me |
| 1073 | $CH_2$ | 2 | 4 | i-Pr | (3) Cl | H | Me | 1-MePr |
| 1074 | $CH_2$ | 2 | 3 | i-Pr | (4) Cl | H | 1-MePr | Me |
| 1075 | $CH_2$ | 2 | 4 | i-Pr | (4)-CN | H | Me | Me |
| 1076 | $CH_2$ | 2 | 3 | i-Pr | (2) Ac | H | Me | Me |
| 1077 | $CH_2$ | 2 | 3 | i-Pr | (3)EtCO- | H | Et | Et |
| 1078 | $CH_2$ | 2 | 3 | i-Pr | (4) Ac | H | i-Pr | i-Pr |
| 1079 | $CH_2$ | 2 | 3 | i-Pr | (4)PhCO- | H | c-Pr | c-Pr |
| 1080 | $CH_2$ | 2 | 3 | i-Pr | (2)i-PrO- | H | n-Bu | n-Bu |
| 1081 | $CH_2$ | 2 | 3 | i-Pr | (3)MeO- | H | t-Bu | t-Bu |
| 1082 | $CH_2$ | 2 | 4 | i-Pr | (4)-MeO | H | Me | Me |
| 1083 | $CH_2$ | 2 | 3 | i-Pr | (4)-$CF_3$ | H | Et | Et |
| 1084 | $CH_2$ | 2 | 3 | i-Pr | (4)$CF_3O$- | H | n-Pr | n-Pr |
| 1085 | $CH_2$ | 2 | 3 | i-Pr | (4)1-imidazol | H | t-Bu | t-Bu |
| 1086 | $CH_2$ | 2 | 3 | i-Pr | (3)n-BuNH- | H | Me | Me |
| 1087 | $CH_2$ | 2 | 4 | i-Pr | (2) Ac | (4) Cl | n-Hex | n-Hex |
| 1088 | $CH_2$ | 2 | 3 | i-Pr | (2)t-BuCO- | (4) F | Me | Me |
| 1089 | $CH_2$ | 2 | 3 | i-Pr | (2) Cl | (4) Ac | Et | Et |
| 1090 | $CH_2$ | 2 | 3 | i-Pr | (2) F | (4) Ac | Et | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 1091 | O | 2 | 2 | Me | H | H | n-Pr | Et |
| 1092 | O | 2 | 2 | Me | (2) F | H | Et | i-Pr |
| 1093 | O | 2 | 2 | Me | (3) F | H | i-Pr | Et |
| 1094 | O | 2 | 2 | Me | (4) F | H | Me | Me |
| 1095 | O | 2 | 2 | Me | (2) Cl | H | Et | Et |
| 1096 | O | 2 | 2 | Me | (3) Cl | H | n-Pr | n-Pr |
| 1097 | O | 2 | 2 | Me | (4) Cl | H | t-Bu | t-Bu |
| 1098 | O | 2 | 2 | Me | (4)-CN | H | Me | Me |
| 1099 | O | 2 | 2 | Me | (2)EtCO- | H | Et | Et |
| 1100 | O | 2 | 2 | Me | (3)n-BuCO- | H | n-Pr | n-Pr |
| 1101 | O | 2 | 2 | Me | (4) Ac | H | i-Pr | i-Pr |
| 1102 | O | 2 | 2 | Me | (4)PhCO- | H | 2-MePr | 2-MePr |
| 1103 | O | 2 | 2 | Me | (2)MeO- | H | Me | Me |
| 1104 | O | 2 | 2 | Me | (3)c-BuS- | H | Et | Et |
| 1105 | O | 2 | 2 | Me | (4)3-MeBuO- | H | Et | c-Pr |
| 1106 | O | 2 | 2 | Me | (4)-$CF_3$ | H | c-Pr | Et |
| 1107 | O | 2 | 2 | Me | (4)$CF_3O$- | H | n-Bu | n-Bu |
| 1108 | O | 2 | 2 | Me | (4)1-imidazol | H | t-Bu | t-Bu |
| 1109 | O | 2 | 2 | Me | (4)n-PrCO- | H | 3-MeBu | 3-MeBu |
| 1110 | O | 2 | 2 | Me | (2)t-BuCO- | (4) Cl | c-Hex | c-Hex |
| 1111 | O | 2 | 2 | Me | (2) Ac | (4) F | Me | Me |
| 1112 | O | 2 | 2 | Me | (2) Cl | (4) Ac | Et | Et |
| 1113 | O | 2 | 2 | Me | (2) F | (4)n-PrCO- | Et | n-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 1114 | O | 2 | 2 | Et | H | H | n-Bu | Et |
| 1115 | O | 2 | 2 | Et | (2) F | H | Me | Me |
| 1116 | O | 2 | 2 | Et | (3) F | H | Et | Et |
| 1117 | O | 2 | 2 | Et | (4) F | H | n-Pr | n-Pr |
| 1118 | O | 2 | 2 | Et | (2) Cl | H | t-Bu | t-Bu |
| 1119 | O | 2 | 2 | Et | (3) Cl | H | Me | Me |
| 1120 | O | 2 | 2 | Et | (4) Cl | H | Et | Et |
| 1121 | O | 2 | 2 | Et | (4)-CN | H | n-Pr | n-Pr |
| 1122 | O | 2 | 2 | Et | (2) Ac | H | i-Pr | i-Pr |
| 1123 | O | 2 | 2 | Et | (3)n-PrCO- | H | 2-MePr | 2-MePr |
| 1124 | O | 2 | 2 | Et | (4) Ac | H | 1-MePr | 1-MePr |
| 1125 | O | 2 | 2 | Et | (4)PhCO- | H | 1,1-diMePr | 1,1-diMePr |
| 1126 | O | 2 | 2 | Et | (2)t-BuO- | H | Me | Me |
| 1127 | O | 2 | 2 | Et | (3)MeS- | H | Et | Et |
| 1128 | O | 2 | 2 | Et | (4)2-MeBuO- | H | Et | t-Bu |
| 1129 | O | 2 | 2 | Et | (4)-$CF_3$ | H | t-Bu | Et |
| 1130 | O | 2 | 2 | Et | (4)$CF_3O$- | H | n-Pr | n-Pr |
| 1131 | O | 2 | 2 | Et | (4)1-imidazol | H | Me | Me |
| 1132 | O | 2 | 2 | Et | (4)i-PrNH- | H | Et | c-Bu |
| 1133 | O | 2 | 2 | Et | (2)EtCO- | (4) Cl | c-Bu | Et |
| 1134 | O | 2 | 2 | Et | (2) Ac | (4) F | Me | Me |
| 1135 | O | 2 | 2 | Et | (2) Cl | (4) Ac | Et | Et |
| 1136 | O | 2 | 2 | Et | (2) F | (4) Ac | 2-MePr | 2-MePr |

Table 6 (Cont'd)

| Comp. No. | A | m | n | $R^3$ | $X^1$ | $X^2$ | $R^2$ | $R^1$ |
|---|---|---|---|---|---|---|---|---|
| 1137 | O | 2 | 2 | n-Pr | H | H | 1,2-diMePr | 1,2-diMePr |
| 1138 | O | 2 | 2 | n-Pr | (2) F | H | Me | Me |
| 1139 | O | 2 | 2 | n-Pr | (3) F | H | Et | Et |
| 1140 | O | 2 | 2 | n-Pr | (4) F | H | n-Pr | n-Pr |
| 1141 | O | 2 | 2 | n-Pr | (2) Cl | H | i-Pr | i-Pr |
| 1142 | O | 2 | 2 | n-Pr | (3) Cl | H | 2-MePr | 2-MePr |
| 1143 | O | 2 | 2 | n-Pr | (4) Cl | H | 1-MePr | 1-MePr |
| 1144 | O | 2 | 2 | n-Pr | (4)-CN | H | 1,1-diMePr | 1,1-diMePr |
| 1145 | O | 2 | 2 | n-Pr | (2)n-BuCO- | H | 2,3-diMeBu | 2,3-diMeBu |
| 1146 | O | 2 | 2 | n-Pr | (3) Ac | H | Me | Me |
| 1147 | O | 2 | 2 | n-Pr | (4)EtCO- | H | Et | Et |
| 1148 | O | 2 | 2 | n-Pr | (4)PhCO- | H | n-Pr | n-Pr |
| 1149 | O | 2 | 2 | n-Pr | (2)EtS- | H | t-Bu | t-Bu |
| 1150 | O | 2 | 2 | n-Pr | (3)MeO- | H | Me | Me |
| 1151 | O | 2 | 2 | n-Pr | (4)4-MePenO- | H | n-Hex | n-Hex |
| 1152 | O | 2 | 2 | n-Pr | (4)-$CF_3$ | H | Me | Me |
| 1153 | O | 2 | 2 | n-Pr | (4)$CF_3O$- | H | Et | Et |
| 1154 | O | 2 | 2 | n-Pr | (4)1-imidazol | H | Et | 2-MePr |
| 1155 | O | 2 | 2 | n-Pr | (2)n-PrCONH- | H | 2-MePr | Et |
| 1156 | O | 2 | 2 | n-Pr | (2)n-PrCO- | (4) Cl | Me | Me |
| 1157 | O | 2 | 2 | n-Pr | (2) Ac | (4) F | Et | Et |
| 1158 | O | 2 | 2 | n-Pr | (2) Cl | (4)i-PrCO- | n-Pr | n-Pr |
| 1159 | O | 2 | 2 | n-Pr | (2) F | (4) Ac | t-Bu | t-Bu |
| 1160 | O | 2 | 2 | i-Pr | H | H | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1161 | O | 2 | 2 | i-Pr | (2) F | H | n-Hex | n-Hex |
| 1162 | O | 2 | 2 | i-Pr | (3) F | H | Me | Me |
| 1163 | O | 2 | 2 | i-Pr | (4) F | H | Et | Et |
| 1164 | O | 2 | 2 | i-Pr | (2) Cl | H | n-Pr | n-Pr |
| 1165 | O | 2 | 2 | i-Pr | (3) Cl | H | i-Pr | i-Pr |
| 1166 | O | 2 | 2 | i-Pr | (4) Cl | H | 2-MePr | 2-MePr |
| 1167 | O | 2 | 2 | i-Pr | (4)-CN | H | 1-MePr | 1-MePr |
| 1168 | O | 2 | 2 | i-Pr | (2) Ac | H | Me | Me |
| 1169 | O | 2 | 2 | i-Pr | (3)t-BuCO- | H | Et | 1-MePr |
| 1170 | O | 2 | 2 | i-Pr | (4) Ac | H | 1-MePr | Et |
| 1171 | O | 2 | 2 | i-Pr | (4)PhCO- | H | Me | Me |
| 1172 | O | 2 | 2 | i-Pr | (2)MeO- | H | Et | Et |
| 1173 | O | 2 | 2 | i-Pr | (3)MeO- | H | n-Pr | i-Pr |
| 1174 | O | 2 | 2 | i-Pr | (4)1-MePrO- | H | i-Pr | n-Pr |
| 1175 | O | 2 | 2 | i-Pr | (4)-$CF_3$ | H | n-Bu | n-Bu |
| 1176 | O | 2 | 2 | i-Pr | (4)$CF_3$O- | H | t-Bu | t-Bu |
| 1177 | O | 2 | 2 | i-Pr | (4)1-imidazol | H | 3-MeBu | 3-MeBu |
| 1178 | O | 2 | 2 | i-Pr | (2)EtCONH- | H | 2-MeBu | 2-MeBu |
| 1179 | O | 2 | 2 | i-Pr | (2) Ac | (4) Cl | c-Hex | c-Hex |
| 1180 | O | 2 | 2 | i-Pr | (2) Ac | (4) F | 4-MePen | 4-MePen |
| 1181 | O | 2 | 2 | i-Pr | (2) Cl | (4) Ac | Me | Me |
| 1182 | O | 2 | 2 | i-Pr | (2) F | (4)n-PrCO- | Et | Et |
| 1183 | O | 2 | 3 | Me | H | H | n-Pr | c-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 1184 | O | 2 | 3 | Me | (2) F | H | c-Pr | n-Pr |
| 1185 | O | 2 | 3 | Me | (3) F | H | Me | Me |
| 1186 | O | 2 | 3 | Me | (4) F | H | Me | Me |
| 1187 | O | 2 | 3 | Me | (2) Cl | H | Me | Me |
| 1188 | O | 2 | 4 | Me | (3) Cl | H | Et | Et |
| 1189 | O | 2 | 3 | Me | (4) Cl | H | i-Pr | c-Pr |
| 1190 | O | 2 | 3 | Me | (4)-CN | H | c-Pr | i-Pr |
| 1191 | O | 2 | 4 | Me | (2) Ac | H | t-Bu | t-Bu |
| 1192 | O | 2 | 3 | Me | (3)n-BuCO- | H | c-Bu | c-Bu |
| 1193 | O | 2 | 3 | Me | (4) Ac | H | Me | Me |
| 1194 | O | 2 | 3 | Me | (4)PhCO- | H | 1,1-diMePr | 1,1-diMePr |
| 1195 | O | 2 | 3 | Me | (2)MeO- | H | 1,2-diMePr | 1,2-diMePr |
| 1196 | O | 2 | 4 | Me | (3)MeO- | H | 2,2-diMePr | 2,2-diMePr |
| 1197 | O | 2 | 3 | Me | (4)n-PrS- | H | n-Hex | n-Hex |
| 1198 | O | 2 | 3 | Me | (4)-$CF_3$ | H | c-Hex | c-Hex |
| 1199 | O | 2 | 3 | Me | (4)$CF_3O$- | H | Et | Et |
| 1200 | O | 2 | 3 | Me | (4)1-imidazol | H | Me | Me |
| 1201 | O | 2 | 3 | Me | (2)n-BuCO- | H | Et | Et |
| 1202 | O | 2 | 3 | Me | (2) Ac | (4) Cl | i-Pr | i-Pr |
| 1203 | O | 2 | 3 | Me | (2) Ac | (4) F | Me | Me |
| 1204 | O | 2 | 3 | Me | (2) Cl | (4) Ac | n-Pr | n-Pr |
| 1205 | O | 2 | 4 | Me | (2) F | (4)EtCO- | n-Pr | n-Bu |
| 1206 | O | 2 | 3 | Et | H | H | n-Bu | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 1207 | O | 2 | 3 | Et | (2) F | H | Me | Me |
| 1208 | O | 2 | 3 | Et | (3) F | H | Me | Me |
| 1209 | O | 2 | 3 | Et | (4) F | H | Me | Me |
| 1210 | O | 2 | 4 | Et | (2) Cl | H | c-Pen | c-Pen |
| 1211 | O | 2 | 3 | Et | (3) Cl | H | c-Hex | c-Hex |
| 1212 | O | 2 | 3 | Et | (4) Cl | H | Me | Me |
| 1213 | O | 2 | 3 | Et | (4)-CN | H | Me | Me |
| 1214 | O | 2 | 3 | Et | (2) Ac | H | Me | Me |
| 1215 | O | 2 | 3 | Et | (3) Ac | H | Et | Et |
| 1216 | O | 2 | 3 | Et | (4) Ac | H | Me | Me |
| 1217 | O | 2 | 3 | Et | (4)PhCO- | H | Me | Me |
| 1218 | O | 2 | 3 | Et | (2)i-PrS- | H | n-Pr | t-Bu |
| 1219 | O | 2 | 3 | Et | (3)1,2,2-triMePr- | H | t-Bu | n-Pr |
| 1220 | O | 2 | 3 | Et | (4)MeO- | H | n-Pr | n-Pr |
| 1221 | O | 2 | 3 | Et | (4)-$CF_3$ | H | Me | Me |
| 1222 | O | 2 | 3 | Et | (4)$CF_3$O- | H | Me | Me |
| 1223 | O | 2 | 3 | Et | (4)1-imidazol | H | Me | Me |
| 1224 | O | 2 | 3 | Et | (4)AcNH- | H | Et | Et |
| 1225 | O | 2 | 3 | Et | (2) Ac | (4) Cl | Me | Me |
| 1226 | O | 2 | 3 | Et | (2) Ac | (4) F | Me | Me |
| 1227 | O | 2 | 3 | Et | (2) Cl | (4) Ac | t-Bu | t-Bu |
| 1228 | O | 2 | 3 | Et | (2) F | (4) Ac | Me | Me |
| 1229 | O | 2 | 3 | n-Pr | H | H | n-Pr | c-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 1230 | O | 2 | 3 | n-Pr | (2) F | H | c-Bu | n-Pr |
| 1231 | O | 2 | 4 | n-Pr | (3) F | H | n-Pen | n-Pen |
| 1232 | O | 2 | 3 | n-Pr | (4) F | H | c-Pen | c-Pen |
| 1233 | O | 2 | 3 | n-Pr | (2) Cl | H | n-Hex | n-Hex |
| 1234 | O | 2 | 3 | n-Pr | (3) Cl | H | Me | Me |
| 1235 | O | 2 | 3 | n-Pr | (4) Cl | H | Me | Me |
| 1236 | O | 2 | 4 | n-Pr | (4)-CN | H | Et | Et |
| 1237 | O | 2 | 4 | n-Pr | (2) Ac | H | n-Pr | n-Pr |
| 1238 | O | 2 | 3 | n-Pr | (3)n-PenCO- | H | i-Pr | i-Pr |
| 1239 | O | 2 | 3 | n-Pr | (4) Ac | H | Me | Me |
| 1240 | O | 2 | 3 | n-Pr | (4)PhCO- | H | Et | Et |
| 1241 | O | 2 | 3 | n-Pr | (2)MeO- | H | i-Pr | i-Pr |
| 1242 | O | 2 | 4 | n-Pr | (3)2,2-diMePrO- | H | t-Bu | t-Bu |
| 1243 | O | 2 | 3 | n-Pr | (4)MeO- | H | 3-MeBu | 3-MeBu |
| 1244 | O | 2 | 3 | n-Pr | (4)-$CF_3$ | H | 4-MePen | 4-MePen |
| 1245 | O | 2 | 3 | n-Pr | (4)$CF_3O$- | H | Et | Et |
| 1246 | O | 2 | 3 | n-Pr | (4)1-imidazol | H | Me | Me |
| 1247 | O | 2 | 3 | n-Pr | (4)2-MePrCONH- | H | Me | Me |
| 1248 | O | 2 | 3 | n-Pr | (2) Ac | (4) Cl | Et | Et |
| 1249 | O | 2 | 3 | n-Pr | (2)t-BuCO- | (4) F | i-Pr | c-Pr |
| 1250 | O | 2 | 3 | n-Pr | (2) Cl | (4) Ac | c-Pr | i-Pr |
| 1251 | O | 2 | 3 | n-Pr | (2) F | (4) Ac | t-Bu | t-Bu |
| 1252 | O | 2 | 4 | i-Pr | H | H | c-Bu | c-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 1253 | O | 2 | 3 | i-Pr | (2) F | H | Me | Me |
| 1254 | O | 2 | 3 | i-Pr | (3) F | H | Et | Et |
| 1255 | O | 2 | 3 | i-Pr | (4) F | H | n-Pr | n-Pr |
| 1256 | O | 2 | 3 | i-Pr | (2) Cl | H | t-Bu | t-Bu |
| 1257 | O | 2 | 3 | i-Pr | (3) Cl | H | Me | Me |
| 1258 | O | 2 | 3 | i-Pr | (4) Cl | H | n-Hex | n-Hex |
| 1259 | O | 2 | 3 | i-Pr | (4)-CN | H | Me | Me |
| 1260 | O | 2 | 4 | i-Pr | (2)EtCO- | H | Et | Et |
| 1261 | O | 2 | 3 | i-Pr | (3) Ac | H | n-Pr | n-Pr |
| 1262 | O | 2 | 3 | i-Pr | (4) Ac | H | Me | Me |
| 1263 | O | 2 | 3 | i-Pr | (4)PhCO- | H | t-Bu | t-Bu |
| 1264 | O | 2 | 3 | i-Pr | (2)MeO- | H | Me | Me |
| 1265 | O | 2 | 3 | i-Pr | (3)MeO- | H | 1,1-diMePr | 1,1-diMePr |
| 1266 | O | 2 | 3 | i-Pr | (4)2,3-diMeBuO- | H | Me | Me |
| 1267 | O | 2 | 4 | i-Pr | (4)-$CF_3$ | H | Et | Et |
| 1268 | O | 2 | 3 | i-Pr | (4)$CF_3O$- | H | i-Pr | i-Pr |
| 1269 | O | 2 | 3 | i-Pr | (4)1-imidazol | H | Me | Me |
| 1270 | O | 2 | 3 | i-Pr | (3)t-BuCONH- | H | Me | Me |
| 1271 | O | 2 | 3 | i-Pr | (2)n-PrCO- | (4) Cl | Et | Et |
| 1272 | O | 2 | 4 | i-Pr | (2)n-BuCO- | (4) F | n-Pr | n-Pr |
| 1273 | O | 2 | 3 | i-Pr | (2) Cl | (4) Ac | t-Bu | t-Bu |
| 1274 | O | 2 | 3 | i-Pr | (2) F | (4) Ac | n-Pr | 2-MePr |
| 1275 | O | 3 | 2 | Me | H | H | 2-MePr | n-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 1276 | O | 3 | 2 | Me | (2) F | H | n-Pr | 1-MePr |
| 1277 | O | 3 | 2 | Me | (3) F | H | 1-MePr | n-Pr |
| 1278 | O | 4 | 2 | Me | (4) F | H | n-Bu | n-Bu |
| 1279 | O | 3 | 2 | Me | (2) Cl | H | n-Pen | n-Pen |
| 1280 | O | 3 | 2 | Me | (3) Cl | H | c-Hex | c-Hex |
| 1281 | O | 3 | 2 | Me | (4) Cl | H | Me | Me |
| 1282 | O | 3 | 2 | Me | (4)-CN | H | Et | Et |
| 1283 | O | 3 | 2 | Me | (2) Ac | H | Me | Me |
| 1284 | O | 4 | 2 | Me | (3)n-PenCO- | H | Et | Et |
| 1285 | O | 3 | 2 | Me | (4) Ac | H | i-Pr | c-Pr |
| 1286 | O | 3 | 2 | Me | (4)PhCO- | H | c-Pr | i-Pr |
| 1287 | O | 3 | 2 | Me | (2)1,2-diMePrO- | H | t-Bu | t-Bu |
| 1288 | O | 3 | 2 | Me | (3)MeO- | H | c-Bu | c-Bu |
| 1289 | O | 3 | 2 | Me | (4)MeO- | H | 1,1-diMePr | 1,1-diMePr |
| 1290 | O | 3 | 2 | Me | (4)-$CF_3$ | H | 1,2,2-triMePr | 1,2,2-triMePr |
| 1291 | O | 3 | 2 | Me | (4)$CF_3O$- | H | Me | Me |
| 1292 | O | 4 | 2 | Me | (4)1-imidazol | H | Et | Et |
| 1293 | O | 3 | 2 | Me | (2)t-BuCONH- | H | Me | Me |
| 1294 | O | 3 | 2 | Me | (2) Ac | (4) Cl | Me | Me |
| 1295 | O | 3 | 2 | Me | (2) Ac | (4) F | Et | Et |
| 1296 | O | 3 | 2 | Me | (2) Cl | (4) Ac | n-Pr | n-Pr |
| 1297 | O | 3 | 2 | Me | (2) F | (4)i-PrCO- | i-Pr | i-Pr |
| 1298 | O | 3 | 2 | Et | H | H | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1299 | O | 3 | 2 | Et | (2) F | H | Et | 1-MePr |
| 1300 | O | 3 | 2 | Et | (3) F | H | 1-MePr | Et |
| 1301 | O | 3 | 2 | Et | (4) F | H | Me | Me |
| 1302 | O | 3 | 2 | Et | (2) Cl | H | Et | Et |
| 1303 | O | 3 | 2 | Et | (3) Cl | H | n-Pr | i-Pr |
| 1304 | O | 3 | 2 | Et | (4) Cl | H | i-Pr | n-Pr |
| 1305 | O | 3 | 2 | Et | (4)-CN | H | n-Bu | n-Bu |
| 1306 | O | 4 | 2 | Et | (2) Ac | H | t-Bu | t-Bu |
| 1307 | O | 3 | 2 | Et | (3)2-MePrCO- | H | 3-MeBu | 3-MeBu |
| 1308 | O | 3 | 2 | Et | (4) Ac | H | 2-MeBu | 2-MeBu |
| 1309 | O | 4 | 2 | Et | (4)PhCO- | H | c-Hex | c-Hex |
| 1310 | O | 3 | 2 | Et | (2)t-BuS- | H | i-Pr | n-Bu |
| 1311 | O | 4 | 2 | Et | (3)t-BuS- | H | n-Bu | i-Pr |
| 1312 | O | 3 | 2 | Et | (4)1-MePrO- | H | i-Pr | t-Bu |
| 1313 | O | 3 | 2 | Et | (4)-$CF_3$ | H | t-Bu | i-Pr |
| 1314 | O | 3 | 2 | Et | (4)-$OCF_3$ | H | Me | Me |
| 1315 | O | 3 | 2 | Et | (4)-1-imidazol | H | Et | Et |
| 1316 | O | 3 | 2 | Et | (2)i-PrCONH- | H | n-Pr | n-Pr |
| 1317 | O | 3 | 2 | Et | (2) Ac | (4) Cl | t-Bu | t-Bu |
| 1318 | O | 3 | 2 | Et | (2) Ac | (4) F | n-Pr | 2-MePr |
| 1319 | O | 4 | 2 | Et | (2) Cl | (4) Ac | 2-MePr | n-Pr |
| 1320 | O | 3 | 2 | Et | (2) F | (4) Ac | n-Pr | 1-MePr |

Table 6 (Cont'd)

| Comp. No. | A | m | n | $R^3$ | $X^1$ | $X^2$ | $R^2$ | $R^1$ |
|---|---|---|---|---|---|---|---|---|
| 1321 | O | 3 | 2 | n-Pr | H | H | 1-MePr | n-Pr |
| 1322 | O | 3 | 2 | n-Pr | (2) F | H | n-Bu | n-Bu |
| 1323 | O | 3 | 2 | n-Pr | (3) F | H | n-Pen | n-Pen |
| 1324 | O | 3 | 2 | n-Pr | (4) F | H | c-Hex | c-Hex |
| 1325 | O | 4 | 2 | n-Pr | (2) Cl | H | n-Hex | n-Hex |
| 1326 | O | 3 | 2 | n-Pr | (3) Cl | H | Me | Me |
| 1327 | O | 3 | 2 | n-Pr | (4) Cl | H | Et | Et |
| 1328 | O | 3 | 2 | n-Pr | (4)-CN | H | i-Pr | i-Pr |
| 1329 | O | 3 | 2 | n-Pr | (2) Ac | H | t-Bu | t-Bu |
| 1330 | O | 4 | 2 | n-Pr | (3) Ac | H | H | H |
| 1331 | O | 3 | 2 | n-Pr | (4)n-PrCO- | H | Me | Me |
| 1332 | O | 3 | 2 | n-Pr | (4)PhCO- | H | Et | Et |
| 1333 | O | 3 | 2 | n-Pr | (2)MeO- | H | n-Pr | n-Pr |
| 1334 | O | 4 | 2 | n-Pr | (3)n-PenS- | H | t-Bu | t-Bu |
| 1335 | O | 3 | 2 | n-Pr | (4)MeO- | H | c-Pen | c-Pen |
| 1336 | O | 3 | 2 | n-Pr | (4)-$CF_3$ | H | n-Hex | n-Hex |
| 1337 | O | 3 | 2 | n-Pr | (4)$CF_3O$- | H | Me | Et |
| 1338 | O | 3 | 2 | n-Pr | (4)1-imidazol | H | Et | Me |
| 1339 | O | 3 | 2 | n-Pr | (4)EtCONH- | H | Me | Me |
| 1340 | O | 3 | 2 | n-Pr | (2) Ac | (4) Cl | i-Pr | i-Pr |
| 1341 | O | 4 | 2 | n-Pr | (2) Ac | (4) F | 2-MePr | 2-MePr |
| 1342 | O | 3 | 2 | n-Pr | (2) Cl | (4)n-PrCO- | n-Pen | n-Pen |

Table 6 (Cont'd)

| Comp. No. | A | m | n | $R^3$ | $X^1$ | $X^2$ | $R^2$ | $R^1$ |
|---|---|---|---|---|---|---|---|---|
| 1343 | O | 3 | 2 | n-Pr | (2) F | (4) Ac | 4-MePen | 4-MePen |
| 1344 | O | 3 | 2 | i-Pr | H | H | Me | n-Pr |
| 1345 | O | 4 | 2 | i-Pr | (2) F | H | n-Pr | Me |
| 1346 | O | 3 | 2 | i-Pr | (3) F | H | Et | Et |
| 1347 | O | 3 | 2 | i-Pr | (4) F | H | n-Pr | n-Pr |
| 1348 | O | 3 | 2 | i-Pr | (2) Cl | H | t-Bu | t-Bu |
| 1349 | O | 3 | 2 | i-Pr | (3) Cl | H | n-Hex | n-Hex |
| 1350 | O | 3 | 2 | i-Pr | (4) Cl | H | c-Hex | c-Hex |
| 1351 | O | 3 | 2 | i-Pr | (4)-CN | H | 4-MePen | 4-MePen |
| 1352 | O | 3 | 2 | i-Pr | (2) Ac | H | 3-MePen | 3-MePen |
| 1353 | O | 3 | 2 | i-Pr | (3)EtCO- | H | 2-MePen | 2-MePen |
| 1354 | O | 4 | 2 | i-Pr | (4) Ac | H | 1-MePen | 1-MePen |
| 1355 | O | 3 | 2 | i-Pr | (4)PhCO- | H | 1,2,2-triMePr | 1,2,2-triMePr |
| 1356 | O | 3 | 2 | i-Pr | (2)MeO- | H | 1,1,2-triMePr | 1,1,2-triMePr |
| 1357 | O | 3 | 2 | i-Pr | (3)1,1-diMeBuO- | H | 1,1-diMeBu | 1,1-diMeBu |
| 1358 | O | 3 | 2 | i-Pr | (4)MeO- | H | 2,2-diMeBu | 2,2-diMeBu |
| 1359 | O | 3 | 2 | i-Pr | (4)-$CF_3$ | H | 1,3-diMeBu | 1,3-diMeBu |
| 1360 | O | 3 | 2 | i-Pr | (4)$CF_3O$- | H | 2,3-diMeBu | 2,3-diMeBu |
| 1361 | O | 4 | 2 | i-Pr | (4)1-imidazol | H | i-Pr | i-Pr |
| 1362 | O | 3 | 2 | i-Pr | (4)n-PrCONH- | H | 2-MePr | 2-MePr |
| 1363 | O | 3 | 2 | i-Pr | (2) Ac | (4) Cl | n-Pen | n-Pen |

Table 6 (Cont'd)

| Comp. No. | A | m | n | $R^3$ | $X^1$ | $X^2$ | $R^2$ | $R^1$ |
|---|---|---|---|---|---|---|---|---|
| 1364 | O | 3 | 2 | i-Pr | (2)n-PenCO- | (4) F | 4-MePen | 4-MePen |
| 1365 | O | 3 | 2 | i-Pr | (2) Cl | (4) Ac | Me | n-Pr |
| 1366 | O | 3 | 2 | i-Pr | (2) F | (4) Ac | n-Pr | Me |
| 1367 | O | 3 | 3 | Me | H | H | Et | Et |
| 1368 | O | 3 | 3 | Me | (2) F | H | n-Pr | n-Pr |
| 1369 | O | 4 | 3 | Me | (3) F | H | t-Bu | t-Bu |
| 1370 | O | 3 | 3 | Me | (4) F | H | n-Hex | n-Hex |
| 1371 | O | 3 | 4 | Me | (2) Cl | H | Me | Et |
| 1372 | O | 3 | 3 | Me | (3) Cl | H | Et | Me |
| 1373 | O | 3 | 3 | Me | (4) Cl | H | i-Pr | c-Bu |
| 1374 | O | 4 | 4 | Me | (4)-CN | H | c-Bu | i-Pr |
| 1375 | O | 3 | 3 | Me | (2) Ac | H | i-Pr | 2-MePr |
| 1376 | O | 3 | 3 | Me | (3)n-PrCO- | H | 2-MePr | i-Pr |
| 1377 | O | 3 | 3 | Me | (4) Ac | H | Me | Et |
| 1378 | O | 3 | 3 | Me | (4)PhCO- | H | Et | Me |
| 1379 | O | 3 | 4 | Me | (2)2-MePenO- | H | i-Pr | 1-MePr |
| 1380 | O | 3 | 3 | Me | (3)MeO- | H | 1-MePr | i-Pr |
| 1381 | O | 3 | 3 | Me | (4)n-HexS- | H | n-Pen | n-Pen |
| 1382 | O | 3 | 3 | Me | (4)-$CF_3$ | H | 2,3-diMeBu | 2,3-diMeBu |
| 1383 | O | 3 | 3 | Me | (4)$CF_3O$- | H | H | H |
| 1384 | O | 3 | 3 | Me | (4)1-imidazol | H | Me | Me |
| 1385 | O | 3 | 3 | Me | (3)n-BuCONH- | H | Et | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 1386 | O | 3 | 4 | Me | (2) Ac | (4) Cl | n-Pr | n-Pr |
| 1387 | O | 4 | 3 | Me | (2) Ac | (4) F | t-Bu | t-Bu |
| 1388 | O | 3 | 3 | Me | (2) Cl | (4)i-PrCO- | c-Pen | c-Pen |
| 1389 | O | 3 | 3 | Me | (2) F | (4) Ac | n-Hex | n-Hex |
| 1390 | O | 4 | 4 | Et | H | H | H | H |
| 1391 | O | 3 | 3 | Et | (2) F | H | Me | Me |
| 1392 | O | 3 | 3 | Et | (3) F | H | Et | Et |
| 1393 | O | 3 | 3 | Et | (4) F | H | n-Pr | n-Pr |
| 1394 | O | 3 | 3 | Et | (2) Cl | H | t-Bu | t-Bu |
| 1395 | O | 4 | 3 | Et | (3) Cl | H | c-Pen | c-Pen |
| 1396 | O | 3 | 3 | Et | (4) Cl | H | n-Hex | n-Hex |
| 1397 | O | 3 | 3 | Et | (4)-CN | H | Me | Et |
| 1398 | O | 3 | 3 | Et | (2) Ac | H | Et | Me |
| 1399 | O | 3 | 3 | Et | (3)2-MePrCO- | H | Me | Me |
| 1400 | O | 3 | 3 | Et | (4) Ac | H | Et | Et |
| 1401 | O | 3 | 3 | Et | (4)PhCO- | H | i-Pr | i-Pr |
| 1402 | O | 3 | 3 | Et | (2)c-PenS- | H | c-Bu | c-Bu |
| 1403 | O | 3 | 3 | Et | (3)1,1-diMeBu- | H | n-Pen | n-Pen |
| 1404 | O | 4 | 3 | Et | (4)MeO- | H | 2-MePen | 2-MePen |
| 1405 | O | 3 | 3 | Et | (4)-$CF_3$ | H | Me | Me |
| 1406 | O | 3 | 4 | Et | (4)$CF_3O$- | H | Et | Et |
| 1407 | O | 3 | 3 | Et | (4)1-imidazol | H | c-Pr | n-Bu |

Table 6 (Cont'd)

| Comp. No. | A | m | n | $R^3$ | $X^1$ | $X^2$ | $R^2$ | $R^1$ |
|---|---|---|---|---|---|---|---|---|
| 1408 | O | 4 | 3 | Et | (3)n-PenCONH- | H | n-Bu | c-Pr |
| 1409 | O | 3 | 3 | Et | (2) Ac | (4) Cl | Me | Me |
| 1410 | O | 3 | 3 | Et | (2) Ac | (4) F | c-Pr | t-Bu |
| 1411 | O | 3 | 3 | Et | (2) Cl | (4)n-PrCO- | t-Bu | c-Pr |
| 1412 | O | 3 | 3 | Et | (2) F | (4) Ac | Me | Me |
| 1413 | O | 3 | 3 | n-Pr | H | H | Et | Et |
| 1414 | O | 4 | 4 | n-Pr | (2) F | H | i-Pr | i-Pr |
| 1415 | O | 3 | 3 | n-Pr | (3) F | H | n-Pr | i-Pr |
| 1416 | O | 3 | 3 | n-Pr | (4) F | H | i-Pr | n-Pr |
| 1417 | O | 3 | 3 | n-Pr | (2) Cl | H | 1-MePr | 1-MePr |
| 1418 | O | 3 | 3 | n-Pr | (3) Cl | H | c-Pr | c-Bu |
| 1419 | O | 3 | 3 | n-Pr | (4) Cl | H | c-Bu | c-Pr |
| 1420 | O | 3 | 3 | n-Pr | (4)-CN | H | n-Pen | n-Pen |
| 1421 | O | 3 | 3 | n-Pr | (2) Ac | H | 4-MePen | 4-MePen |
| 1422 | O | 3 | 3 | n-Pr | (3) Ac | H | H | H |
| 1423 | O | 3 | 3 | n-Pr | (4)n-PenCO- | H | Me | Me |
| 1424 | O | 4 | 3 | n-Pr | (4)PhCO- | H | Et | Et |
| 1425 | O | 3 | 3 | n-Pr | (2)MeO- | H | n-Pr | n-Pr |
| 1426 | O | 3 | 4 | n-Pr | (3)EtS- | H | t-Bu | t-Bu |
| 1427 | O | 3 | 3 | n-Pr | (4)EtO- | H | c-Pen | c-Pen |
| 1428 | O | 3 | 3 | n-Pr | (4)-$CF_3$ | H | n-Hex | n-Hex |
| 1429 | O | 3 | 3 | n-Pr | (4)$CF_3O$- | H | Me | Et |

Table 6 (Cont'd)

| Comp. No. | A | m | n | $R^3$ | $X^1$ | $X^2$ | $R^2$ | $R^1$ |
|---|---|---|---|---|---|---|---|---|
| 1430 | O | 3 | 3 | n-Pr | (4)1-imidazol | H | Et | Me |
| 1431 | O | 3 | 3 | n-Pr | (4)i-PrCONH- | H | Me | n-Pr |
| 1432 | O | 3 | 3 | n-Pr | (2)EtCO- | (4) Cl | n-Pr | Me |
| 1433 | O | 3 | 3 | n-Pr | (2) Ac | (4) F | Et | Et |
| 1434 | O | 3 | 3 | n-Pr | (2) Cl | (4) Ac | n-Pr | n-Pr |
| 1435 | O | 4 | 4 | n-Pr | (2) F | (4) Ac | t-Bu | t-Bu |
| 1436 | O | 3 | 3 | i-Pr | H | H | Me | Me |
| 1437 | O | 3 | 3 | i-Pr | (2) F | H | Et | Et |
| 1438 | O | 4 | 4 | i-Pr | (3) F | H | n-Pr | n-Pr |
| 1439 | O | 3 | 3 | i-Pr | (4) F | H | i-Pr | i-Pr |
| 1440 | O | 3 | 3 | i-Pr | (2) Cl | H | 1-MePr | 1-MePr |
| 1441 | O | 3 | 3 | i-Pr | (3) Cl | H | Me | Me |
| 1442 | O | 3 | 3 | i-Pr | (4) Cl | H | Et | Et |
| 1443 | O | 3 | 3 | i-Pr | (4)-CN | H | n-Pr | n-Pr |
| 1444 | O | 3 | 3 | i-Pr | (2) Ac | H | t-Bu | t-Bu |
| 1445 | O | 3 | 3 | i-Pr | (3)EtCO- | H | c-Pen | c-Pen |
| 1446 | O | 4 | 4 | i-Pr | (4) Ac | H | n-Hex | n-Hex |
| 1447 | O | 3 | 3 | i-Pr | (4)PhCO- | H | Me | Et |
| 1448 | O | 3 | 3 | i-Pr | (2)n-PrO- | H | Et | Me |
| 1449 | O | 3 | 3 | i-Pr | (3)i-PrO- | H | Me | Me |
| 1450 | O | 3 | 3 | i-Pr | (4)n-PrO- | H | i-Pr | i-Pr |
| 1451 | O | 3 | 3 | i-Pr | (4)-$CF_3$ | H | c-Pr | 1-MePr |

Table 6 (Cont'd)

| Comp. No. | A | m | n | $R^3$ | $X^1$ | $X^2$ | $R^2$ | $R^1$ |
|---|---|---|---|---|---|---|---|---|
| 1452 | O | 3 | 3 | i-Pr | (4)$CF_3O$- | H | 1-MePr | c-Pr |
| 1453 | O | 3 | 3 | i-Pr | (4)1-imidazol | H | Me | Et |
| 1454 | O | 4 | 3 | i-Pr | (4)t-BuCONH- | H | Et | Me |
| 1455 | O | 3 | 3 | i-Pr | (2) Ac | (4) Cl | c-Pr | 2-MePr |
| 1456 | O | 4 | 3 | i-Pr | (2) Ac | (4) F | 2-MePr | c-Pr |
| 1457 | O | 3 | 3 | i-Pr | (2) Cl | (4)n-PrCO- | H | H |
| 1458 | O | 3 | 3 | i-Pr | (2) F | (4) Ac | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1459 | $CH_2$ | 1 | 2 | Me | (4)$PhSO_2NH$- | H | Et | Et |
| 1460 | $CH_2$ | 1 | 2 | Et | (3)$PhSO_2NH$- | H | n-Pr | n-Pr |
| 1461 | $CH_2$ | 1 | 2 | n-Pr | (2)$PhSO_2NH$- | H | t-Bu | t-Bu |
| 1462 | $CH_2$ | 1 | 2 | i-Pr | (4)$PhSO_2NH$- | H | c-Pen | c-Pen |
| 1463 | $CH_2$ | 1 | 4 | Me | (3)$PhSO_2NH$- | H | n-Hex | n-Hex |
| 1464 | $CH_2$ | 1 | 3 | Et | (4)$PhSO_2NH$- | H | Me | Me |
| 1465 | $CH_2$ | 1 | 3 | n-Pr | (4)$PhSO_2NH$- | H | Me | Me |
| 1466 | $CH_2$ | 1 | 3 | i-Pr | (4)$PhSO_2NH$- | H | Me | Me |
| 1467 | $CH_2$ | 2 | 2 | Me | (4)$PhSO_2NH$- | H | Et | Et |
| 1468 | $CH_2$ | 2 | 2 | Et | (4)$PhSO_2NH$- | H | i-Pr | i-Pr |
| 1469 | $CH_2$ | 2 | 2 | n-Pr | (3)$PhSO_2NH$- | H | c-Bu | c-Bu |
| 1470 | $CH_2$ | 2 | 2 | i-Pr | (2)$PhSO_2NH$- | H | n-Pen | n-Pen |
| 1471 | $CH_2$ | 2 | 3 | Me | (4)$PhSO_2NH$- | H | 2-MePen | 2-MePen |
| 1472 | $CH_2$ | 2 | 3 | Et | (2)$PhSO_2NH$- | H | Me | Me |
| 1473 | $CH_2$ | 2 | 3 | n-Pr | (4)$PhSO_2NH$- | H | Et | Et |
| 1474 | $CH_2$ | 2 | 4 | i-Pr | (3)$PhSO_2NH$- | H | n-Pr | n-Pr |
| 1475 | O | 2 | 2 | Me | (2)$PhSO_2NH$- | H | i-Pr | i-Pr |
| 1476 | O | 2 | 2 | Et | (2)$PhSO_2NH$- | H | i-Pr | i-Pr |
| 1477 | O | 2 | 2 | n-Pr | (4)$PhSO_2NH$- | H | 2-MePr | 2-MePr |
| 1478 | O | 2 | 2 | i-Pr | (4)$PhSO_2NH$- | H | n-Pen | n-Pen |
| 1479 | O | 2 | 3 | Me | (4)$PhSO_2NH$- | H | 4-MePen | 4-MePen |
| 1480 | O | 2 | 4 | Et | (4)$PhSO_2NH$- | H | Me | n-Pr |
| 1481 | O | 2 | 3 | n-Pr | (3)$PhSO_2NH$- | H | n-Pr | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1482 | O | 2 | 3 | i-Pr | (3)$PhSO_2NH-$ | H | Et | Et |
| 1483 | O | 3 | 2 | Me | (4)$PhSO_2NH-$ | H | n-Pr | n-Pr |
| 1484 | O | 3 | 2 | Et | (2)$PhSO_2NH-$ | H | n-Bu | t-Bu |
| 1485 | O | 3 | 2 | n-Pr | (2)$PhSO_2NH-$ | H | t-Bu | n-Bu |
| 1486 | O | 3 | 2 | i-Pr | (4)$PhSO_2NH-$ | H | n-Bu | c-Bu |
| 1487 | O | 4 | 3 | Me | (4)$PhSO_2NH-$ | H | c-Bu | n-Bu |
| 1488 | O | 3 | 3 | Et | (3)$PhSO_2NH-$ | H | H | H |
| 1489 | O | 3 | 3 | n-Pr | (4)$PhSO_2NH-$ | H | Me | Me |
| 1490 | O | 3 | 3 | i-Pr | (4)$PhSO_2NH-$ | H | Et | Et |
| 1491 | $CH_2$ | 1 | 2 | EtOMe- | (4)$-NO_2$ | H | n-Pr | n-Pr |
| 1492 | $CH_2$ | 1 | 2 | EtOEt- | (4)$-NH_2$ | H | n-Bu | 2-MePr |
| 1493 | $CH_2$ | 1 | 2 | EtOBu- | (4)$CH_3SO_2NH-$ | H | 2-MePr | n-Bu |
| 1494 | $CH_2$ | 1 | 2 | EtOEt- | (4) F | H | Et | Et |
| 1495 | $CH_2$ | 1 | 3 | MeOEt- | (4)$-NO_2$ | H | Me | Me |
| 1496 | $CH_2$ | 1 | 3 | MeOEt- | (4)$-NH_2$ | H | Me | Me |
| 1497 | $CH_2$ | 1 | 3 | MeOEt- | (4)$CH_3SO_2NH-$ | H | Et | Et |
| 1498 | $CH_2$ | 1 | 3 | n-PenOPr- | (4) F | H | n-Bu | 1-MePr |
| 1499 | $CH_2$ | 2 | 2 | c-PenOEt- | (4)$-NO_2$ | H | 1-MePr | n-Bu |
| 1500 | $CH_2$ | 2 | 2 | c-PenOBu- | (4)$-NH_2$ | H | t-Bu | c-Bu |
| 1501 | $CH_2$ | 2 | 2 | c-PenOEt- | (4)$CH_3SO_2NH-$ | H | c-Bu | t-Bu |
| 1502 | $CH_2$ | 2 | 2 | i-PenOEt- | (4) F | H | Me | Me |
| 1503 | $CH_2$ | 2 | 3 | n-BuOEt- | (4)$-NO_2$ | H | Et | Et |
| 1504 | $CH_2$ | 2 | 4 | n-BuOEt- | (4)$-NH_2$ | H | i-Pr | i-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 1505 | $CH_2$ | 2 | 3 | n-BuOMe- | (4)$CH_3SO_2NH$- | H | c-Bu | c-Bu |
| 1506 | $CH_2$ | 2 | 3 | i-PrOEt- | (4) F | H | n-Pen | n-Pen |
| 1507 | O | 2 | 2 | n-PrOEt- | (4)-$NO_2$ | H | 2-MePen | 2-MePen |
| 1508 | O | 2 | 2 | n-PrOEt- | (4)-$NH_2$ | H | Me | Me |
| 1509 | O | 2 | 2 | n-PrOEt- | (4)$CH_3SO_2NH$- | H | Et | Et |
| 1510 | O | 2 | 2 | t-BuOPr- | (4) F | H | t-Bu | 2-MePr |
| 1511 | O | 2 | 4 | c-HexOEt- | (4)-$NO_2$ | H | 2-MePr | t-Bu |
| 1512 | O | 2 | 3 | c-HexOEt- | (4)-$NH_2$ | H | t-Bu | 1-MePr |
| 1513 | O | 2 | 3 | c-HexOEt- | (4)$CH_3SO_2NH$- | H | 1-MePr | t-Bu |
| 1514 | O | 2 | 3 | MeOEt- | (4) F | H | Me | Me |
| 1515 | O | 3 | 2 | n-HexOEt- | (4)-$NO_2$ | H | Et | Et |
| 1516 | O | 3 | 2 | n-HexOEt- | (4)-$NH_2$ | H | n-Pr | n-Pr |
| 1517 | O | 3 | 2 | n-HexOEt- | (4)$CH_3SO_2NH$- | H | i-Pr | i-Pr |
| 1518 | O | 3 | 2 | i-PrOEt- | (4) F | H | c-Bu | 2-MePr |
| 1519 | O | 3 | 3 | MeOPr- | (4)-$NO_2$ | H | 2-MePr | c-Bu |
| 1520 | O | 4 | 3 | MeOBu- | (4)-$NH_2$ | H | c-Bu | 1-MePr |
| 1521 | O | 3 | 3 | MeOEt- | (4)$CH_3SO_2NH$- | H | 1-MePr | c-Bu |
| 1522 | O | 3 | 4 | MeOMe- | (4) F | H | 1-MePr | 2-MePr |
| 1523 | $CH_2$ | 1 | 2 | di(n-Pr)NEt- | (4)-$NO_2$ | H | 2-MePr | 1-MePr |
| 1524 | $CH_2$ | 1 | 2 | di(i-Pr)NEt- | (4)-$NH_2$ | H | n-Pen | n-Pen |
| 1525 | $CH_2$ | 1 | 2 | di(c-Pr)NEt- | (4)$CH_3SO_2NH$- | H | c-Pen | c-Pen |
| 1526 | $CH_2$ | 1 | 2 | diEtNEt- | (4) F | H | Et | Et |
| 1527 | $CH_2$ | 1 | 3 | diMeNEt- | (4)-$NO_2$ | H | Me | Me |

Table 6 (Cont'd)

| Comp. No. | A | m | n | $R^3$ | $X^1$ | $X^2$ | $R^2$ | $R^1$ |
|---|---|---|---|---|---|---|---|---|
| 1528 | $CH_2$ | 1 | 3 | diMeNEt- | (4)-$NH_2$ | H | Me | Me |
| 1529 | $CH_2$ | 1 | 3 | diMeNEt- | (4)$CH_3SO_2NH$- | H | Me | Me |
| 1530 | $CH_2$ | 1 | 3 | di(n-Bu)NEt- | (4) F | H | n-Hex | n-Hex |
| 1531 | $CH_2$ | 2 | 2 | diEtNEt- | (4)-$NO_2$ | H | c-Hex | c-Hex |
| 1532 | $CH_2$ | 2 | 2 | diEtNPr- | (4)-$NH_2$ | H | H | H |
| 1533 | $CH_2$ | 2 | 2 | diEtNPr- | (4)$CH_3SO_2NH$- | H | Me | Me |
| 1534 | $CH_2$ | 2 | 2 | diMeNMe- | (4) F | H | Et | Et |
| 1535 | $CH_2$ | 2 | 3 | di(i-Pr)NEt- | (4)-$NO_2$ | H | n-Pr | n-Pr |
| 1536 | $CH_2$ | 2 | 3 | di(i-Pr)NEt- | (4)-$NH_2$ | H | t-Bu | t-Bu |
| 1537 | $CH_2$ | 2 | 3 | di(i-Pr)NBu- | (4)$CH_3SO_2NH$- | H | c-Pen | c-Pen |
| 1538 | $CH_2$ | 2 | 4 | di(t-Bu)NEt- | (4) F | H | n-Hex | n-Hex |
| 1539 | O | 2 | 2 | di(n-Pen)NEt- | (4)-$NO_2$ | H | Me | Et |
| 1540 | O | 2 | 2 | di(n-Pen)NMe- | (4)-$NH_2$ | H | Et | Me |
| 1541 | O | 2 | 2 | di(n-Pen)NEt- | (4)$CH_3SO_2NH$- | H | n-Pr | n-Pr |
| 1542 | O | 2 | 2 | di(c-Hex)NEt- | (4) F | H | t-Bu | t-Bu |
| 1543 | O | 2 | 3 | di(c-Hex)NBu- | (4)-$NO_2$ | H | 1,1-diMePr | 1,1-diMePr |
| 1544 | O | 2 | 3 | di(c-Hex)NPr- | (4)-$NH_2$ | H | n-Hex | n-Hex |
| 1545 | O | 2 | 3 | di(c-Hex)NMe- | (4)$CH_3SO_2NH$- | H | Me | n-Bu |
| 1546 | O | 2 | 3 | diMeNEt- | (4) F | H | Me | Me |
| 1547 | O | 3 | 2 | MeEtNEt- | (4)-$NO_2$ | H | Me | Me |
| 1548 | O | 3 | 2 | EtPrNMe- | (4)-$NH_2$ | H | Et | Et |
| 1549 | O | 4 | 2 | MeEtNPr- | (4)$CH_3SO_2NH$- | H | i-Pr | i-Pr |

Table 6 (Cont'd)

| Comp. No. | A | m | n | $R^3$ | $X^1$ | $X^2$ | $R^2$ | $R^1$ |
|---|---|---|---|---|---|---|---|---|
| 1550 | O | 3 | 2 | MeBuNEt- | (4) F | H | c-Bu | c-Bu |
| 1551 | O | 3 | 3 | di(2-MePr)NEt- | (4)-$NO_2$ | H | n-Pen | n-Pen |
| 1552 | O | 4 | 3 | di(1-MePr)NEt- | (4)-$NH_2$ | H | 2-MePen | 2-MePen |
| 1553 | O | 4 | 4 | MeBuNPr- | (4)$CH_3SO_2NH$- | H | Me | Me |
| 1554 | O | 3 | 3 | BuPenNMe- | (4) F | H | Et | Et |
| 1555 | $CH_2$ | 1 | 2 | H | H | H | i-Pr | i-Pr |
| 1556 | $CH_2$ | 1 | 3 | H | H | H | Me | Me |
| 1557 | $CH_2$ | 2 | 2 | H | H | H | n-Pr | n-Pr |
| 1558 | $CH_2$ | 2 | 3 | H | (4) MeO- | H | t-Bu | t-Bu |
| 1559 | O | 2 | 2 | H | H | H | n-Pen | n-Hex |
| 1560 | O | 2 | 3 | H | (4)-CN | H | n-Hex | n-Pen |
| 1561 | O | 3 | 2 | H | (4) Ac | H | n-Pen | c-Hex |
| 1562 | O | 3 | 3 | H | H | H | c-Hex | n-Pen |
| 1563 | $CH_2$ | 1 | 2 | H | (4) F | (4) Ac | c-Pen | n-Hex |
| 1564 | $CH_2$ | 1 | 3 | H | (4) Cl | H | n-Hex | c-Pen |
| 1565 | $CH_2$ | 2 | 2 | H | (3) F | H | c-Pen | c-Hex |
| 1566 | $CH_2$ | 2 | 3 | H | (4) AcNH- | H | c-Hex | c-Pen |
| 1567 | O | 2 | 2 | H | (4) F | H | n-Pr | n-Pr |
| 1568 | O | 2 | 3 | H | (4) F | H | Me | Me |
| 1569 | O | 3 | 2 | H | (4) MeS- | H | Me | Me |
| 1570 | O | 3 | 4 | H | (4)-$CF_3$ | H | Et | Et |

In above Tables 5 & 6, many substitution names are described using the following abbreviations:

Me: Methyl Et: Ethyl Pr: Propyl Bu: Butyl Pen: Pentyl Hex: Hexyl Ac : Acetyl Ph: Phenyl Et(before $SO_2$): Ethane Bu (before $SO_2$): Butane Pr(before $SO_2$): Propane Pen (before $SO_2$): Pentane Hex(before $SO_2$): Hexane n: normal i: iso t: tert c: cyclo

For example, substituted groups are represented as follows:

2,2-diMePro means a 2,2- dimethylpropyl group. 1,2,2-triMeBu means a 1,2,2-trimethylbutyl group. (2-MePr)$SO_2$NH-means a 2-methylpropanesulfonylamino group. MeO means a methoxy group. MeS means a methylthio group. EtCONH means an ethylcarbonylamino group. PhCO means a benzoyl group. Di(2-MePr)NEt means an di(2-methylpropyl)aminoethyl group.

The positions of $X^1$ and $X^2$ in the phenyl group are described in the parentheses. For example, (4)-$NH_2$ means "4-amino", i. e., an amino group is substituted at the position 4 (fourth position) of the phenyl group.

Table 7

| Example No. | NMR (Solvent);δppm | IR ($cm^{-1}$) |
|---|---|---|
| 1 | ($CDCl_3$); 1.29(t,3H),2.08(m,2H), 2.77(t,2H),3.46(s,3H),3.59(s,3H), 4.20(q,2H) | (Neat) 2984,1744, 1716,1647 |
| 2 | ($CDCl_3$); 2.03-2.13(m,2H),2.77(t,2H), 3.49(s,2H),3.59(t,2H),3.75(s,3H) | (Neat) 2957,1749, 1717,1625, 1438,1327 |
| 3 | ($CDCl_3$); 2.08(m,2H),2.70(t,2H), 3.35(s,3H),3.44(s,3H),3.66(t,2H), 5.63(s,3H) | (KBr) 1702,1656, 1441 |
| 4 | ($CDCl_3$); 2.07-2.15(m,2H), 2.62-2.68(m,2H),3.28(t,2H), 3.34(s,3H),3.44(s,3H),5.63(s,1H) | |
| 5 | ($CDCl_3$); 1.22(t,3H),1.30(t,3H), 2.04-2.14(m,2H),2.69(t,2H), 3.67(t,2H),3.89-4.04(m,4H), 5.59(s,1H) | (Neat) 2980,1699, 1660,1463, 1401,1346 |
| 6 | ($CDCl_3$);1.44(s,3H),1.46(s,3H), 1.55(s,3H),1.57(s,3H), 2.00-2.10(m,2H),2.62(t,2H), 3.64(t,2H),4.16-4.35(m,1H), 5.10-5.20(m,1H),5.49(s,1H) | (Neat) 2971,1701, 1661,1440, 1378,1360 |
| 7 | ($CDCl_3$); 1.84-1.94(m,2H),2.61(t,2H), 3.33(s,3H),3.41(s,3H),3.56(t,2H), 4.52(s,2H),5.61(s,1H),7.33(m,5H) | |
| 8 | ($CDCl_3$);2.62-2.68(m,2H), 2.80-2.87(m,2H),3.34(s,3H), 3.45(s,3H),3.74(s,3H),5.57(s,1H) | (KBr) 2958,1739, 1702,1663, 1440,1207 |
| 9 | ($CDCl_3$); 1.80-1.90(m,2H),2.64(t,2H), 3.34(s,3H),3.76(t,2H),5.65(s,1H) | |
| 10 | ($CDCl_3$); 3.35(s,3H),3.39(s,3H), 3.56(s,2H),3.79(s,3H),5.69(s,1H) | (Neat) 2956,1652, 1456 |

Table 7 (Cont'd)

| Example No. | NMR (Solvent);δppm | IR ($cm^{-1}$) |
|---|---|---|
| 11 | ($CDCl_3$); 2.78(t,2H),3.33(s,6H), 3.45(s,6H),3.95(t,2H),5.69(s,1H) | (KBr) 3363,1653, 1458,1078, 1047, 765 |
| 12 | ($CDCl_3$); 1.04(t,3H),1.76(m,2H), 2.45-2.64(m,8H),3.34(s,3H), 3.58(t,2H),5.63(s,3H) | (Neat) 3429,2966, 1701,1444, 1374 |
| 13 | ($CDCl_3$); 1.81(m,2H),2.31(s,3H), 2.52-2.62(m,6H),3.34(s,3H), 3.42(s,3H),5.64(s,1H) | (Neat) 3399,2953, 2800,1702, 1655,1446 |
| 14 | ($CDCl_3$); 0.90(t,3H),1.43-1.52(m,2H), 1.70-1.81(m,2H), 2.42-2.63(m,8H), 3.34(s,3H), 3.42(s,3H),3.57(t,2H), 5.63(s,1H) | (Neat) 3447,2959, 1702,1655, 1457,1373, 1051 |
| 15 | ($CDCl_3$); 1.02(d,6H),1.68-1.78(m,2H), 2.49-2.60(m,7H), 3.34(s,3H), 3.42(s,3H), 3.52(t,2H), 5.63(s,1H) | (Neat) 3421,2965, 1702,1655, 1457,1374, 1035 |
| 16 | ($CDCl_3$); 2.35(s,3H),2.62(t,2H), 2.63-2.81(m,4H),3.34(s,3H), 3.43(s,3H),3.63(t,2H),5.62(s,1H) | (Neat) 3446,2956, 1699,1457, 1374 |
| 17 | ($CDCl_3$); 1.08(t,3H),2.61-2.82(m,8H), 3.34(s,3H),3.58(t,2H),5.61(s,1H) | |
| 18 | ($CDCl_3$); 0.87-0.97(m,3H), 1.44-1.57(m,2H),2.47-2.85(m,9H), 3.33(s,3H),3.43(s,3H),3.59(t,2H), 5.61(s,1H) | (Neat) 3393,2961, 1699,1653, 1457,1055 |

Table 7 (Cont'd)

| Example No. | NMR (Solvent); δppm | IR ($cm^{-1}$) |
|---|---|---|
| 19 | ($CDCl_3$); 1.04(d,6H),2.54-2.80(m,6H), 2.90-3.05(m,1H),3.34(s,3H), 3.43(s,3H),3.53(t,2H),5.61(s,1H) | (Neat) 3447,2966, 1653,1457, 1373 |
| 20 | ($CDCl_3$); 1.07(t,3H),1.66-1.84(m,2H), 2.49-2.72(m,8H),3.34(s,3H), 3.42(s,3H),3.80(t,2H),5.64(s,1H) | (Neat) 3241,2962, 1656,1445, 1062, 764 |
| 21 | ($CDCl_3$); 1.12(t,3H),1.72-1.83(m,2H), 2.50-2.75(m,6H), 3.34(s,3H),3.42 (s,3H),5.63(s,1H) | (Neat) 3398,3032, 2850,1704, 1648,1608, 1474 |
| 22 | ($CDCl_3$); 1.72-1.83(m,2H),2.57(t,2H), 2.71-2.80(m,4H),3.34(s,3H),3.42 (s,3H),3.49(t,2H),5.63(s,1H) | |
| 23 | ($CDCl_3$); 1.13(t,3H),2.60-2.80(m,4H), 2.92(t,2H),3.33(s,3H),3.44(s,3H), 5.63(s,1H) | (Neat) 3421,2995, 1698,1653, 1457,1163, 1124,1034, 1011 |
| 24 | ($CDCl_3$); 1.95-2.10(m,2H),2.56(t,2H), 3.35(s,3H),3.82(t,2H),5.63(s,1H), 7.75-7.95(m,4H) | |
| 25 | ($CDCl_3$); 1.65-1.80(m,2H),2.58(t,2H), 2.83(t,2H),3.33(s,3H),3.43(s,3H), 5.62(s,1H) | (Neat) 3366,2950, 1698,1652, 1447 |
| 26 | ($CDCl_3$); 1.90-2.00(m,2H),2.47(s,3H), 2.57(t,2H),3.32(s,3H),3.37(s,3H), 4.14(t,2H),5.49(s,1H),7.37(d,2H), 7.8(d,2H) | |

Table 7 (Cont'd)

| Example No. | NMR (Solvent);δppm | IR ($cm^{-1}$) |
|---|---|---|
| 27 | ($CDCl_3$);1.02(s,3H),1.05(s,3H), 1.65-1.80(m,2H),2.54(dd,2H), 2.61(dd,2H),2.85(dd,2H), 2.90-3.05(m,1H),3.33(s,3H), 3.37(s,3H),4.04(t,2H),5.62(s,1H), 6.92(d,2H),8.19(d,2H) | (Neat) 2966,1699, 1654,1508, 1457,1340 |
| 28 | ($CDCl_3$); 1.07(t,3H),1.77(m,2H), 2.53-2.69(m,6H),2.92(t,2H), 3.34(s,3H),3.40(s,3H),4.11(t,2H), 5.64(s,1H),6.94(d,2H),8.20(d,2H) | (Neat) 2961,1693, 1656,1591, 1339,1264, 1107, 857, 753 |
| 29 | ($CDCl_3$); 1.70-1.90(m,2H),2.36(s,3H), 2.50-2.60(m,4H),2.87(dd,2H),3.34 (s,3H),3.41(s,3H),4.16(t,2H),5.65 (s,1H),6.96(d,2H),8.21(d,2H) | (KBr) 2953,1692, 1651,1593, 1500,1336, 1259 |
| 30 | ($CDCl_3$);1.71-1.82(m,2H), 2.47-2.67(m,6H),2.92(t,2H), 3.33(s,3H),3.39(s,3H),4.11(t,2H), 5.64(s,1H),6.94(d,2H),8.19(d,2H) | (KBr) 3245,2960, 1698,1660, 1511,1341, 1263 |
| 31 | ($CDCl_3$); 1.02(t,3H),1.66-1.76(m,2H), 1.89-1.99(m,2H),2.45-2.68(m,8H), 3.33(s,3H),3.35(s,3H),4.11(t,2H), 5.59(s,1H),6.94(d,2H),8.19(d,2H) | (Neat) 2966,1702, 1660,1593, 1511,1341, 1264,1111, 753 |
| 32 | ($CDCl_3$); 1.09(t,3H),2.64-2.74(m,4H), 2.87(t,2H),2.97(t,2H),3.31(s,3H), 3.44(s,3H),4.12(t,2H),5.65(s,1H), 6.93(d,2H),8.20(d,2H) | (Neat) 3447,2969, 1702,1660, 1448,1051 |
| 33 | ($CDCl_3$); 2.42(s,3H),2.65-2.85(m,4H), 2.92(t,2H),3.32(s,3H),3.43(s,3H), 4.15(t,2H),5.68(s,1H),6.97(d,2H), 8.20(d,2H) | (Neat) 2953,1699, 1659,1511, 1340,1263 |

Table 7 (Cont'd)

| Example No. | NMR (Solvent);δppm | IR ($cm^{-1}$) |
|---|---|---|
| 34 | ($CDCl_3$); 1.04(s,3H),1.06(s,3H), 2.63(t,2H),2.82(t,2H),2.90(t,2H), 2.90-3.00(m,1H),3.28(s,3H), 3.43(s,3H),4.04(t,2H),5.63(s,1H), 6.91(d,2H),8.19(d,2H) | (Neat) 2965,1699, 1660,1521, 1442,1340, 1264 |
| 35 | ($CDCl_3$); 0.91(t,3H),1.46-1.55(m,2H), 2.53-2.59(m,2H),2.67(dd,2H), 2.87(dd,2H),2.97(t,2H),3.31(s,3H), 3.44(s,3H),4.11(t,2H),5.65(s,1H), 6.94(d,2H),8.19(d,2H) | (KBr) 3266,2961, 1701,1660, 1593,1509 |
| 36 | ($CDCl_3$); 1.79-1.87(m,2H),2.60(t,2H), 2.81(t,2H),3.06(t,2H),3.34(s,3H), 3.42(s,3H),4.17(t,2H),5.64(s,1H), 6.79(d,2H),8.21(d,2H) | (KBr)3307, 2952,2816, 1702,1667, 1504,1330, 1268 |
| 37 | ($CDCl_3$); 1.05(t,3H),1.69-1.79(m,2H), 2.51-2.66(m,6H),3.33(s,3H), 3.37(s,3H),3.99(t,2H),5.63(s,1H), 6.80(d,2H),7.22(d,2H) | (Neat) 2967,1701, 1655,1492, 1244, 826 763 |
| 38 | ($CDCl_3$); 1.05(t,3H),1.69-1.80(m,2H), 2.51-2.67(m,6H),2.87(t,2H), 3.34(s,3H),3.37(s,3H),4.0(t,2H), 5.63(s,1H),6.56-6.69(m,3H), 7.17-7.26(m,1H) | (Neat) 2967,1703, 1660,1616, 1448,1137, 762 |
| 39 | ($CDCl_3$); 1.05(t,3H),1.69-1.8(m,2H), 2.52-2.67(m,6H),2.86(t,2H), 3.33(s,3H),3.38(s,3H),3.98(t,2H), 5.63(s,1H),6.78-7.0(m,4H) | (Neat) 2966,1702, 1660,1507, 1444,1209, 829, 763 |
| 40 | ($CDCl_3$); 1.05(t,3H),1.70-1.81(m,2H), 2.54-2.68(m,6H),2.90(t,2H), 3.33(s,3H),3.37(s,3H),4.08(t,2H), 5.62(s,1H),6.87-7.09(m,4H) | (Neat) 2967,1702, 1660,1508, 1456,1260, 751 |

Table 7 (Cont'd)

| Example No. | NMR (Solvent);δppm | IR ($cm^{-1}$) |
|---|---|---|
| 41 | ($CDCl_3$); 1.70-1.90(m,2H),2.34(s,3H), 2.50-2.60(m,4H),2.81(t,2H), 3.33(s,3H),3.39(s,3H),4.04(t,2H), 5.63(s,1H),6.50-6.70(m,3H), 7.10-7.30(m,1H) | (KBr) 2995,1695, 1652,1483, 1288,1132, 1032 |
| 42 | ($CDCl_3$); 1.77(m,2H),2.34(s,3H), 2.53-2.59(m,4H),2.79(t,2H), 3.34(s,3H),3.39(s,3H),4.01(t,2H), 5.64(s,1H),6.80-6.85(m,2H), 6.94-7.0(m,2H) | (Neat) 2951,1702, 1661,1508, 1457,1209, 830 |
| 43 | ($CDCl_3$); 1.06(t,3H),1.71-1.81(m,2H), 2.52-2.67(m,6H),2.89(t,2H), 3.33(s,3H),3.39(s,3H),4.06(t,2H), 5.63(s,1H),6.93(d,2H),7.59(d,2H) | (Neat) 2967,2224, 1701,1656, 1605,1508, 1457,1260, 1173 |
| 44 | ($CDCl_3$); 1.06(t,3H),1.75(m,2H), 2.52-2.68(m,9H),2.90(t,2H), 3.33(s,3H),3.37(s,3H),4.09(t,2H), 5.63(s,1H),6.91(d,2H),7.93(d,2H) | (Neat) 2967,1702, 1660,1600, 1442,1256, 1173, 837, 763 |
| 45 | ($CDCl_3$); 1.06(t,3H),1.65-1.80(m,2H), 2.40-2.70(m,6H),2.63(s,3H), 2.93(t,2H),3.32(s,3H),3.34(s,3H), 4.12(t,2H),5.60(s,1H), 6.95-7.10(m,2H),7.40-7.50(m,1H), 7.65-7.75(m,1H) | (Neat) 2967,1703, 1669,1451, 1295,1242 |
| 46 | ($CDCl_3$); 1.75-1.81(m,2H),2.35(s,3H), 2.56(s,3H),2.54-2.59(m,4H), 2.83(t,2H),3.33(s,3H),3.40(s,3H), 4.12(t,2H),5.64(s,1H),6.92(d,2H), 7.93(d,2H) | (Neat) 2950,1702, 1660,1600, 1440,1256, 1174, 763 |

Table 7 (Cont'd)

| Example No. | NMR (Solvent); δppm | IR ($cm^{-1}$) |
|---|---|---|
| 47 | ($CDCl_3$); 0.90(t,3H),1.42-1.53(m,2H), 1.69-1.80(m,2H),2.47-2.58(m,4H), 2.56(s,3H),2.64(t,2H),2.89(t,2H), 3.33(s,3H),3.36(s,3H),4.08(t,2H), 5.62(s,1H),6.90(d,2H),7.93(d,2H) | (Neat) 2958,1702, 1660,1600, 1438,1359, 1255,1173 |
| 48 | ($CDCl_3$); 1.03(d,6H),1.68-1.76(m,2H), 2.51-2.62(m,4H),2.56(s,3H), 2.83(t,2H),2.94-2.99(m,1H), 3.33(s,6H),4.01(t,2H),5.61(s,1H), 6.89(d,2H),7.92(d,2H) | (Neat) 2965,1702, 1660,1600, 1440,1360, 1255,1172 |
| 49 | ($CDCl_3$); 1.06(t,3H),1.65-1.75(m,2H), 2.50-2.70(m,6H),2.89(t,2H), 3.33(s,3H),3.38(s,3H),4.06(t,2H), 5.63(s,1H),6.94(d,2H),7.53(d,2H) | (Neat) 2967,1661, 1520,1455, 1329,1259 |
| 50 | ($CDCl_3$); 1.06(t,3H),1.7-1.81(m,2H), 2.52-2.67(m,6H),2.87(t,2H), 3.33(s,3H),3.38(s,3H),4.0(t,2H), 5.63(s,1H),6.86(d,2H),7.14(d,2H) | (Neat) 2969,1704, 1663,1508, 1457,1245, 1199, 762 |
| 51 | ($CDCl_3$); 1.07(t,3H),1.71-1.82(m,2H), 2.53-2.69(m,6H),2.92(t,2H), 3.33(s,3H),3.39(s,3H),4.11(t,2H), 5.64(s,1H),6.94(d,2H), 7.45-7.51(m,2H),7.54-7.58(m,1H), 7.74-7.77(m,2H),7.82(d,2H) | (Neat) 2967,2824, 1702,1660, 1600,1446, 1256 |
| 52 | ($CDCl_3$); 1.07(t,3H),1.71-1.84(m,2H), 2.51-2.69(m,6H),2.90(t,2H), 3.33(s,3H),3.39(s,3H),4.05(t,2H), 5.64(s,1H),6.93-7.33(m,6H), 7.77(s,1H) | (Neat) 2967,1701, 1656,1521, 1457,1247, 1059, 834, 763 |
| 53 | ($CDCl_3$); 1.04(d,6H),1.66-1.78(m,2H), 2.53-2.64(m,4H),2.84(t,2H), 2.93-3.0(m,1H),3.33(s,3H), 3.36(s,3H),3.96(t,2H),5.63(s,1H), 6.94(d,2H),7.20(d,2H),7.29(d,2H), 7.77(s,1H) | (Neat) 2964,1699, 1654,1520, 1457,1247, 1059, 833, 762 |

Table 7 (Cont'd)

| Example No. | NMR (Solvent); δppm | IR ($cm^{-1}$) |
|---|---|---|
| 54 | ($CDCl_3$); 0.91(t,3H),1.45-1.53(m,2H), 1.70-1.79(m,2H),2.48-2.67(m,6H), 2.89(t,2H),3.33(s,3H),3.38(s,3H), 4.04(t,2H),5.63(s,1H),6.95(d,2H), 6.98-7.21(m,2H),7.30(d,2H), 7.77(s,1H) | (Neat) 2958,1701, 1659,1520, 1444,1246, 1058, 833, 736 |
| 55 | ($CDCl_3$); 1.77-1.85(m,2H),2.36(s,3H), 2.54-2.6(m,4H),2.83(t,2H), 3.33(s,3H),3.41(s,3H),4.09(t,2H), 5.65(s,1H),6.98(d,2H), 7.19-7.21(m,2H),7.3(d,2H),7.77(s,1H) | (Neat) 2951,1699, 1654,1521, 1245,1059, 834 |
| 56 | ($CDCl_3$); 1.10(t,3H),2.65-2.74(m,4H), 2.88(t,2H),2.95(t,2H),3.31(s,3H), 3.44(s,3H),4.06(t,2H),5.65(s,1H), 6.96(d,2H),7.20(d,2H),7.30(d,2H), 7.77(s,1H) | (Neat) 3114,2968, 1699,1659, 1521,1464, 1246,1059 |
| 57 | ($CDCl_3$); 1.05(t,3H),1.71-1.79(m,2H), 2.48-2.66(m,6H),2.61(s,3H), 2.92(t,2H),3.33(s,3H),3.37(s,3H), 4.09(t,2H),5.61(s,1H),6.90(d,1H), 7.37-7.41(m,1H),7.66(d,1H) | (KBr) 2950,2830, 1698,1656, 1621,1477, 1353,1279, 1220,1017 |
| 58 | ($CDCl_3$); 1.06(t,3H),1.69-1.79(m,2H), 2.49-2.67(m,6H),2.63(s,3H), 2.91(t,2H),3.33(s,3H),3.37(s,3H), 4.09(t,2H),5.61(s,1H), 6.89-6.94(m,1H),7.11-7.19(m,1H), 7.40-7.44(m,1H) | (Neat) 2967,2817, 1702,1661, 1491,1269, 1223,1183 |
| 59 | ($CDCl_3$); 1.06(t,3H),1.71-1.82(m,2H), 2.54-2.68(m,6H),2.56(s,3H), 2.94(t,2H),3.33(s,3H),3.39(s,3H), 4.15(t,2H),6.95-7.01(m,1H), 7.66-7.74(m,2H) | (Neat) 2967,2821, 1701,1655, 1612,1516, 1436,1280 |

Table 7 (Cont'd)

| Example No. | NMR (Solvent);δppm | IR ($cm^{-1}$) |
|---|---|---|
| 60 | ($CDCl_3$); 1.72-1.82(m,2H),2.33(s,3H), 2.50-2.57(m,4H),2.63(s,3H), 2.85(t,2H),3.34(s,3H),3.39(s,3H), 4.13(t,2H),5.62(s,1H), 6.89-6.94(m,1H),7.12-7.19(m,1H), 7.41-7.45(m,1H) | (KBr) 2963,1704, 1664,1500, 1468,1422, 1287,1182 |
| 61 | ($CDCl_3$); 1.08(t,3H),2.62(s,3H), 2.6-2.85(m,6H),2.95(t,2H), 3.31(s,3H),3.41(s,3H),4.08(t,2H), 5.62(s,1H),6.9(dd,1H), 7.11-7.19(m,1H),7.47(dd,1H) | (Neat) 2970,1702, 1661,1491, 1269,1224, 763 |
| 62 | ($CDCl_3$); 1.01(t,3H),1.64-1.76(m,2H), 1.95-2.02(m,2H),2.46-2.66(m,8H), 2.61(s,3H),3.34(s,3H),3.37(s,3H), 4.09(t,2H),5.59(s,1H),6.89(d,1H), 7.36-7.40(m,1H),7.67(d,1H) | (Neat) 2967,1702, 1662,1472, 1400,1273, 1222, 814 |
| 63 | ($CDCl_3$); 1.77-1.85(m,2H),2.59(t,2H), 2.79(t,2H),2.99(t,2H),3.34(s,3H), 3.42(s,3H),4.03(t,2H),5.63(s,1H), 6.82-6.86(m,2H),6.95-7.01(m,2H) | (Neat) 2935,1699, 1654,1508, 1457,1208 |
| 64 | ($CDCl_3$); 1.02(d,6H),1.66-1.72(m,2H), 2.51-2.63(m,4H),2.84-2.95(m,3H), 3.33(s,6H),3.83(s,3H),4.00(t,2H), 5.60(s,1H),6.88-6.91(m,4H) | (Neat) 2963,1701, 1655,1508, 1457,1253, 1226, 745 |
| 65 | ($CDCl_3$); 1.75-1.80(m,2H),2.34(s,6H), 2.53-2.59(m,4H),2.67-2.80(m,4H), 2.92(t,2H),3.33(s,3H),3.38(s,3H), 4.00(t,2H),5.62(s,1H),6.79-6.83 (m,2H),6.92-7.00(m,2H) | (Neat) 2947,1701, 1659,1507, 1458,1207, 1038, 763 |
| 67 | (DMSO-$d_6$);1.28(t,3H),1.99(m,2H), 2.66(t,2H),3.16(s,3H),3.33(s,3H), 3.60(m,4H),4.49(t,2H),5.67(s,1H), 7.17(d,2H),7.81(d,2H) | (KBr) 3393,1698, 1662,1510, 1228,1060, 807, 758 |

Table 7 (Cont'd)

| Example No. | NMR (Solvent); δppm | IR ($cm^{-1}$) |
|---|---|---|
| 68 | ($CDCl_3$); 1.85(t,2H),2.45(s,3H), 2.56-2.95(m,6H),3.33(s,3H), 3.40(s,3H),4.08(t,2H),5.61(s,1H), 6.63(d,2H),6.73(d,2H) | (Neat) 3382,2955, 1698,1508, 1473,1233 |
| 69 | ($CDCl_3$); 1.00(t,3H),1.75-1.90(m,2H), 2.05-2.20(m,2H),2.69(t,2H),3.02 (t,2H),3.19(t,2H),3.32(s,3H), 3.30-3.45(m,2H),3.41(s,3H), 4.33(t,2H),5.60(s,1H),6.64(d,2H), 6.71(d,2H) | (Neat) 3326,1969, 2621,1698, 1656,1522 |
| 70 | ($CDCl_3$); 1.05(t,3H),1.70-1.91(m,4H), 2.45-2.64(m,8H),3.31(s,3H),3.34 (s,3H),3.93(t,2H),5.57(s,1H), 6.60(d,2H),6.70(d,2H) | (Neat) 3354,2967, 1700,1656, 1236, 826, 763 |
| 71 | ($CDCl_3$); 1.13(t,3H),2.65-3.00(m,8H), 3.32(s,3H),3.42(s,3H),4.02(t,2H), 5.61(s,1H),6.63(d,2H),6.71(d,2H) | (Neat) 3354,2967, 1652,1506, 1236 |
| 72 | ($CDCl_3$); 2.41(s,3H),2.65-2.90(m,8H), 3.32(s,3H),3.41(s,3H),4.01(t,2H), 5.64(s,1H),6.63(d,2H),6.73(d,2H) | (Neat) 3354,2949, 1698,1652, 1509,1456, 1234 |
| 73 | ($CDCl_3$); 1.10(d,6H),2.65-3.00(m,6H), 3.00-3.20(m,1H),3.31(s,3H), 3.41(s,3H),3.90-4.05(m,2H), 5.60(s,1H),6.63(d,2H),6.70(d,2H) | (Neat) 3355,2966, 1698,1653, 1507,1457 |
| 74 | ($CDCl_3$); 0.97(t,3H),1.60-1.80(m,2H), 2.55-3.30(m,10H),3.31(s,3H), 3.45(s,3H),4.10-4.25(m,2H), 5.62(s,1H),6.65-6.75(m,4H) | (Neat) 3349,2964, 1699,1660, 1512,1471, 1230 |
| 75 | ($CDCl_3$); 1.76-1.84(m,2H),2.58(t,2H), 2.78(t,2H),2.96(t,2H),3.34(s,3H), 3.41(s,3H),4.00(t,2H),5.62(s,1H), 6.64(d,2H),6.75(d,2H) | (Neat) 3354,2930, 1698,1655, 1510,1457, 1236 |

Table 7 (Cont'd)

| Example No. | NMR (Solvent); δppm | IR ($cm^{-1}$) |
|---|---|---|
| 76 | ($CDCl_3$); 0.89(t,3H),1.45(q,2H), 1.55-1.75(m,2H),2.40-2.50(m,4H), 2.56(t,2H),2.70-2.90(m,4H), 3.33(s,3H),3.36(s,3H),5.57(s,1H), 7.35(d,2H),8.13(d,2H) | (Neat) 2959,1699, 1653,1521, 1457,1343 |
| 77 | ($CDCl_3$); 0.92(t,3H),1.25-1.44(m,4H), 1.64-1.72(m,2H),2.39-2.57(m,6H), 2.69-2.86(m,4H),3.34(s,3H), 3.35(s,3H),5.57(s,1H),7.34(d,2H), 8.14(d,2H) | (Neat) 2956,2811, 1702,1663, 1519,1457, 1345,1110 |
| 78 | ($CDCl_3$); 0.98(d,6H),1.55-1.64(m,2H), 2.36(t,2H),2.46(t,2H), 2.65-2.70(m,2H),2.77-2.90(m,2H), 2.92-3.02(m,1H),3.33(s,3H), 3.34(s,3H),5.54(s,1H),7.33(d,2H), 8.13(d,2H) | (Neat) 2946,1703, 1661,1518, 1457,1345 |
| 79 | ($CDCl_3$); 1.68-1.78(m,2H),2.31(s,3H), 2.40-2.51(m,4H),2.66(t,2H), 2.88(t,2H),3.33(s,3H),3.36(s,3H), 5.57(s,1H),7.36(d,2H),8.14(d,2H) | (Neat) 2955,2795, 1698,1655, 1514,1344 |
| 80 | ($CDCl_3$); 0.89(t,3H),1.41-1.50(m,2H), 1.64-1.72(m,2H),2.40-2.48(m,4H), 2.56(t,2H),2.70-2.76(m,2H), 2.82-2.86(m,2H),3.34(s,6H), 5.56(s,1H),7.42-7.54(m,2H), 8.04-8.08(m,2H) | (Neat) 2959,1703, 1660,1528, 1443,1351 |
| 81 | ($CDCl_3$); 0.88(t,3H),1.37-1.52(m,2H), 1.60-1.72(m,2H),2.56(t,2H), 2.74(dd,2H),3.02(dd,2H),3.35(s,3H), 3.39(s,3H),5.57(s,1H), 7.20-7.90(m,4H) | (Neat) 2959,1703 1660,1526 1444,1350 |
| 82 | ($CDCl_3$); 1.06(t,3H),2.53-2.69(m,4H), 2.74-2.89(m,6H),3.33(s,3H), 3.38(s,3H),5.59(s,1H),7.34(d,2H), 8.15(d,2H) | (Neat) 2947,2597 1700,1659 1522,1474 1348 |

Table 7 (Cont'd)

| Example No. | NMR (Solvent);δppm | IR ($cm^{-1}$) |
|---|---|---|
| 83 | ($CDCl_3$); 1.67-1.78(m,2H), 2.57(t,2H), 2.69-2.92(m,6H), 3.33(s,3H),3.38(s,3H), 7.19-7.34(m,5H) | (KBr) 3300,2914, 2821,2775, 1698,1656, 1623,1451, 1377,1131 |
| 84 | ($CDCl_3$); 1.03(t,3H),1.62-1.67(m,2H), 2.42(t,2H),2.53-2.64(m,4H), 2.70-2.86(m,4H),3.34(s,3H), 3.35(s,3H),5.57(s,1H),7.34(d,2H), 8.14(d,2H) | (Neat) 2960,1706, 1660,1619, 1514,1343 |
| 85 | ($CDCl_3$); 1.72-1.80(m,2H),2.54(t,2H), 2.73(t,2H),2.91(s,4H),3.34(s,3H), 3.40(s,3H),5.60(s,1H),7.37(d,2H), 8.17(d,2H) | (KBr) 3315,2949, 2831,1698, 1655,1510, 1343,1109 |
| 86 | ($CDCl_3$); 0.89(t,3H),1.19-1.27(m,6H), 1.38-1.52(m,2H),1.64-1.75(m,2H), 2.40-2.48(m,4H),2.56(t,2H), 2.70-2.76(m,2H),2.81-2.87(m,2H), 3.82-3.89(m,2H),3.96-4.03(m,2H), 5.55(s,1H),7.34(d,2H),8.15(d,2H) | (Neat) 2962,1698, 1661,1519, 1458,1345 |
| 87 | ($CDCl_3$); 0.89(t,3H),1.44(s,3H), 1.46(s,3H),1.52(s,3H),1.54(s,3H), 1.57-1.70(m,4H),2.37-2.48(m,4H), 2.56(t,2H),2.71-2.77(m,2H), 2.82-2.87(m,2H),4.10-4.26(m,1H), 5.12-5.19(m,1H),5.44(s,1H), 7.35(d,2H),8.15(d,2H) | (Neat) 2965,1700, 1662,1519, 1438,1345 |
| 88 | ($CDCl_3$); 1.65-1.71(m,2H), 2.41-2.46(m,2H),2.62(t,2H), 2.73(t,2H),2.80-2.84(m,4H), 3.34(s,6H),3.36(s,3H),3.44(t,2H), 5.57(s,1H),7.35(d,2H),8.14(d,2H) | (Neat) 2932,1702, 1660,1518, 1457,1345, 1111, 763 |

Table 7 (Cont'd)

| Example No. | NMR (Solvent); δppm | IR ($cm^{-1}$) |
|---|---|---|
| 89 | ($CDCl_3$); 1.67-1.75(m,2H),2.28(s,6H), 2.37-2.47(m,4H),2.58-2.70(m,4H), 2.75-2.88(m,4H),3.34(s,3H), 3.36(s,3H),5.57(s,1H),7.35(d,2H), 8.15(d,2H) | (Neat) 2947,2819, 1702,1660, 1518,1457, 1345, 763 |
| 90 | ($CDCl_3$); 1.03(t,3H),1.75-1.90(m,2H), 2.40-2.85(m,10H),3.32(s,3H), 3.42(s,3H),5.63(s,1H),7.34(d,2H), 8.13(d,2H) | (Neat) 2967,1707, 1660,1518, 1446,1345, 1111 |
| 91 | ($CDCl_3$); 1.00(t,3H),1.63-1.83(m,4H), 2.44-2.56(m,8H),2.74(t,2H), 3.33(s,3H),3.41(s,3H),5.62(s,1H), 7.34(d,2H),8.14(d,2H) | (Neat) 2934,1699, 1653,1521, 1457,1343, 1345, 764 |
| 92 | ($CDCl_3$); 0.89(t,3H),1.38-1.46(m,2H), 1.65-1.83(m,4H),2.35-2.54(m,8H), 2.73(t,2H),3.34(s,3H),3.41(s,3H), 5.62(s,1H),7.33(d,2H),8.15(d,2H) | (Neat) 2957,2807, 1703,1662, 1518,1457, 1345 |
| 93 | ($CDCl_3$); 0.99(t,3H),1.68-1.78(m,4H), 2.41-2.61(m,10H),3.34(s,3H), 3.39(s,3H), 5.62(s,1H),7.10(d,2H), 7.24(d,2H) | (Neat) 2945,2674, 1698,1655, 1474,1087 |
| 94 | ($CDCl_3$); 0.99(t,3H),1.66-1.81(m,4H), 2.42-2.64(m,10H),3.34(s,3H), 3.39(s,3H),5.62(1H,s), 6.85-6.96(m,3H),7.19-7.28(m,1H) | (Neat) 2932,2681, 1693,1658, 1616,1245 |
| 95 | ($CDCl_3$); 0.99(t,3H),1.63-1.78(m,4H), 2.41-2.58(m,10H),3.34(s,3H), 3.39(s,3H),3.79(s,3H),5.62(s,1H), 6.83(d,2H),7.09(d,2H) | (Neat) 2951,2681, 1698,1655, 1246,1032 |
| 96 | ($CDCl_3$); 0.99(t,3H),1.68-1.78(m,4H), 2.42-2.56(m,8H),2.68(t,2H), 3.34(s,3H),3.41(s,3H), 5.62(s,1H), 7.29(d,2H),7.58(d,2H) | (Neat) 2964,2811, 2227,1703, 1660,1620, 1445 |

Table 7 (Cont'd)

| Example No. | NMR (Solvent);δppm | IR ($cm^{-1}$) |
|---|---|---|
| 97 | ($CDCl_3$); 0.99(t,3H),1.62-1.81(m,4H), 2.43-2.56(m,8H),2.61(t,2H), 3.34(s,3H),3.38(s,3H),5.61(s,1H), 7.16-7.31(m,5H) | (Neat) 2944,1689, 1652,1612, 1474 |
| 98 | ($CDCl_3$); 1.02(t,3H),1.65-1.80(m,2H), 2.40-2.80(m,10H),3.33(s,3H), 3.40(s,3H),5.61(s,1H),7.10(d,2H), 7.24(d,2H) | (Neat) 2967,1703, 1661,1445 |
| 99 | ($CDCl_3$); 1.03(t,3H),1.70-1.82(m,2H), 2.44-2.73(m,10H),3.33(s,3H), 3.40(s,3H),5.61(s,1H), 6.85-6.96(m,3H),7.18-7.27(m,1H) | (Neat) 2967,1703, 1664,1448 |
| 100 | ($CDCl_3$); 1.01(t,3H),1.75-1.95(m,2H), 2.05-2.25(m,2H),2.60-2.75(m,2H), 2.90-3.30(m,8H),3.36(s,3H), 3.43(s,3H),5.64(s,1H),6.63(d,2H), 6.99(d,2H) | (KBr) 3421,2943, 1655,1519, 1473 |
| 101 | ($CDCl_3$); 0.93(t,3H),1.25-1.50(m,4H), 1.68-1.73(m,2H),2.40-2.75(m,8H), 3.34(s,3H),3.35(s,3H),5.55(s,3H), 6.61(d,2H),6.96(d,2H) | (Neat) 3356,2955, 2805,1701, 1659,1518, 1457 |
| 102 | ($CDCl_3$); 1.22(d,6H),1.85-2.00(m,2H), 2.53-2.56(m,2H),2.77-2.95(m,3H), 3.33(s,3H),3.40(s,3H),5.54(s,1H), 6.61(d,2H),6.99(d,2H) | (Neat) 3355,2963, 1698,1653, 1519,1447, 1374,1280, 1177 |
| 103 | ($CDCl_3$); 1.66-1.78(m,2H),2.32(s,3H), 2.40-2.70(m,8H),3.34(s,3H), 3.35(s,3H),5.57(s,1H),6.61(d,2H), 6.97(d,2H) | (Neat) 3355,2948, 1652,1518, 1456, 824 |

Table 7 (Cont'd)

| Example No. | NMR (Solvent); δppm | IR ($cm^{-1}$) |
|---|---|---|
| 104 | ($CDCl_3$); 0.94(t,3H),1.58-1.66(m,2H), 1.83-1.88(m,2H),2.51(t,2H), 2.64-2.87(m,8H),3.34(s,3H), 3.38(s,3H),5.57(s,1H), 6.51-6.58(m,3H),7.03-7.09(m,1H) | (Neat) 3356,2957, 1699,1656, 1459,1374 |
| 105 | ($CDCl_3$); 1.00(t,3H),1.67-1.78(m,2H), 1.98-2.04(m,2H),2.63(t,2H), 2.84-3.10(m,8H),3.33(s,3H), 3.41(s,3H),5.57(s,1H), 6.64-6.70(m,2H),6.94-7.10(m,2H) | (Neat) 3355,2958, 1698,1652, 1457,1031 |
| 106 | ($CDCl_3$); 1.10(t,3H), 2.66-2.80(m,10H),3.33(s,3H), 3.39(s,3H),5.57(s,1H),6.63(d,2H), 6.97(d,2H) | (Neat) 3356,2967, 2817,1698, 1653,1518, 1457,1372 |
| 107 | ($CDCl_3$); 1.06(t,3H),1.62-1.70(m,2H), 2.41(t,2H), 2.53-2.65(m,8H), 3.33(s,3H),3.34(s,3H),5.56(s,1H), 6.61(d,2H),6.96(d,2H) | (Neat) 3442,3355, 3231,2964, 2808,1699, 1656,1518, 1440 |
| 108 | ($CDCl_3$); 1.67-1.77(m,2H),2.51(t,2H), 2.67-2.72(m,4H),2.82(t,2H), 3.34(s,3H),3.39(s,3H),5.59(s,1H), 6.64(d,2H),6.99(d,2H) | (KBr) 3397,2920, 2827,1698, 1652,1519, 1473,1122 |
| 109 | ($CDCl_3$); 1.03(t,3H),1.16-1.31(m,6H), 1.85-1.90(m,2H),2.09-2.34(m,2H), 2.64-2.69(m,2H),2.82-3.21(m,8H), 3.90-4.03(m,4H),5.50(s,1H), 6.65(d,2H),7.01(d,2H) | (KBr) 3422,2977, 2618,1695, 1652,1519, 1465 |
| 110 | ($CDCl_3$); 1.01(t,3H),1.43(s,3H), 1.46(s,3H),1.55(s,3H),1.57(s,3H), 1.79-1.88(m,2H),2.02-2.21(m,2H), 2.54-2.60(m,2H),2.96-3.48(m,8H), 4.23-4.27(m,1H),5.08-5.18(m,1H), 5.41(s,1H),6.64(d,2H),6.91(d,2H) | (Neat) 3355,2971, 2594,1698, 1652,1519, 1444,1379 |

Table 7 (Cont'd)

| Example No. | NMR (Solvent);δppm | IR ($cm^{-1}$) |
|---|---|---|
| 111 | ($CDCl_3$); 1.59-1.70(m,2H), 2.35-2.43(m,2H),2.53-2.73(m,8H), 3.34(s,6H),3.35(s,3H),3.45(t,2H), 5.56(s,1H),6.60(d,2H),6.96(d,2H) | (Neat) 3357,2927, 1699,1655, 1518,1457, 1114, 733 |
| 112 | ($CDCl_3$); 1.61-1.71(m,2H),2.24(s,6H), 2.34-2.58(m,2H),2.60-2.68(m,10H), 3.34(s,6H),5.55(s,1H),6.60(d,2H), 6.95(d,2H) | (Neat) 3362,2948, 1699,1655, 1518,1457, 763 |
| 113 | ($CDCl_3$); 1.30-1.40(t,3H), 2.50-3.20(m,10H),3.33(s,3H), 3.47(s,3H),5.44(s,1H),6.64(d,2H), 6.96(d,2H) | (Neat) 3351,2947, 2571,1669, 1471,1286, 1030 |
| 114 | (DMSO-$d_6$); 1.15(t,3H), 1.70-1.93(m,4H),2.45-2.78(m,10H), 3.61(s,3H),3.75(s,3H),5.63(s,1H), 6.51(d,2H),6.87(d,2H) | |
| 115 | ($CDCl_3$); 0.87(t,3H),1.38-1.46(m,2H), 1.59-1.75(m,4H),2.33-2.53(m,10H), 3.34(s,3H),3.38(s,3H),5.60(s,1H), 6.62(d,2H),6.96(d,2H) | (Neat) 3358,2956, 2811,1701, 1659,1518, 1443 |
| 116 | ($CDCl_3$); 1.01(s,3H),1.04(s,3H), 1.60-1.75(m,2H),1.82(bs,1H), 2.50-2.65(m,4H),2.81(t,2H), 2.90-3.00(m,1H),2.96(s,3H), 3.33(s,6H),3.94(d,2H),5.59(s,1H), 6.83(d,2H),7.20(d,2H) | (Neat) 3228,2965, 1699,1653, 1508,1457, 1333,1155 |
| 117 | ($CDCl_3$); 1.06(t,3H),1.68-1.78(m,2H), 2.51-2.67(m,6H),2.86(t,2H), 2.95(s,3H),3.33(s,3H),3.37(s,3H), 4.01(t,2H),5.6(s,1H,),6.86(d,2H), 7.2(d,2H) | (Neat) 2966,1699, 1654,1509, 1155, 764 |

Table 7 (Cont'd)

| Example No. | NMR (Solvent);δppm | IR ($cm^{-1}$) |
|---|---|---|
| 118 | ($CDCl_3$); 0.90(t,3H),1.40-1.60(m,4H), 1.73(bs,1H),2.30-2.60(m,6H), 2.68(bs,4H),2.99(s,3H),3.34(s,6H), 5.42(s,1H),7.17(s,4H) | (Neat) 3229,2958, 1696,1652, 1512,1471, 1337,1155 |
| 119 | ($CDCl_3$); 1.68-1.78(m,2H),2.54(t,2H), 2.71(t,2H), 2.75-2.89(m,4H), 3.01(s,3H),3.34(s,3H),3.39(s,3H), 5.55(s,3H),7.15-7.22(m,4H) | (KBr) 3448,2930, 1702,1654, 1508,1474, 1246,1153 |
| 120 | ($CDCl_3$);1.72-1.84(m,2H),2.36(s,3H), 2.53-2.61(m,4H),2.82(t,2H), 2.94(s,3H),3.33(s,3H),3.39(s,3H), 4.05(t,2H),5.63(s,1H),6.84(d,2H), 7.29(d,2H) | (Neat) 3274,2977, 1700,1655, 1155 |
| 121 | ($CDCl_3$); 0.90(t,3H),1.43-1.53(m,2H), 1.66-1.78(m,2H),2.45-2.65(m,6H), 2.86(t,2H),2.95(s,3H),3.33(s,3H), 3.36(s,3H),4.00(t,2H),5.61(s,1H), 6.85(d,2H),7.20(d,2H) | (Neat) 3231,2959, 1699,1652, 1509,1471, 1332,1155 |
| 122 | ($CDCl_3$); 1.01(t,3H),1.6-1.7(m,2H), 1.84-1.93(m,2H),2.41-2.7(m,8H), 2.96(s,3H),3.29(s,3H),3.34(s,3H), 4.01(t,2H),5.48(s,1H),6.85(d,2H), 7.17(d,2H) | (Neat) 2965,1699, 1653,1509, 1473,1331, 1155, 974, 763 |
| 123 | ($CDCl_3$); 1.09(t,3H),2.64-2.73(m,4H), 2.80-3.00(m,4H),2.94(s,3H), 3.30(s,3H),3.32(s,3H),4.01(t,2H), 5.66(s,1H),6.85(d,2H),7.23(d,2H), 7.47(bs,1H) | (Neat) 3230,2970, 1698,1653, 1508,1457, 1331,1155 |
| 124 | ($CDCl_3$); 2.41(s,3H),2.65-2.90(m,6H), 2.95(s,3H),3.32(s,3H),3.42(s,3H), 4.05(t,2H),5.69(s,1H),6.87(d,2H), 7.02(bs,1H),7.21(d,2H) | (Neat) 3219,2950, 1652,1508, 1456,1331, 1246,1155 |

Table 7 (Cont'd)

| Example No. | NMR (Solvent); δppm | IR ($cm^{-1}$) |
|---|---|---|
| 125 | ($CDCl_3$); 1.03(s,3H),1.05(s,3H), 2.62(t,2H),2.78-2.90(m,4H), 2.96(s,3H),2.90-3.00(m,1H), 3.29(s,3H),3.41(s,3H),3.93(t,2H), 5.64(s,1H),6.83(d,2H),7.21(d,2H) | (Neat) 3220,2966, 1698,1653, 1508,1457, 1332,1155 |
| 126 | ($CDCl_3$); 0.90(t,3H),1.42-1.55(m,2H), 2.54(dd,2H),2.66(dd,2H), 2.84-2.95(m,4H),2.95(s,3H), 3.31(s,3H),3.42(s,3H),4.00(t,2H), 5.65(s,1H),6.85(d,2H),7.21(d,2H) | (Neat) 3228,2960, 1698,1652, 1508,1457, 1332,1155 |
| 127 | ($CDCl_3$); 1.75-1.85(m,2H),2.59(t,2H), 2.78(t,2H),2.94(s,3H),3.00(t,2H), 3.33(s,3H),3.41(s,3H),5.63(s,1H), 6.86(d,2H),7.20(d,2H) | (Neat) 3587,2933, 1698,1653, 1509,1457, 1328,1153 |
| 128 | ($CDCl_3$); 0.93(t,3Hz), 1.27-1.45(m,4H),1.54-1.59(m,2H), 2.37(t,2H),2.45-2.51(m,4H), 2.67(s,4H),2.99(s,3H),3.39(s,6H), 5.43(s,1H),7.17(s,4H) | (Neat) 3228,2955, 2807,1701, 1653,1512, 1465,1337, 1224,1155 |
| 129 | ($CDCl_3$); 0.99(d,6H),1.35-1.43(m,2H), 1.65-1.80(m,2H),2.26-2.32(m,2H), 2.41(t,2H),2.64(s,4H), 2.95-3.05(m,1H),2.99(s,3H), 3.33(s,3H),3.34(s,3H),5.31(s,1H), 7.17(s,3H) | (Neat) 3230,2964, 1699,1653, 1511,1458, 1337,1155 |
| 130 | ($CDCl_3$); 1.60-1.73(m,2H),2.30(s,3H), 2.38-2.77(m,8H),2.99(s,3H), 3.34(s,3H),3.35(s,3H),5.49(s,1H), 7.17(s,4H) | (Neat) 3216,2949, 2797,1699, 1653,1511, 1457,1337, 1155 |

Table 7 (Cont'd)

| Example No. | NMR (Solvent);δppm | IR ($cm^{-1}$) |
|---|---|---|
| 131 | ($CDCl_3$); 0.89(t,2H),1.41-1.50(m,2H), 1.61-1.66(m,2H),2.37-2.54(m,6H), 2.70(s,4H),3.00(s,3H),3.35(s,6H), 5.54(s,1H),6.98-7.11(m,3H), 7.22-7.27(m,1H) | (Neat) 3219,2958, 1701,1655, 1471,1334, 1152 |
| 132 | ($CDCl_3$); 1.06(t,3H), 2.53-2.77(m,10H),3.00(s,3H), 3.34(s,3H),3.38(s,3H),5.56(s,1H), 7.16(s,4H) | (Neat) 3219,2968, 2817,1698, 1652,1512, 1472,1336, 1155 |
| 133 | ($CDCl_3$); 1.04(t,3H),1.55-1.63(m,2H), 2.38(t,2H),2.50(t,2H),2.59(q,2H), 2.68(s,4H),2.99(s,3H),3.34(s,3H), 3.35(s,3H),5.44(s,1H),7.17(s,4H) | (Neat) 3221,2965, 1701,1656, 1616,1512, 1468,1336, 1155 |
| 134 | ($CDCl_3$); 0.90(t,3H),1.19-1.27(m,6H), 1.42-1.50(m,6H),1.56-1.62(m,2H), 2.36-2.53(m,6H),2.68(bs,4H), 2.99(s,3H),3.81-3.89(m,2H), 3.95-4.03(m,2H),5.39(s,1H), 7.18(s,4H) | (Neat) 3230,2960, 1698,1652, 1512,1464, 1339,1155 |
| 135 | ($CDCl_3$); 0.90(t,3H),1.43(s,3H), 1.46(s,3H),1.39-1.60(m,2H), 1.51(s,3H),1.53(s,3H), 1.66-1.82(m,2H),2.30-2.36(m,2H), 2.42-2.51(m,4H),2.64-2.72(m,4H), 3.00(s,3H),4.10-4.23(m,1H), 5.10-5.19(m,1H),5.23(s,1H), 7.18(s,4H) | (Neat) 3246,2965, 1699,1652, 1512,1443, 1378,1338, 1156 |
| 136 | ($CDCl_3$); 1.55-1.61(m,2H),2.25(s,6H), 2.34-2.41(m,5H),2.49-2.78(m,8H), 2.98(s,3H),3.34(s,3H),3.35(s,3H), 5.41(s,,1H),7.17(s,4H) | (Neat) 2945,1699, 1654,1457, 1338,1154, 763 |

Table 7 (Cont'd)

| Example No. | NMR (Solvent);δppm | IR ($cm^{-1}$) |
|---|---|---|
| 137 | ($CDCl_3$); 1.02(t,3H),1.68-1.81(m,2H), 2.40-2.75(m,10H),2.98(s,3H), 3.33(s,3H),3.41(s,3H),5.62(s,1H), 7.10-7.23(m,4H) | (Neat) 3274,2977, 1700,1655, 1155 |
| 138 | ($CDCl_3$); 1.00(t,3H),1.56-1.77(m,4H), 2.39-2.63(m,10H),2.99(s,3H), 3.35(s,3H),3.38(s,3H), 5.50(s,1H), 7.16(s,4H) | (Neat) 2935,1699, 1655,1468, 1332,1223, 1154 |
| 139 | ($CDCl_3$); 0.89(t,3H),1.38-1.50(m,2H), 1.55-1.68(m,2H),1.71-1.79(m,2H), 2.34-2.48(m,8H),2.60(t,2H), 2.99(s,3H),3.35(s,3H),3.38(s,3H), 5.50(s,1H),7.17(s,4H) | (Neat) 3228,2956, 1702,1659, 1512,1467, 1335,1155 |
| 140 | ($CDCl_3$); 0.89(t,3H),1.36(t,3H), 1.46(q,2H),1.52-1.67(m,2H), 2.33-2.54(m,6H),2.67(s,4H), 3.09(q,2H),3.34(s,3H),3.35(s,3H), 5.53(s,1H),7.13(d,2H),7.20(d,2H) | (Neat) 3217,2958, 1699,1652, 1512,1456, 1336,1149 |
| 141 | ($CDCl_3$); 0.87(t,3H),1.35-1.57(m,4H), 2.30-2.48(m,6H),2.62(s,4H), 3.32(s,3H),3.34(s,3H),5.43(s,1H), 6.80-7.78(m,9H) | (Neat) 3185,2958, 1653,1508, 1457,1340, 1163 |

## Claims

1. A pyrimidinedione derivative of formula (1)

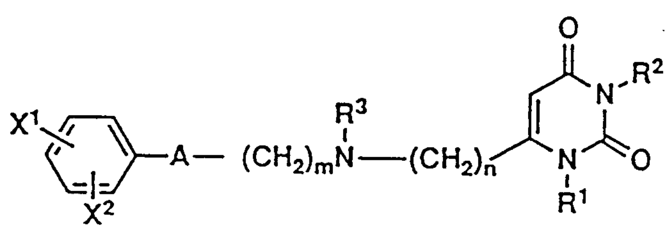

(1)

wherein

$R^1$ and $R^2$ each independently represent a hydrogen atom or an alkyl group of 1 to 6 carbon atoms;
$R^3$ represents a hydrogen atom, an alkyl group of 1 to 6 carbon atoms, an alkyl group of 1 to 4 carbon atoms which is substituted by a dialkylamino group of 2 to 12 carbon atoms, or an alkyl group of 1 to 4 carbon atoms

which is substituted by an alkyloxy group of 1 to 6 carbon atoms;
A represents a methylene group or an oxygen atom;
$X^1$ and $X^2$ each independently represent a hydrogen atom, an alkanoyl group of 2 to 6 carbon atoms, a benzoyl group, an alkanoylamino group of 2 to 6 carbon atoms, an amino group, a nitro group, an alkylsulfonylamino group of 1 to 6 carbon atoms, a benzenesulfonylamino group, a cyano group, a fluorine atom, a chlorine atom, a trifluoromethyl group, an imidazole group, an alkyloxy group of 1 to 6 carbon atoms, a trifluoromethoxy group, or an alkylthio group of 1 to 6 carbon atoms;
m represents an integer of 1 to 4; and
n represents an integer of 2 to 4;
or a pharmaceutically acceptable salt thereof

**2.** A pyrimidinedione derivative or a pharmaceutically acceptable salt thereof as claimed in claim 1 wherein $X^1$ and $X^2$ each independently represent a hydrogen atom, an alkanoyl group of 2 to 6 carbon atoms, a benzoyl group, an amino group, a nitro group, an alkylsulfonylamino group of 1 to 6 carbon atoms, a benzenesulfonylamino group, a cyano group, a fluorine atom, a chlorine atom, a trifluoromethyl group, an imidazole group, an alkyloxy group of 1 to 6 carbon atoms, or a trifluoromethoxy group.

**3.** A pyrimidinedione derivative or a pharmaceutically acceptable salt thereof as claimed in claim 1 or 2 wherein $R^3$ represents a hydrogen atom or an alkyl group of 1 to 4 carbon atoms.

**4.** A pyrimidinedione derivative or a pharmaceutically acceptable salt thereof as claimed in claim 1, 2 or 3 wherein $R^1$ and $R^2$ each independently represent an alkyl group of 1 to 3 carbon atoms.

**5.** A pyrimidinedione derivative or a pharmaceutically acceptable salt thereof as claimed in claim 4 wherein $X^1$ and $X^2$ each independently represent a hydrogen atom, an alkanoyl group of 2 to 6 carbon atoms, an amino group, a nitro group, an alkylsulfonylamino group of 1 to 6 carbon atoms, a fluorine atom, a chlorine atom or an imidazole group, and $R^3$ represents a hydrogen atom or an alkyl group of 1 to 4 carbon atoms.

**6.** A process for producing a pyrimidinedione derivative of formula (1) as claimed in any preceding claim which comprises reacting a benzene derivative of formula (2)

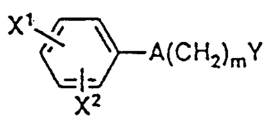

(2)

wherein Y represents a chlorine atom, a bromine atom, an iodine atom, a mesyloxy group or a tosyloxy group, and $X^1$, $X^2$, A and m are as defined in claim 1, with a pyrimidinedione derivative of formula (3)

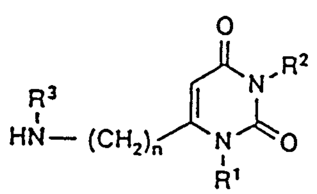

(3)

wherein $R^1$, $R^2$, $R^3$ and n are as defined in claim 1.

**7.** A process for producing a pyrimidinedione derivative of formula (1) as claimed in any of claims 1-5 which comprises reacting an alkylamine derivative of formula (4)

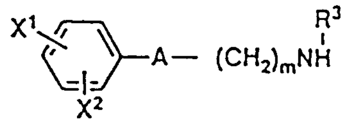

(4)

wherein $X^1$, $X^2$, A, $R^3$ and m are as defined in claim 1, with a pyrimidinedione derivative of formula (5)

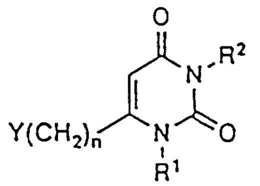

(5)

wherein Y represents a chlorine atom, a bromine atom, an iodine atom, a mesyloxy group or a tosyloxy group, and $R^1$, $R^2$ and n are as defined in claim 1.

8. A process for producing a pyrimidinedione derivative of formula (1) (where A represents an oxygen atom) as claimed in any of claims 1-5 which comprises reacting a phenol derivative of formula (6).

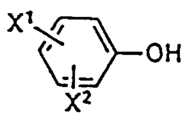

(6)

wherein $X^1$ and $X^2$ are as defined in claim 1, with a pyrimidinedione derivative of formula (7)

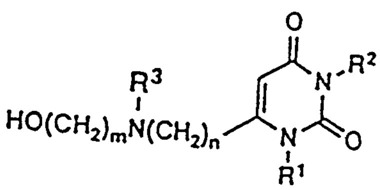

(7)

wherein $R^1$, $R^2$, $R^3$, m and n are as defined in claim 1.

9. An antiarrhythmic composition comprising (a) as active ingredient, a pyrimidinedione derivative of formula (1) or a pharmaceutically acceptable salt thereof as claimed in any of claims 1-5 and (b) a pharmaceutically acceptance carrier or diluent.

10. A pyrimidinedione derivative of formula (8)

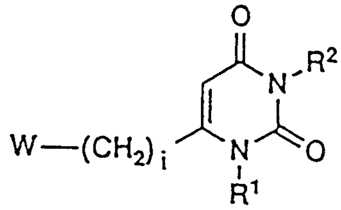

(8)

wherein $R^1$ and $R^2$ are as defined in claim 1 or claim 4; W represents a hydroxyl group, a chlorine atom, a bromine atom, an iodine atom, a mesyloxy group, a tosyloxy group, a benzyloxy group, a phthaloylamino group, an alkyloxycarbonyl group of 2 to 7 carbon atoms, a vinyl group, $-NHR^3$ or $-NR^3(CH_2)_mOH$ (wherein $R^3$ and m are as defined

in claim 1); and i represents an integer of 1 to 3 when W is an alkyloxycarbonyl group of 2 to 7 carbon atoms, an integer of 0 or 2 when W is a vinyl group, or an integer of 2 to 4 in other cases.

11. Use of a pyrimidinedione derivative of formula (1) or a pharmaceutically acceptable salt thereof as claimed in any of claims 1-5 for the preparation of antiarrhythmic agents.

**Patentansprüche**

1. Pyrimidindionderivat der Formel (1)

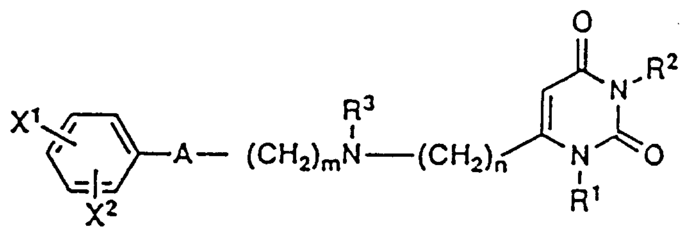

(1)

worin

R¹ und R² jeweils unabhängig voneinander ein Wasserstoffatom oder eine Alkylgruppe mit 1 bis 6 Kohlenstoffatomen sind; $R^3$ ein Wasserstoffatom, eine Alkylgruppe mit 1 bis 6 Kohlenstoffatomen, eine Alkylgruppe mit 1 bis 4 Kohlenstoffatomen, welche mit einer Dialkylaminogruppe mit 2 bis 12 Kohlenstoffatomen substituiert ist, oder eine Alkylgruppe mit 1 bis 4 Kohlenstoffatomen, welche mit einer Alkyloxygruppe mit 1 bis 6 Kohlenstoffatomen substituiert ist, ist;
A eine Methylengruppe oder ein Sauerstoffatom ist;
$X^1$ und $X^2$ jeweils unabhängig voneinander ein Wasserstoffatom, eine Alkanoylgruppe mit 2 bis 6 Kohlenstoffatomen, eine Benzoylgruppe, eine Alkanoylaminogruppe mit 2 bis 6 Kohlenstoffatomen, eine Aminogruppe, eine Nitrogruppe, eine Alkylsulfonylaminogruppe mit 1 bis 6 Kohlenstoffatomen, eine Benzolsulfonylaminogruppe, eine Cyanogruppe, ein Fluoratom, ein Chloratom, eine Trifluormethylgruppe, eine Imidazolgruppe, eine Alkyloxygruppe mit 1 bis 6 Kohlenstoffatomen, eine Trifluormethoxygruppe oder eine Alkylthiogruppe mit 1 bis 6 Kohlenstoffatomen sind;
m eine ganze Zahl von 1 bis 4 ist; und n eine ganze Zahl von 2 bis 4 ist; oder ein pharmazeutisch geeignetes Salz davon.

2. Pyrimidindionderivat oder ein pharmazeutisch geeignetes Salz davon gemäß Anspruch 1, worin $X^1$ und $X^2$ jeweils unabhängig voneinander ein Wasserstoffatom, eine Alkanoylgruppe mit 2 bis 6 Kohlenstoffatomen, eine Benzoylgruppe, eine Aminogruppe, eine Nitrogruppe, eine Alkylsulfonylaminogruppe mit 1 bis 6 Kohlenstoffatomen, eine Benzolsulfonylaminogruppe, eine Cyanogruppe, ein Fluoratom, ein Chloratom, eine Trifluormethylgruppe, eine Imidazolgruppe, eine Alkyloxygruppe mit 1 bis 6 Kohlenstoffatomen oder eine Trifluormethoxygruppe sind.

3. Pyrimidindionderivat oder ein pharmazeutisch geeignetes Salz davon gemäß Anspruch 1 oder 2, worin $R^3$ ein Wasserstoffatom oder eine Alkylgruppe mit 1 bis 4 Kohlenstoffatomen ist.

4. Pyrimidindionderivat oder ein pharmazeutisch geeignetes Salz davon gemäß Anspruch 1,2 oder 3, worin $R^1$ und $R^2$ jeweils unabhängig voneinander eine Alkylgruppe mit 1 bis 3 Kohlenstoffatomen sind.

5. Pyrimidindionderivat oder ein pharmazeutisch geeignetes Salz davon gemäß Anspruch 4, worin $X^1$ und $X^2$ jeweils unabhängig voneinander ein Wasserstoffatom, eine Alkanoylgruppe mit 2 bis 6 Kohlenstoffatomen, eine Aminogruppe, eine Nitrogruppe, eine Alkylsulfonylaminogruppe mit 1 bis 6 Kohlenstoffatomen, ein Fluoratom, ein Chloratom oder eine Imidazolgruppe sind, und $R^3$ ein Wasserstoffatom oder eine Alkylgruppe mit 1 bis 4 Kohlenstoffatomen ist.

6. Verfahren zur Herstellung eines Pyrimidindionderivats der Formel (1) gemäß einem der voranstehenden Ansprüche, welches die Reaktion eines Benzolderivats der Formel (2)

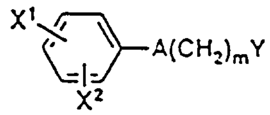

(2)

worin Y ein Chloratom, ein Bromatom, ein Jodatom, eine Mesyloxygruppe oder eine Tosyloxygruppe ist, und $X^1$, $X^2$, A und m wie in Anspruch 1 definiert sind, mit einem Pyridindionderivat der Formel (3)

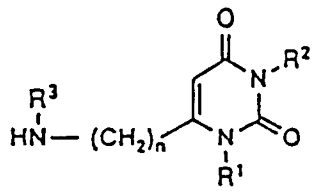

(3)

worin $R^1$, $R^2$, $R^3$ und n wie in Anspruch 1 definiert sind, umfaßt.

**7.** Verfahren zur Herstellung eines Pyrimidindionderivats der Formel (1) gemäß einem der Ansprüche 1-5, welches die Reaktion eines Alkylaminderivats der Formel (4)

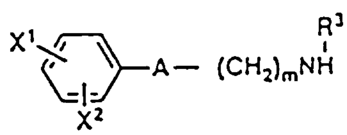

(4)

worin $X^1$, $X^2$, A, $R^3$ und m wie in Anspruch 1 definiert sind, mit einem Pyrimidindionderivat der Formel (5)

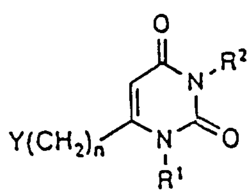

(5)

worin Y ein Chloratom, ein Bromatom, ein Jodatom, eine Mesyloxygruppe oder eine Tosyloxygruppe ist, und $R^1$, $R^2$ und n wie in Anspruch 1 definiert sind, umfaßt.

**8.** Verfahren zur Herstellung eines Pyrimidindionderivats der Formel (1) (worin A ein Sauerstoffatom darstellt) gemäß einem der Ansprüche 1-5, welches die Reaktion eines Phenolderivats der Formel (6)

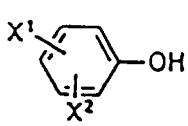

(6)

worin $X^1$ und $X^2$ wie in Anspruch 1 definiert sind, mit einem Pyrimidindionderivat der Formel (7)

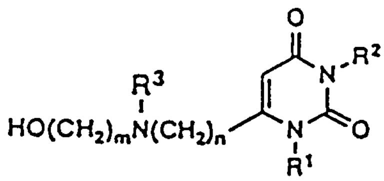

(7)

worin $R^1$, $R^2$, $R^3$, m und n wie in Anspruch 1 definiert sind, umfaßt.

9. Antiarrhythmische Zusammensetzung, die (a) als aktiven Bestandteil ein Pyrimidindionderivat der Formel (1) oder ein pharmazeutisch geeignetes Salz davon gemäß einem der Ansprüche 1-5 und (b) ein pharmazeutisch geeignetes Trägermaterial oder Verdünnungsmittel umfaßt.

10. Pyrimidindionderivat der Formel (8)

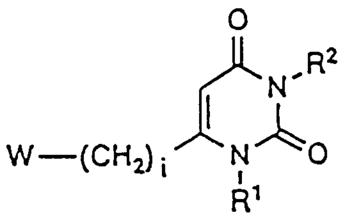

(8)

worin $R^1$ und $R^2$ wie in Anspruch 1 oder 4 definiert sind; W eine Hydroxylgruppe, ein Chloratom, ein Bromatom, ein Jodatom, eine Mesyloxygruppe, eine Tosyloxygruppe, eine Benzyloxygruppe, eine Phtaloylaminogruppe, eine Alkyloxycarbonylgruppe mit 2 bis 7 Kohlenstoffatomen, eine Vinylgruppe, $-NHR^3$ oder $-NR^3(CH_2)_mOH$ (worin $R^3$ und m wie in Anspruch 1 definiert sind) sind; und i eine ganze Zahl von 1 bis 3 ist, wenn W eine Alkyloxycarbonylgruppe mit 2 bis 7 Kohlenstoffatomen ist, 0 oder 2 ist, wenn W eine Vinylgruppe ist, oder in anderen Fällen eine ganze Zahl von 2 bis 4 ist.

11. Verwendung eines Pyrimidindionderivats der Formel (1) oder eines pharmazeutisch geeigneten Salzes davon gemäß einem der Ansprüche 1-5 zur Herstellung von antiarrhythmischen Mitteln.

**Revendications**

1. Dérivé de pyrimidinedione de formule (1)

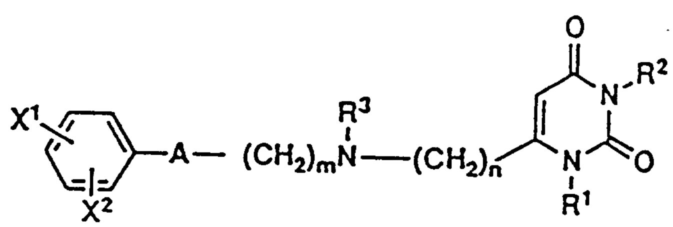

(1)

dans laquelle

$R^1$ et $R^2$ représentent chacun indépendamment un atome d'hydrogène ou un groupe alkyle de 1 à 6 atomes de carbone ;
$R^3$ représente un atome d'hydrogène, un groupe alkyle de 1 à 6 atomes de carbone, un groupe alkyle de 1 à 4 atomes de carbone qui est substitué par un groupe dialkylamino de 2 à 12 atomes de carbone ou un groupe alkyle de 1 à 4 atomes de carbone qui est substitué par un groupe alkyloxy de 1 à 6 atomes de carbone ;
A représente un groupe méthylène ou un atome d'oxygène ;
$X^1$ et $X^2$ représentent chacun indépendamment un atome d'hydrogène, un groupe alcanoyle de 2 à 6 atomes de carbone, un groupe benzoyle, un groupe alcanoylamino de 2 à 6 atomes de carbone, un groupe amino, un

groupe nitro, un groupe alkylsulfonylamino de 1 à 6 atomes de carbone, un groupe benzènesulfonylamino, un groupe cyano, un atome de fluor, un atome de chlore, un groupe trifluorométhyle, un groupe imidazole, un groupe alkyloxy de 1 à 6 atomes de carbone, un groupe trifluorométhoxy ou un groupe alkylthio de 1 à 6 atomes de carbone ;

m représente un entier de 1 à 4 ; et

n représente un entier de 2 à 4 ;

ou un sel pharmaceutiquement acceptable de celui-ci.

2. Dérivé de pyrimidinedione ou sel pharmaceutiquement acceptable de celui-ci selon la revendication 1, où $X^1$ et $X^2$ représentent chacun indépendamment un atome d'hydrogène, un groupe alcanoyle de 2 à 6 atomes de carbone, un groupe benzoyle, un groupe amino, un groupe nitro, un groupe alkylsulfonylamino de 1 à 6 atomes de carbone, un groupe benzènesulfonylamino, un groupe cyano, un atome de fluor, un atome de chlore, un groupe trifluorométhyle, un groupe imidazole, un groupe alkyloxy de 1 à 6 atomes de carbone ou un groupe trifluorométhoxy.

3. Dérivé de pyrimidinedione ou sel pharmaceutiquement acceptable de celui-ci selon la revendication 1 ou 2, où $R^3$ représente un atome d'hydrogène ou un groupe alkyle de 1 à 4 atomes de carbone.

4. Dérivé de pyrimidinedione ou sel pharmaceutiquement acceptable de celui-ci selon la revendication 1, 2 ou 3, où $R^1$ et $R^2$ représentent chacun indépendamment un groupe alkyle de 1 à 3 atomes de carbone.

5. Dérivé de pyrimidinedione ou sel pharmaceutiquement acceptable de celui-ci selon la revendication 4, où $X^1$ et $X^2$ représentent chacun indépendamment un atome d'hydrogène, un groupe alcanoyle de 2 à 6 atomes de carbone, un groupe amino, un groupe nitro, un groupe alkylsulfonylamino de 1 à 6 atomes de carbone, un atome de fluor, un atome de chlore ou un groupe imidazole, et $R^3$ représente un atome d'hydrogène ou un groupe alkyle de 1 à 4 atomes de carbone.

6. Procédé pour la production d'un dérivé de pyrimidinedione de formule (1) selon l'une quelconque des revendications précédentes, qui comprend la réaction d'un dérivé du benzène de formule (2)

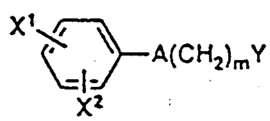

(2)

dans laquelle Y représente un atome de chlore, un atome de brome, un atome d'iode, un groupe mésyloxy ou un groupe toxyloxy, et $X^1$, $X^2$, A et m sont tels que définis dans la revendication 1, avec un dérivé de pyridiminedione de formule (3)

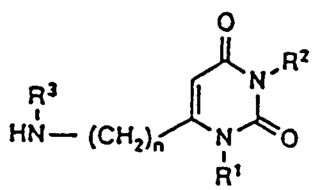

(3)

dans laquelle $R^1$, $R^2$, $R^3$ et n sont tels que définis dans la revendication 1.

7. Procédé pour la production d'un dérivé de pyrimidinedione de formule (1) selon l'une quelconque des revendications 1 à 5, qui comprend la réaction d'un dérivé d'alkylamine de formule (4)

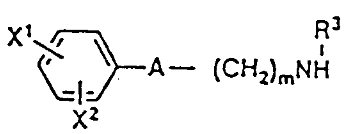

(4)

dans laquelle $X^1$, $X^2$, A, $R^3$ et m sont tels que définis dans la revendication 1, avec un dérivé de pyrimidinedione

de formule (5)

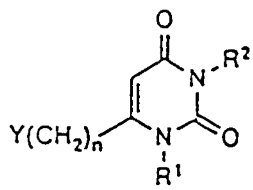

(5)

dans laquelle Y représente un atome de chlore, un atome de brome, un atome d'iode, un groupe mésyloxy ou un groupe tosyloxy, et $R^1$, $R^2$ et n sont tels que définis dans la revendication 1.

**8.** Procédé pour la production d'un dérivé de pyridiminedione de formule (1) (dans laquelle A représente un atome d'oxygène) selon l'une quelconque des revendications 1 à 5, qui comprend la réaction d'un dérivé du phénol de formule (6)

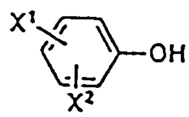

(6)

dans laquelle $X^1$ et $X^2$ sont tels que définis dans la revendication 1, avec un dérivé de pyrimidinedione de formule (7)

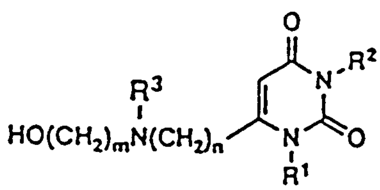

(7)

dans laquelle $R^1$, $R^2$, $R^3$, m et n sont tels que définis dans la revendication 1.

**9.** Composition antiarythmique comprenant (a), en tant qu'ingrédient actif, un dérivé de pyrimidinedione de formule (1) ou un sel pharmaceutiquement acceptable de celui-ci selon l'une quelconque des revendications 1 à 5 et (b) un véhicule ou diluant pharmaceutiquement acceptables.

**10.** Dérivé de pyrimidinedione de formule (8)

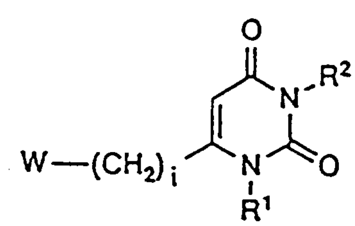

(8)

dans laquelle $R^1$ et $R^2$ sont tels que définis dans la revendication 1 ou la revendication 4 ; W représente un groupe hydroxy, un atome de chlore, un atome de brome, un atome d'iode, un groupe mésyloxy, un groupe tosyloxy, un groupe benzyloxy, un groupe phtaloylamino, un groupe alkyloxycarbonyle de 2 à 7 atomes de carbone, un groupe vinyle, $-NHR^3$ ou $-NR^3(CH_2)_mOH$ (où $R^3$ et m sont tels que définis dans la revendication 1) ; et i représente un entier de 1 à 3 lorsque W est un groupe alkyloxycarbonyle de 2 à 7 atomes de carbone, un entier 0 ou 2 lorsque W est un groupe vinyle, ou un entier de 2 à 4 dans les autres cas.

**11.** Utilisation d'un dérivé de pyrimidinedione de formule (1) ou d'un sel pharmaceutiquement acceptable de celui-ci

selon l'une quelconque des revendications 1 à 5 pour la préparation d'agents antiarythmiques.